(19) Europäisches Patentamt
European Patent Office
Office européen des brevets

(11) EP 3 915 636 A1

(12) **EUROPEAN PATENT APPLICATION**
published in accordance with Art. 153(4) EPC

(43) Date of publication:
**01.12.2021 Bulletin 2021/48**

(21) Application number: **20748123.5**

(22) Date of filing: **30.01.2020**

(51) Int Cl.:
***A61P** / (2006.01)*

(86) International application number:
**PCT/JP2020/003311**

(87) International publication number:
**WO 2020/158841 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.01.2019 JP 2019014899**
**31.05.2019 JP 2019103322**

(71) Applicant: **Nissan Chemical Corporation**
**Tokyo 103-6119 (JP)**

(72) Inventors:
- **NISHINO, Taito**
  **Shiraoka-shi, Saitama 349-0294 (JP)**
- **AIHARA, Ayako**
  **Shiraoka-shi, Saitama 349-0294 (JP)**
- **OTSUKA, Keiichiro**
  **Shiraoka-shi, Saitama 349-0294 (JP)**
- **IWAWAKI, Takumi**
  **Shiraoka-shi, Saitama 349-0294 (JP)**
- **MIKASHIMA, Takumi**
  **Tokyo 103-6119 (JP)**
- **KAMAURA, Masahiro**
  **Funabashi-shi, Chiba 274-8507 (JP)**
- **FUKASAWA, Natsuki**
  **Shiraoka-shi, Saitama 349-0294 (JP)**
- **NAKAJIMA, Hiroyuki**
  **Shiraoka-shi, Saitama 349-0294 (JP)**
- **SHIGETA, Yukihiro**
  **Funabashi-shi, Chiba 274-8507 (JP)**
- **IWAMOTO, Toshimasa**
  **Funabashi-shi, Chiba 274-8507 (JP)**

(74) Representative: **J A Kemp LLP**
**80 Turnmill Street**
**London EC1M 5QU (GB)**

(54) **HYDRAZIDE COMPOUND AND KINASE INHIBITOR**

(57) The present invention aims to provide a novel compound capable of promoting cell proliferation in a cell culture (particularly three-dimensional cell culture). The hydrazide compound represented by the formula (I);

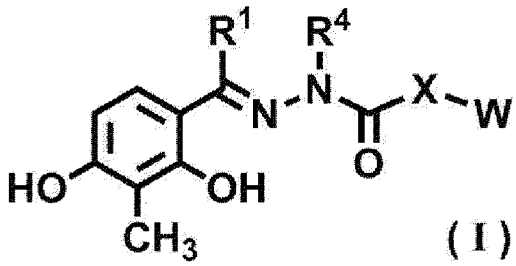

(I)

wherein each symbol is as defined in DESCRIPTION, or a salt thereof, and a composition containing same can remarkably promote cell proliferation, sphere formation, cyst formation and/or organoid formation, and can also remarkably inhibit the activities of kinase such as LATS1, LATS2 and the like.

EP 3 915 636 A1

## Description

[Technical Field]

The present invention relates to a novel hydrazide compound, a composition containing the hydrazide compound for addition to a medium, a method for culturing a cell or tissue which characteristically uses the composition for addition to a medium, a kinase inhibitor containing the hydrazide compound, and the like.

[Background Art]

In recent years, experiments using cells have been extremely frequently performed in many fields including the life science field for the purpose of elucidating the action mechanism of life phenomena and establishing treatment methods for diseases and the like. For example, as one example in the field of drug discovery, there is a method of having a candidate compound act on cancer cells to be a treatment target and screening for a compound capable of suppressing proliferation of the cancer cells. In such screening, tens of thousands of candidate compounds may be screened, and in such embodiment, it is necessary to prepare a large amount of homogeneous cells. However, cells of higher organisms such as human and the like require a period of about one day even for cells that divide relatively quickly. In addition, some cancer cells and stem cells require more than a few months for a single cell division. This is the factor preventing rapid cell procurement. From such background, construction of means capable of promoting proliferation of slow-dividing cells has been demanded. For example, there are reports that a thiol compound having a particular structure promotes proliferation of hematopoietic progenitor cells, and that polyprenyl compounds promote proliferation of hepatocytes and the like (patent documents 1, 2).

On the other hand in the field of drug discovery screening, three-dimensional culture of cells is attracting attention in recent years. Three-dimensional culture is a cell culture technique that is between in vitro and in vivo. In three-dimensional culture, cells can form a steric structure such as a sphere (also referred to as spheroid) or the like, and therefore, an assay that is closer to a living body compared with general two-dimensional culture can be available. Hence, three-dimensional culture may be able to identify a compound for treating diseases that could not be identified by drug discovery screening using two-dimensional culture (non-patent document 1).

Kinases (protein phosphorylating enzymes, protein kinases) are enzymes that phosphorylate the hydroxyl groups of serine, threonine, or tyrosine in proteins, and are a group of enzymes responsible for signal transduction such as cell proliferation and differentiation. Not less than 500 kinds of genes encoding kinases have been cloned so far. Protein kinases are present throughout the cells and are deeply involved in the regulation and control of cell proliferation, differentiation, and functional expression (non-patent document 2) .

Large tumor suppressor (hereinafter to be abbreviated as LATS) 1 and LATS2 are protein kinases that are major factors constituting the Hippo signal transduction pathway (patent document 3, non-patent documents 3, 4). It is known that this transduction pathway is activated when the cell density increases and contact inhibition is applied, or when cells are injured by active oxygen, DNA damage, or the like (non-patent documents 5, 6, 7). When this transduction pathway is activated, LATS phosphorylates the transcriptional co-factor Yes-associated protein (hereinafter to be abbreviated as YAP) or transcriptional co-activator with PDZ-binding motif (hereinafter to be abbreviated as TAZ). Phosphorylated YAP or TAZ transfers from the cell nucleus to the cytoplasm and is subjected to proteolysis, thus suppressing the expression of the target gene of YAP or TAZ. Known examples of the genes whose expression is regulated by YAP include CCND1, CTGF, BIRC2, CYR61, AMOTL2, TGFB2 and the like (non-patent documents 8, 9, 10, 11). Activation of the Hippo signal transduction pathway leads to the suppression of the expression of these genes, and causes suppression of cell proliferation and induction of cell death. In recent years, it has been reported that cell proliferation in three-dimensional culture in vitro is promoted by genetic deletion of LATS1 and LATS2 (non-patent document 12).

Hippo signal transduction pathway is known to regulate self-renewal and differentiation of stem cells (non-patent document 13). Hippo signal transduction pathway is necessary for maintaining the skin, intestines and nerves, and repair during tissue damage is inhibited unless YAP functions normally (non-patent document 14). Furthermore, deletion of the Hippo signal transduction pathway has been reported to ameliorate systolic heart failure after myocardial infarction (non-patent document 15). In this way, Hippo signal transduction pathway controls the proliferation, maintenance and recovery of cells, tissues and organs. Thus, inhibitors of the Hippo signal transduction pathway and activators of YAP and TAZ are expected to be therapeutic drugs for diseases caused by failure of cell proliferation. Examples of such diseases include burn, trauma, muscular atrophy, ischemic diseases, and neurodegenerative diseases (e.g., Alzheimer's, Parkinson's and Huntington's diseases). As an example of an inhibitor of the Hippo signal transduction pathway, XMU-MP-1 has been reported as an inhibitor of MST1 (non-patent document 16). XMU-MP-1 has been shown to promote recovery from hepatopathy by inhibiting the Hippo signal transduction pathway. In addition, IBS008738 has been reported as an activator of TAZ, and the effect of promoting recovery of muscle damage by this activator has been shown (non-patent document 17). Furthermore, it has been reported that compounds that inhibit LATS1 and LATS2 have an effect

of promoting repair of eyeball, skin and liver (patent document 3). As described above, a regulator of the Hippo signal transduction pathway is expected to be a therapeutic drug for various diseases.

[Document List]

[Patent documents]

patent document 1: Japanese Translation of PCT Application Publication No. H11-504000
patent document 2: WO 2008/155920
patent document 3: US Patent No. 5994503
patent document 4: WO 2018/198077

[Non-patent documents]

non-patent document 1: Susan Breslin, Drug Discovery Today 2013, 18 (5) :240-249
non-patent document 2: Robert Roskoski Jr., Pharmacological Research 2016,100: 1-23
non-patent document 3: Justice RW et al., Genes & Development 1995, 9(5):534-546
non-patent document 4: Hergovich A, Cell & Bioscience 2013, 3 (1) : 32
non-patent document 5: Pefani DE et al., The FEBS Journal 2016, 283 (8) :1392-1403
non-patent document 6: Meng Z et al., Genes & Development 2016, 30(1):1-17
non-patent document 7: Zhao B et al., Current Opinion in Cell Biology 2008, 20 (6) : 638-646
non-patent document 8: Imajo M et al., The EMBO Journal 2012, 31:1109-1122
non-patent document 9: Hong W et al, Seminars in Cell & Developmental Biology 2012, 23(7):785-793
non-patent document 10: Gujral TS et al., Proceedings of the National Academy of Sciences of the United States of America 2017, 114 (18) :E3729-E3738
non-patent document 11: Lee DH et al., Nature Communication 2016, 7:11961
non-patent document 12: Moroishi T et al., Cell 2016, 167:1525-1539
non-patent document 13: Aylon Y et al., Cell Death & Differentiation 2014, 21 (4) : 624-633
non-patent document 14: Johnson R et al., Nature Reviews Drug Discovery 2014, 13(1):63-79
non-patent document 15: Leach JP et al., Nature 2017, 550 (7675) :260-264
non-patent document 16: Fan F et al., Science Translational Medicine 2016, 8 (352) : 352ra108
non-patent document 17: Yang Z et al., Molecular and Cellular Biology 2014, 34 (9) : 1607-1621

[Summary of Invention]

[Technical Problem]

The present invention aims to provide novel compounds capable of promoting cell proliferation in cell culture (particularly three-dimensional cell culture).

[Solution to Problem]

The present inventors have conducted intensive studies of the aforementioned problems and found that the hydrazide compounds newly synthesized at this time can promote proliferation of various cells under culture (particularly, three-dimensional culture) extremely well. Furthermore, the present inventors have also found that the compound inhibits LATS1 and LATS2. Based on such finding, they have conducted further studies and completed the present invention. Therefore, the present invention provides the following.

[1] A hydrazide compound represented by the formula (I);

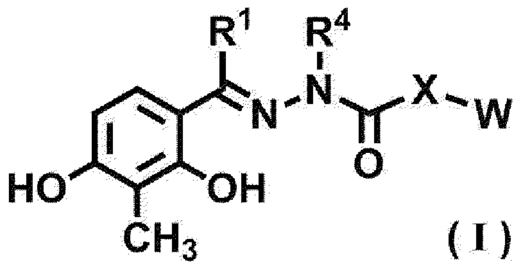

(I)

wherein X is $-N(R^5)C(R^2)(R^3)-$, $-C(R^2)(R^3)N(R^5)-$ or $-C(R^2)(R^3)O-$,
W is a ring represented by any of the following D-1, D-2, D-3, D-4, D-5, D-6, D-7, D-8, D-9, D-10, D-11 and D-12,

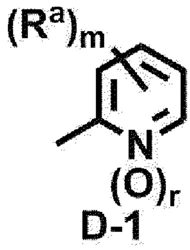
D-1

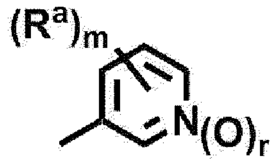
D-2

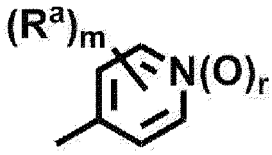
D-3

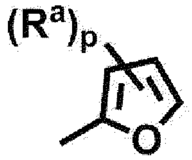
D-4

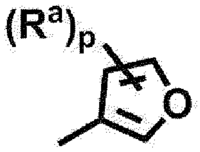
D-5

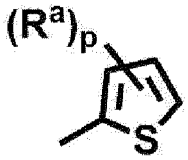
D-6

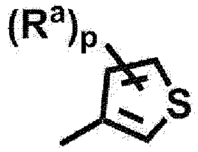
D-7

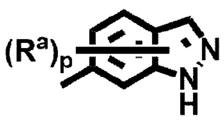
D-8

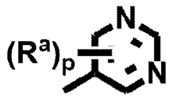
D-9

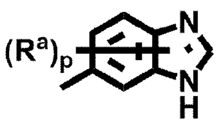
D-10

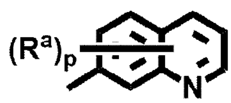
D-11

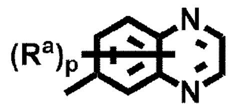
D-12

$R^1$ is $C_1$ - $C_6$ alkyl or halo ($C_1$ - $C_6$) alkyl,
$R^2$ and $R^3$ are each independently a hydrogen atom or $C_1$ - $C_6$ alkyl,
$R^4$ and $R^5$ are each independently a hydrogen atom or $C_1$ - $C_6$ alkyl,
$R^a$ is hydroxy, nitro, a halogen atom, $C_1$ - $C_6$ alkyl, halo ($C_1$ - $C_6$) alkyl, $C_2$ - $C_6$ alkenyl, $C_2$ - $C_6$ alkynyl, $C_1$ - $C_6$ alkoxy, halo ($C_1$ - $C_6$) alkoxy, $C_1$ - $C_6$ alkylthio, halo ($C_1$ - $C_6$) alkylthio, $C_1$ - $C_6$ alkylsulfinyl or $C_1$ - $C_6$ alkylsulfonyl,
m is 0, 1, 2, 3 or 4,
p is 0, 1, 2 or 3, and
r is 0 or 1, or a salt thereof.

[2] The hydrazide compound of [1], wherein

X is $-C(R^2)(R^3)N(R^5)-$,
W is a ring represented by any of D-1, D-2 and D-3,
$R^4$ and $R^5$ are each a hydrogen atom,
$R^a$ is $C_1$ - $C_6$ alkyl or a halogen atom,
m is 0 or 1, and
r is 0, or a salt thereof.

[3] The hydrazide compound of [1], wherein

X is $-N(R^5)C(R^2)(R^3)-$,
W is a ring represented by any of D-2, D-4, D-5, D-6 and D-7,
$R^2$, $R^3$, $R^4$ and $R^5$ are each a hydrogen atom, and
p, m and r are each 0, or a salt thereof.

[4] The hydrazide compound of [1], wherein

X is $-C(R^2)(R^3)O-$,
W is a ring represented by D-2, and
m and r are each 0, or a salt thereof.

[5] The hydrazide compound of [1], wherein

X is $-C(R^2)(R^3)N(R^5)-$,
W is a ring represented by any of D-8, D-9, D-10, D-11 and D-12,
$R^4$ and $R^5$ are each a hydrogen atom, and
p is 0, or a salt thereof.

[6] A composition for addition to a medium, comprising the hydrazide compound of any of [1] to [5] or a salt thereof.
[7] The composition of [6], wherein the composition is for promoting cell proliferation.
[8] The composition of [7], wherein the cell is selected from the group consisting of a normal cell line, a cancer cell line and a stem cell.
[9] The composition of [6], wherein the composition is used for promoting sphere formation, cyst formation or organoid formation.
[10] The composition of [6], wherein the composition is used for producing a cell for transplantation or an organoid for transplantation.
[11] The composition of [6], wherein the composition is for promoting cell adhesion.
[12] A cell culture medium comprising the hydrazide compound of any of [1] to [5] or a salt thereof.
[13] A method for promoting cell proliferation, comprising adding the hydrazide compound of any of [1] to [5] or a salt thereof to a cell culture medium.
[14] The method of [13], wherein the cell is selected from the group consisting of a normal cell line, a cancer cell line and a stem cell.
[15] A method for promoting cell adhesion, comprising adding the hydrazide compound of any of [1] to [5] or a salt thereof to a cell culture medium.
[16] A kinase inhibitor, comprising the hydrazide compound of any of [1] to [5] or a salt thereof.
[17] The kinase inhibitor of [16], wherein the kinase is LATS.
[18] A Hippo signal transduction pathway inhibitor, comprising the hydrazide compound of any of [1] to [5] or a salt thereof.
[19] A pharmaceutical composition comprising the hydrazide compound of any of [1] to [5] or a salt thereof.
[20] The pharmaceutical composition of [19], wherein the composition is used for treating a disease in eye, liver, skin or intestine.

[Advantageous Effects of Invention]

According to the present invention, cell proliferation, sphere formation, cyst formation and/or organoid formation can be promoted remarkably. In addition, according to the present invention, the activity of kinases such as LATS1, LATS2 and the like can be inhibited remarkably. According to the present invention, moreover, diseases associated with cell proliferation failure can be treated and regeneration of damaged tissue can be promoted.

[Brief Description of Drawings]

Fig. 1 is a confocal fluorescence micrograph of Experimental Example 4.
Fig. 2 is a graph showing the effect of the compound of the present invention on the recovery of excised liver in Experimental Example 18.
Fig. 3 is a graph showing the effect of the compound of the present invention on the recovery from colitis in Experimental Example 19.

[Description of Embodiments]

A novel compound used in the present invention is a compound represented by the following formula (I) (hereinafter sometimes to be referred to as "the compound of the present invention").

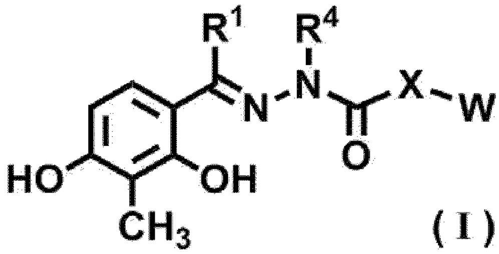

(I)

wherein X is $-N(R^5)C(R^2)(R^3)-$, $-C(R^2)(R^3)N(R^5)-$ or $-C(R^2)(R^3)O-$,
W is a ring represented by any of the following D-1, D-2, D-3, D-4, D-5, D-6, D-7, D-8, D-9, D-10, D-11 and D-12,

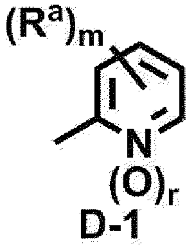
D-1

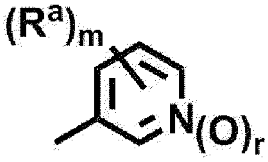
D-2

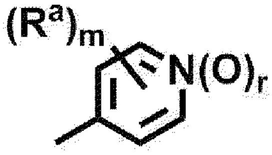
D-3

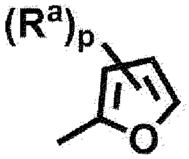
D-4

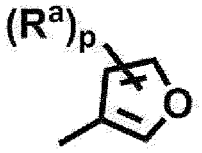
D-5

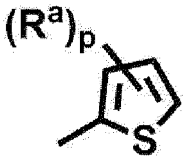
D-6

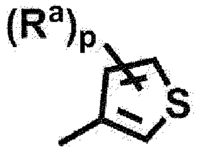
D-7

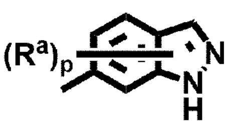
D-8

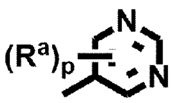
D-9

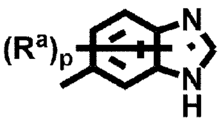
D-10

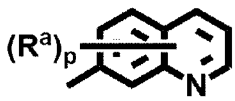
D-11

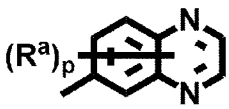
D-12

$R^1$ is $C_1$ - $C_6$ alkyl or halo ($C_1$ - $C_6$) alkyl,
$R^2$ and $R^3$ are each independently a hydrogen atom or $C_1$ - $C_6$ alkyl,
$R^4$ and $R^5$ are each independently a hydrogen atom or $C_1$ - $C_6$ alkyl,
$R^a$ is hydroxy, nitro, a halogen atom, $C_1$ - $C_6$ alkyl, halo ($C_1$ - $C_6$) alkyl, $C_2$ - $C_6$ alkenyl, $C_2$ - $C_6$ alkynyl, $C_1$ - $C_6$ alkoxy, halo ($C_1$ - $C_6$) alkoxy, $C_1$ - $C_6$ alkylthio, halo ($C_1$ - $C_6$) alkylthio, $C_1$ - $C_6$ alkylsulfinyl or $C_1$ - $C_6$ alkylsulfonyl,
m is 0, 1, 2, 3 or 4,
p is 0, 1, 2 or 3, and
r is 0 or 1.

In a compound represented by the formula (I), one embodiment may be that

X is $-C(R^2)(R^3)N(R^5)-$,
W is a ring represented by any of D-1, D-2 and D-3,

$R^4$ and $R^5$ are each a hydrogen atom,
$R^a$ is $C_1$ - $C_6$ alkyl or a halogen atom,
m is 0 or 1, and
r is 0.

In a compound represented by the formula (I), one embodiment may be that

X is $-C(R^2)(R^3)N(R^5)-$,
W is a ring represented by D-2,
$R^2$ is $C_1$ - $C_6$ alkyl,
$R^3$, $R^4$ and $R^5$ are each a hydrogen atom,
$R^a$ is $C_1$ - $C_6$ alkyl,
m is 0, and
r is 0.

In a compound represented by the formula (I), one embodiment may be that

X is $-C(R^2)(R^3)N(R^5)-$,
W is a ring represented by D-2,
$R^2$ is $C_1$ - $C_6$ alkyl,
$R^3$, $R^4$ and $R^5$ are each a hydrogen atom,
$R^a$ is a halogen atom,
m is 1,
r is 0.

In a compound represented by the formula (I), another embodiment may be that

X is $-N(R^5)C(R^2)(R^3)-$,
W is a ring represented by any of D-2, D-4, D-5, D-6 and D-7,
$R^2$, $R^3$, $R^4$ and $R^5$ are each a hydrogen atom, and
p, m and r are each 0.

In a compound represented by the formula (I), a still another embodiment may be that

X is $-C(R^2)(R^3)O-$,
W is a ring represented by D-2, and
m and r are each 0

In a compound represented by the formula (I), a still another embodiment may be that

W is a ring represented by any of D-8, D-9, D-10, D-11 and D-12,
$R^4$ and $R^5$ are each a hydrogen atom, and
p is 0.

The compound represented by the formula (I) may exist in some cases as, for example, a keto-enol structure tautomer represented by the following formula, depending on the kind of the substituents and conditions. The compound of the present invention also includes all isomers that may exist.

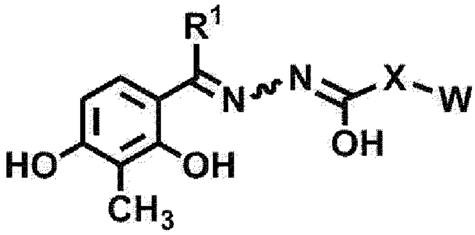
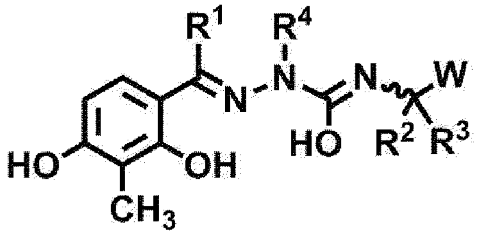

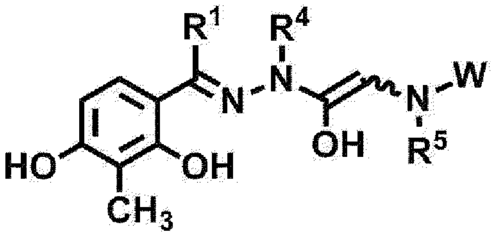 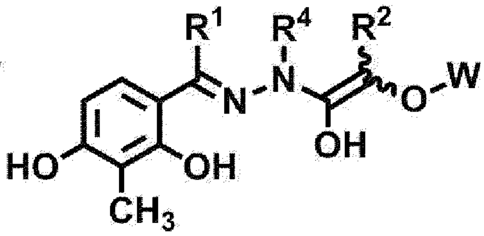

The compounds encompassed in the compound of the present invention may contain geometric isomers of an E-form having an E-steric configuration and Z-form having a Z-steric configuration depending on the kind of the substituent. The compound of the present invention includes E-form, Z-form and a mixture containing E-form and Z-form in any ratio. In the present specification, these are shown, for example, as a bond of a wavy line as described above.

The compounds encompassed in the compound of the present invention have an optically active substance due to the presence of one or more asymmetric carbon atoms. The compound of the present invention includes all optically active or racemic compounds.

Among the compounds encompassed in the compound of the present invention, for example, those that can be converted to acid addition salts according to a conventional method include, but are not limited to, salts with hydrohalic acids such as hydrofluoric acid, hydrochloric acid, hydrobromic acid, hydroiodic acid and the like, salts with inorganic acids such as nitric acid, sulfuric acid, phosphoric acid, chlorine acid, perchloric acid and the like, salts with sulfonic acids such as methanesulfonic acid, ethanesulfonic acid, trifluoromethanesulfonic acid, benzenesulfonic acid, p-toluenesulfonic acid and the like, salts with carboxylic acids such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid, citric acid and the like, and salts with amino acids such as glutamic acid, aspartic acid and the like.

Alternatively, among the compounds encompassed in the compound of the present invention, for example, those that can be converted to a metal salt according to a conventional method include, but are not limited to, salts with alkali metals such as lithium, sodium, and potassium, salts with alkaline earth metals such as calcium, barium, and magnesium, and salts with aluminum.

Specific examples of each substituent shown in the present specification are as follows. As used herein, n- means normal, i- means iso, s- means secondary, t- means tertiary, and Ph means phenyl.

In the present specification, the halogen atom includes a fluorine atom, a chlorine atom, a bromine atom and an iodine atom. In the present specification, the notation of "halo" also means these halogen atoms.

In the present specification, the notation of $C_a$ - $C_b$ alkyl means a linear or branched chain saturated hydrocarbon group having a to b carbon atoms. Specific examples include methyl, ethyl, n-propyl, i-propyl, n-butyl, i-butyl, s-butyl, t-butyl, n-pentyl, 1,1-dimethylpropyl, n-hexyl and the like, and are selected within the range of respective specified number of carbon atoms.

In the present specification, the notation of halo ($C_a$ - $C_b$)alkyl means a linear or branched chain saturated hydrocarbon having a to b carbon atoms, in which a hydrogen atom bonded to a carbon atom is optionally substituted by a halogen atom. When substituted by two or more halogen atoms, the halogen atoms may be the same or different from each other. Specific examples include fluoromethyl, chloromethyl, bromomethyl, iodo methyl, difluoromethyl, dichloromethyl, trifluoromethyl, chlorodifluoromethyl, trichloromethyl, bromodifluoromethyl, 1-fluoroethyl, 2-fluoroethyl, 2-chloroethyl, 2-bromoethyl, 2,2-difluoroethyl, 2,2,2-trifluoroethyl, 2-chloro-2,2-difluoroethyl, 2,2,2-trichloroethyl, 2-bromo-2,2-difluoroethyl, 1,1,2,2-tetrafluoroethyl, 2-chloro-1,1,2-trifluoroethyl, 2-chloro-1,1,2,2-tetrafluoroethyl, pentafluoroethyl, 2,2-difluoropropyl, 3,3,3-trifluoropropyl, 3-bromo-3,3-difluoropropyl, 2,2,3,3-tetrafluoropropyl, 2,2,3,3,3-pentafluoropropyl, 1,1,2,3,3,3-hexafluoropropyl, heptafluoropropyl, 2,2,2-trifluoro-1-(methyl)ethyl, 2,2,2-trifluoro-1-(trifluoromethyl)ethyl, 1,2,2,2-tetrafluoro-1-(trifluoromethyl)ethyl, 2,2,3,4,4,4-hexafluorobutyl, 2,2,3,3,4,4,4-heptafluorobutyl, nonafluorobutyl and the like, and are selected within the range of respective specified number of carbon atoms.

In the present specification, the notation of $C_a$ - $C_b$ alkenyl means the aforementioned linear or branched chain unsaturated hydrocarbon having a to b carbon atoms, and one or more double bonds in a molecule. Specific examples include vinyl, 1-propenyl, 2-propenyl, 1-methylethenyl, 2-butenyl, 2-methyl-2-propenyl, 3-methyl-2-butenyl, 1,1-dimethyl-2-propenyl and the like, and are selected within the range of respective specified number of carbon atoms.

In the present specification, the notation of $C_a$ - $C_b$ alkynyl means the aforementioned linear or branched chain unsaturated hydrocarbon having a to b carbon atoms, and one or more triple bonds in a molecule. Specific examples include ethynyl, propargyl, 2-butynyl, 3-butynyl, 1-pentynyl, 1-hexynyl, 4,4,4-trifluoro-2-butynyl and the like, and are selected within the range of respective specified number of carbon atoms.

In the present specification, the notation of $C_a$ - $C_b$ alkoxy means the aforementioned alkyl having a to b carbon atoms -O- group. Specific examples include methoxy, ethoxy, n-propyloxy, i-propyloxy, n-butyloxy, i-butyloxy, s-butyloxy,

t-butyloxy and the like, and are selected within the range of respective specified number of carbon atoms.

In the present specification, the notation of halo ($C_a$ - $C_b$)alkoxy means the aforementioned haloalkyl having a to b carbon atoms -O-. Specific examples include difluoromethoxy, trifluoromethoxy, chlorodifluoromethoxy, bromodifluoromethoxy, 2-fluoroethoxy, 2-chloroethoxy, 2,2,2-trifluoroethoxy, 1,1,2,2,-tetrafluoroethoxy, 2-chloro-1,1,2-trifluoroethoxy, 1,1,2,3,3,3-hexafluoropropyloxy and the like, and are selected within the range of respective specified number of carbon atoms.

In the present specification, the notation of $C_a$ - $C_b$ alkylthio means the aforementioned alkyl having a to b carbon atoms -S-. Specific examples include methylthio, ethylthio, n-propylthio, i-propylthio, n-butylthio, i-butylthio, s-butylthio, t-butylthio and the like, and are selected within the range of respective specified number of carbon atoms.

In the present specification, the notation of halo ($C_a$ - $C_b$)alkylthio means the aforementioned haloalkyl having a to b carbon atoms -S-. Specific examples include difluoromethylthio, trifluoromethylthio, chlorodifluoromethylthio, bromodifluoromethylthio, 2,2,2-trifluoroethylthio, 1,1,2,2-tetrafluoroethylthio, 2-chloro-1,1,2-trifluoroethylthio, pentafluoroethylthio, 1,1,2,3,3,3-hexafluoropropylthio, heptafluoropropylthio, 1,2,2,2-tetrafluoro-1-(trifluoromethyl)ethylthio, nonafluorobutylthio and the like, and are selected within the range of respective specified number of carbon atoms.

In the present specification, the notation of $C_a$ - $C_b$ alkylsulfinyl means the aforementioned alkyl having a to b carbon atoms -S(O)-. Specific examples include methylsulfinyl, ethylsulfinyl, n-propylsulfinyl, i-propylsulfinyl, n-butylsulfinyl, i-butylsulfinyl, S-butylsulfinyl, t-butylsulfinyl and the like, and are selected within the range of respective specified number of carbon atoms.

In the present specification, the notation of $C_a$ - $C_b$ alkylsulfonyl means the aforementioned alkyl having a to b carbon atoms -$SO_2$-. Specific examples include methylsulfonyl, ethylsulfonyl, n-propylsulfonyl, i-propylsulfonyl, n-butylsulfonyl, i-butylsulfonyl, s-butylsulfonyl, t-butylsulfonyl and the like, and are selected within the range of respective specified number of carbon atoms.

The production method of the compound of the present invention is described in the following.

[Production method A]

A compound represented by the formula (I) can be synthesized, as shown in the following formula, a dehydration reaction of a compound represented by the formula (2-1) (wherein $R^1$ means the same as above) and a compound represented by the formula (3-1) (wherein W and X mean the same as above). As one embodiment, the following scheme shows a case of the formula (I) wherein $R^4$ is a hydrogen atom.

[Reaction scheme 1]

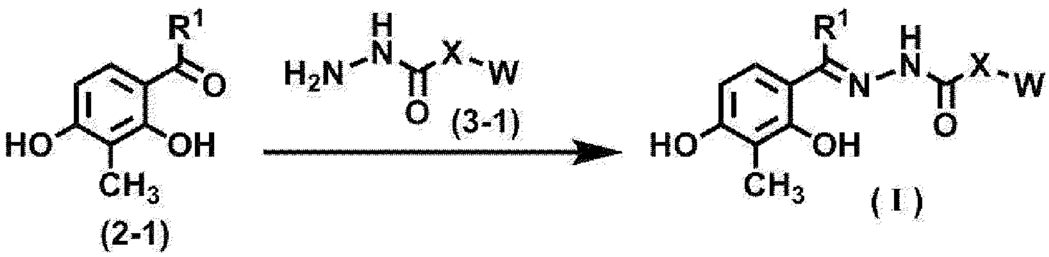

In this reaction, the compound represented by the formula (3-1) can be used within the range of 0.5 - 20 equivalents, preferably 1 - 2 equivalents, with respect to 1 equivalent of the compound represented by the formula (2-1).

This reaction may be performed without a solvent, or water or an organic solvent may be used. For example, the organic solvent includes aromatic hydrocarbons such as toluene, o-xylene and the like, ethers such as 1,4-dioxane, tetrahydrofuran and the like, and polar solvents such as N,N-dimethylformamide, dimethyl sulfoxide and the like. These solvents may be used alone, or two or more kinds of these may be used in a mixture.

As the reaction temperature, any temperature within the range of from 0°C to the refluxing temperature of the reaction mixture, preferably from 100°C to the refluxing temperature of the reaction mixture, can be adopted.

While the reaction time varies depending on the concentration of the reaction substrate and reaction temperature, it may be any within the range of generally 5 min to 100 hr, preferably 1 hr to 72 hr.

Some of the compounds represented by the formula (2-1) used here are known compounds, and some of them are commercially available products. They can also be produced according to the method described in documents [for example, Synthesis, (13), p.1857 (2002) and the like].

Some of the compounds represented by the formula (3-1) used here are known compounds, and some of them are commercially available products. They can also be produced according to the method described in documents [for example, Journal of Radioanalytical and Nuclear Chemistry, 298(1), p.9 (2013) and the like].

The reaction mixture after completion of the reaction is subjected to a general post-treatment such as direct concentration or dissolution in organic solvent, washing with water followed by concentration or placing in ice water, or concentration after extraction with an organic solvent to obtain the desired compound of the present invention. When purification is necessary, the compound can be separated and purified by any purification method such as recrystallization, column chromatography, thin-layer chromatography, liquid chromatography and the like.

When the compounds encompassed in the present invention include an optically active form due to the presence of one or more asymmetric carbon atoms, for example, an optically active form can be obtained by a method according to the aforementioned production method and using a starting material in an optically active form, or obtained from a racemate of the compound by performing optical resolution by liquid chromatography using a chiral column, supercritical fluid chromatography, or the like.

The three-dimensional cell culture (3D cell culture) in the present specification means, for example, culturing cells in a three-dimensional environment using an embedded culture method, a microcarrier culture method, a sphere culture method and the like. Embedded culture is a method of cultivating cells by embedding and fixing the cells in a solid or semisolid gel substrate such as Matrigel (registered trade mark), Geltrex (registered trade mark), agar, methylcellulose, collagen, gelatin, fibrin, agarose, alginates and the like. Microcarrier culture method is a method of cultivating cells in a suspended state by proliferating cells in a single layer on the surface of a fine particle slightly heavier than water (hereinafter to be also referred to as a microcarrier), and stirring the fine particles in a culture container such as a flask and the like. Sphere culture is a culture method including forming an aggregate composed of several dozen - several hundred object cells (hereinafter to be also referred to as a sphere or spheroid), and culturing the aggregates with standing or shaking in a medium. As the three-dimensional cell culture (3D cell culture) in the present invention, a method of culturing cells in a three-dimensional state closer to that in the living body can also be used by dispersing polysaccharides such as hyaluronic acid, deacylated gellan gum, xanthan gum and the like or a derivative of these in a medium to form an atypical three-dimensional network, and maintaining adherent cells suspended in the medium by using the network as a scaffold. At this time, the cells in the three-dimensional cell culture are trapped in the three-dimensional network and do not precipitate. Therefore, the cells can be cultured without a shaking or rotation operation or the like. The three-dimensional cell culture can be performed by a method known per se (e.g., WO 2014/017513).

[Composition for addition to a medium]

The present invention provides a composition for addition to a medium, containing the compound of the present invention (hereinafter sometimes referred to as the composition of the present invention). The composition of the present invention can achieve any or any combination of promoting cell proliferation, promoting sphere formation, promoting cyst (hereinafter sometimes indicated as Cyst) formation, and promoting organoid formation when added to a cell medium, particularly a three-dimensional cell culture medium.

That is, the use of the composition of the present invention is specifically exemplified by the following:

(1) promoting cell proliferation;
(2) promoting sphere formation;
(3) promoting organoid formation;
(4) promoting cyst formation;
(5) promoting cell proliferation and promoting sphere formation;
(6) promoting cell proliferation and promoting organoid formation;
(7) promoting cell proliferation and promoting cyst formation;
(8) promoting sphere formation and promoting organoid formation;
(9) promoting sphere formation and promoting cyst formation;
(10) promoting organoid formation and promoting Cyst formation;
(11) promoting cell proliferation, promoting sphere formation and promoting organoid formation;
(12) promoting cell proliferation, promoting sphere formation and promoting cyst formation;
(13) promoting cell proliferation, promoting organoid formation and promoting Cyst formation;
(14) promoting sphere formation, promoting organoid formation and promoting Cyst formation; or
(15) promoting cell proliferation, promoting sphere formation, promoting organoid formation and promoting Cyst formation.

In the present specification, "promoting cell proliferation" means an increase in the number of cells by, for example, at least 5% or more, at least 10% or more, at least 20% or more, at least 30% or more, at least 40% or more,

at least 50% or more, at least 60% or more, at least 70% or more, at least 80% or more, at least 90% or more, at least 100% or more, at least 150% or more, or at least 200% or more, as compared with the number of a determined cell to be the control.

The composition of the present invention may contain one kind or a combination of two or more kinds of the compound of the present invention as an active ingredient.

In addition, the composition of the present invention optionally contains components other than the compound of the present invention. Such component is not particularly limited as long as the desired effect of the present invention is obtained, and includes, for example, water, saline, dimethyl sulfoxide (DMSO), glycerol, propylene glycol, butylene glycol, and various alcohols such as methanol, ethanol, butanol, propanol and the like, and the like. The composition of the present invention may be sterilized as necessary. The sterilization method is not particularly limited and, for example, radiation sterilization, ethylene oxide gas sterilization, autoclave sterilization, filter sterilization and the like can be mentioned. When filter sterilization (hereinafter sometimes to be referred to as filtration sterilization) is to be performed, the material of the filter part is not particularly limited and, for example, glass fiber, nylon, polyethersulfone (PES), hydrophilic polyvinylidene fluoride (PVDF), cellulose mixed ester, cellulose acetate, polytetrafluoroethylene and the like can be mentioned. While the size of the pore in the filter is not particularly limited, it is preferably 0.1 μm to 10 μm, more preferably 0.1 μm to 1 μm, most preferably 0.1 μm to 0.5 μm. The sterilization treatment may be applied when the composition is in a solid state or a solution state.

The amount of the compound of the present invention as an active ingredient in the composition of the present invention is not particularly limited as long as a medium (particularly, a three-dimensional cell culture medium) added with the composition of the present invention has a concentration that can exert the desired effect of the present invention. As the concentration at which the desired effect of the present invention can be exerted, for example, the lower limit of the concentration of the compound of the present invention in the medium (particularly, three-dimensional cell culture medium) is generally not less than 0.001 μM, preferably not less than 0.01 μM, more preferably not less than 0.1 μM, further preferably not less than 1 μM, particularly preferably not less than 10 μM. The upper limit of the concentration is generally not more than 100 μM, preferably not more than 50 μM, particularly preferably not more than 10 μM.

The composition of the present invention can have any shape during provision or preservation. The composition may be in the form of a formulated solid such as tablet, pill, capsule, granule, or a liquid such as a solution obtained by dissolving in an appropriate solvent using a solubilizer or a suspension, or may be bonded to a substrate or a carrier. Examples of the additive used formulating include preservatives such as p-oxybenzoic acid esters and the like; excipients such as lactose, glucose, sucrose, mannit and the like; lubricants such as magnesium stearate, talc and the like; binders such as poly(vinyl alcohol), hydroxypropylcellulose, gelatin and the like; surfactants such as fatty acid ester and the like; plasticizers such as glycerol and the like; and the like. These additives are not limited to those mentioned above, and can be selected freely as long as they are utilizable for those of ordinary skill in the art.

The cell type whose cell proliferation and the like are promoted by adding the composition of the present invention to a medium (particularly, three-dimensional cell culture medium) is not particularly limited as long as the desired effect is obtained. Examples thereof include cell types such as reproductive cells such as spermatozoon, oocyte and the like, somatic cells constituting the living body, normal cell line, cancer cell line, progenitor cells, stem cell, cells separated from the living body and applied with artificial genetic modification (e.g., gene transfer using virus and genetic modification by genome editing), cells separated from the living body wherein the nucleus is artificially exchanged and the like. While the derivation of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like are preferable. The tissue or organ from which the cells are derived is not particularly limited as long as the desired effect of the present invention can be obtained. Examples of the aforementioned tissue include tissues such as skin, kidney, spleen, adrenal gland, liver, lung, ovary, pancreas, uterus, stomach, colon, small intestine, large intestine, spleen, bladder, prostate, testis, thymus, muscle, connective tissue, bone, cartilage, vascular tissue, blood, heart, eye, brain, nerve tissue and the like. Examples of the aforementioned organ include, but are not limited to, organs such as liver, lung, kidney, heart, pancreas, stomach, spleen, small intestine, large intestine, reproductive organ, eye and the like. When the purpose is to promote organoid formation, the organoid may be preferably composed of cells derived from the small intestine or large intestine (colon). When the purpose is to promote Cyst formation, the Cyst may be preferably composed of cells derived from the kidney.

Examples of the normal cell lines include C3H10T1/2 (mouse embryonic fibroblast), HEK293 (human embryonic kidney cell), MDBK (bovine kidney-derived cell), MDCK (Canine kidney renal tubule epithelial cell), CHO-K1 (Chinese hamster ovary-derived cell), Vero (Cercopithecus aethiops kidney epithelium-derived cell), NIH3T3 (mouse fetal fibroblast), HepaRG (hepatocyte, registered trade mark), HUVEC (human umbilical vein endothelial cell), human primary culture hepatocyte, epidermal keratinocyte, corneal epithelial cell, corneal endothelium cell and the like. Among these, particularly MDCK, C3H10T1/2, CHO-K1, Vero, HUVEC, epidermal keratinocyte, corneal epithelial cell, corneal endothelium cell and NIH3T3 are preferable. Examples of the cancer cell line include, but are not limited to, HBC-4, BSY-1, BSY-2, MCF-7, MCF-7/ADR RES, HS578T, MDA-MB-231, MDA-MB-435, MDA-N, BT-549, T47D as human breast cancer cell lines, HeLa as human cervical carcinoama cell line, A549, EKVX, HOP-62, HOP-92, NCI-H23, NCI-H226, NCI-H322M, NCI-H460, NCI-H522, DMS273, DMS114 as human lung cancer cell line, Caco-2, COLO-205, HCC-2998, HCT-15, HCT-116, HT-29, KM-12, SW-620, WiDr as human colorectal cancer cell line, DU-145, PC-3, LNCaP as human prostate cancer cell line, U251, SF-295, SF-539, SF-268, SNB-75, SNB-78, SNB-19 as human central nervous system cancer cell line, OVCAR-3, OVCAR-4, OVCAR-5, OVCAR-8, SK-OV-3, IGROV-1 as human ovarian cancer cell line, RXF-631L, ACHN, UO-31, SN-12C, A498, CAKI-1, RXF-393L, 786-0, TK-10 as human kidney cancer cell line, MKN45, MKN28, St-4, MKN-1, MKN-7, MKN-74 as human gastric cancer cell line, LOX-IMVI, LOX, MALME-3M, SK-MEL-2, SK-MEL-5, SK-MEL-28, UACC-62, UACC-257, M14, A431 as skin cancer cell line, HuH-7, HepG2, PLC-PRF-5, SK-HEP-1 as human liver cell line, CCRF-CRM, K562, MOLT-4, HL-60TB, RPMI8226, SR, UT7/TPO, Jurkat as leukemia cell line. Among these, human ovarian cancer cell line SKOV3, human liver cell line HuH-7 and human epithelium-like cell cancer-derived cell line A431 are particularly preferable. Furthermore, stem cells are cells concurrently having the ability to replicate itself, and the ability to differentiate into other plural lineages. Examples thereof include, but are not limited to, embryonic stem cells (ES cells), embryonic tumor cells, embryonic germ stem cells, artificial pluripotent stem cells (iPS cells), neural stem cells, hematopoietic stem cells, mesenchymal stem cells (MSC), hepatic stem cells, pancreas stem cells, muscle stem cells, germ stem cells, intestinal stem cells, cancer stem cells, hair follicle stem cells and the like. Examples of the pluripotent stem cells include ES cells, embryonic germ stem cells and iPS cells, from among the aforementioned stem cells. Progenitor cells are cells on the way to differentiate from the aforementioned stem cells into particular somatic cells or reproductive cells. Among these, MSC and iPS cells are particularly preferable.

In one embodiment, when stem cells such as MSCs and the like are cultured using a three-dimensional cell culture medium added with the composition of the present invention, the cell proliferation thereof can be promoted while maintaining the characteristics (e.g., undifferentiated state) of the cells. Maintenance of the undifferentiated state of MSCs can be confirmed by analyzing expression of a cell surface marker by flow cytometry (FCM) (e.g., WO 2016/136986). Examples of the cell surface marker of MSC include CD29, CD73, CD90, CD105 and the like being positive.

In the present specification, the term "the composition of the present invention" can be replaced with the term "the medium additive agent of the present invention".

[Medium]

The present invention provides a medium containing the compound of the present invention or the composition of the present invention (hereinafter sometimes referred to as "the medium of the present invention"). Using the medium of the present invention, any or any combination of promoting cell proliferation, promoting sphere formation, promoting cyst formation, and promoting organoid formation can be achieved. The medium of the present invention is particularly preferably a three-dimensional cell culture medium.

The concentration of the compound of the present invention which is contained in the medium of the present invention as an active ingredient is not particularly limited as long as the desired effect of the present invention is obtained. For example, the lower limit of the concentration is generally not less than 0.001 μM, preferably not less than 0.01 μM, more preferably not less than 0.1 μM, further preferably not less than 1 μM, particularly preferably not less than 10 μM. The upper limit of the concentration is generally not more than 100 μM, preferably not more than 50 μM, particularly preferably not more than 10 μM.

The medium of the present invention can have the same composition as that of a known medium, except that the compound of the present invention or the composition of the present invention is blended.

In one embodiment, the medium of the present invention can be prepared by adding the compound or composition of the present invention to a commercially available medium (particularly three-dimensional cell culture medium). A commercially available medium that can be made into the medium of the present invention by adding the compound of the present invention or the composition of the present invention is not particularly limited as long as the desired effect is obtained. Examples of the medium include Dulbecco's Modified Eagle's medium (DMEM), HamF12 medium (Ham's Nutrient Mixture F12), DMEM/F12 medium, McCoy's 5A medium, Eagle MEM (Eagle's Minimum Essential medium; EMEM), αMEM (alpha Modified Eagle's Minimum Essential medium), MEM (Minimum Essential medium), RPMI1640 medium, Iscove's Modified Dulbecco's medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma-Aldrich), QBSF-60 (manufactured by Qualitybiological), StemPro hESC SFM (manufactured by Invitrogen), Essential 8 (registered trade mark) medium (manufactured by Gibco), mTeSR1 medium (manufactured by STEMCELL Technologies), mTeSR2 medium (manufactured by STEMCELL Technologies), ReproFF (manufactured by ReproCELL), ReproFF2 (manufactured by ReproCELL), StemFit (registered trade mark) AK02N (manufactured by Ajinomoto Co., Inc.), StemFit (registered trade mark) AK03N (manufactured by Ajinomoto Co., Inc.), PSGro hESC/iPSC

medium (manufactured by System Biosciences), NutriStem (registered trade mark) medium (manufactured by Biological Industries), CSTI-7 medium (manufactured by Cell Science & Technology Institute, Inc.), MesenPRO RS medium (manufactured by Gibco), MF- medium (registered trade mark) mesenchymal stem cell proliferation medium (manufactured by TOYOBO CO., LTD.), medium for mesenchymal stem cell (manufactured by PromoCell), Sf-900II (manufactured by Invitrogen), Opti-Pro (manufactured by Invitrogen) corneal epithelial cell basal medium (ATCC (registered trade mark), PCS-700-030 (registered trade mark)), Prigrow medium (manufactured by Applied Biological Materials), Basal medium Eagle, BGJb medium, CMRL1066 medium, keratinocyte basal medium 2 (manufactured by PromoCell), keratinocyte proliferation medium 2 (manufactured by PromoCell), normal human epidermal keratinocyte proliferation medium (manufactured by Kurabo Industries Ltd.), normal human epidermal keratinocyte basal medium (manufactured by Kurabo Industries Ltd.), human EpiVita proliferation medium (manufactured by TOYOBO CO., LTD.), EpiLife (registered trade mark) medium (manufactured by Thermo Fisher Scientific), corneal epithelial cell basal medium (manufactured by ATCC, #PCS-700-030), IntestiCult Organoid Growth medium (manufactured by STEMCELL Technologies), STEMdiff Cerebral Organoid Kit (manufactured by STEMCELL Technologies), STEMdiff Cerebral Organoid Maturation Kit (manufactured by STEMCELL Technologies), HepatiCult Organoid Growth medium (manufactured by STEMCELL Technologies), PancreaCult Organoid Growth medium (manufactured by STEMCELL Technologies), PneumaCult-ALI (manufactured by STEMCELL Technologies), STEMdiff APEL2 (manufactured by STEMCELL Technologies) and the like. In addition, a three-dimensional cell culture medium obtained by adding polysaccharides such as deacylated gellan gum and the like to these media can be used. Examples of such three-dimensional cell culture medium include, but are not limited to, FCeM (registered trade mark) (manufactured by FUJIFILM Wako Pure Chemical Corporation).

In addition, it is possible to add, according to the object, sodium, potassium, calcium, magnesium, phosphorus, chlorine, various amino acids, various vitamins, antibiotics, serum, fatty acids, sugars, cell growth factors, differentiation inducing factors, cell adhesion factors, antibodies, enzymes, cytokines, hormones, lectins, extracellular matrices, bioactive substances, and the like to the above-mentioned medium. Specific examples include, but are not limited to, basic fibroblast growth factor, transforming growth factor-$\alpha$, transforming growth factor-$\beta$, epidermal growth factor, insulin-like growth factor, Wnt protein, amphiregulin, interferons, interleukins, vascular endothelial cell growth factor, platelet derived from growth factor, hepatocyte growth factor, albumin, insulin, $\beta$-mercaptoethanol, Knockout Serum Replacement (KSR, manufactured by Thermo Fisher Scientific), Glutamax (manufactured by Thermo Fisher Scientific), hydrocortisone, epinephrine, L-glutamine, pyruvic acid, selenous acid, Rho-associated protein kinase (ROCK) inhibitor and the like.

When cells are cultivated in the medium of the present invention (particularly three-dimensional cell culture), culture vessels generally used for cell culture such as schales, flasks, plastic bags, Teflon (registered trade mark) bags, dishes, petri dishes, dishes for tissue culture, multidishes, microplates, microwell plates, multiplates, multiwell plates, chamber slides, tubes, trays, culture bags, roller bottles and the like can be used for cultivation. These culture containers may be coated with an extracellular matrix such as collagen, gelatin, laminin, iMatrix-511, iMatrix-411, iMatrix-221, fibronectin, vitronectin, Matrigel (registered trade mark), poly-L-ornithine/laminin, poly-D-lysine or the like. When a cell aggregate (sphere) is formed, these culture containers are desirably low cell-adhesive so that the (adherent) cells to be cultured will not adhere to the culture container. As a cell-nonadhesive culture vessel, a culture vessel having a surface not artificially treated to improve adhesiveness to cells (e.g., coating treatment with extracellular matrix and the like), or a culture vessel having a surface artificially treated to reduce adhesiveness to cells can be used. Examples of such container include, but are not limited to, Sumilon celltight plate (manufactured by SUMITOMO BAKELITE CO., LTD.), PrimeSurface (registered trade mark) plate (manufactured by SUMITOMO BAKELITE CO., LTD.), Ultra-low Attachment surface plate (manufactured by Corning Incorporated), Nunclon Spheraplate (manufactured by Thermo Fisher Scientific) and the like.

[Cell proliferation promoting method, sphere formation promoting method, cyst formation promoting method, and organoid formation promoting method]

The present invention provides a method for promoting cell proliferation, a method for promoting sphere formation, a method for promoting cyst formation or a method for promoting organoid formation (hereinafter these are sometimes collectively referred to as "the method of the present invention"), each including adding the compound of the present invention or the composition of the present invention to a medium.

The medium to be used in the method of the present invention is not particularly limited as long as the desired effect is obtained. Preferred is a three-dimensional cell culture medium. The cell culture conditions (e.g., temperature, carbon dioxide concentration, culture period etc.) used in the method of the present invention may be those for a method known per se, or may be appropriately modified according to the purpose. For example, the temperature for culturing cells in the case of animal cells is generally 25°C - 39°C, preferably 33°C - 39°C (e.g., 37°C). The carbon dioxide concentration is generally 4% by volume - 10% by volume, preferably 4% by volume - 6% by volume, in the atmosphere of culture. The culture period is generally 1 to 35 days, which can be appropriately set according to the purpose of the culture.

A method for forming a cell aggregate (sphere) is not particularly limited, and can be appropriately selected by those of ordinary skill in the art. Examples thereof include a method using a container having a cell non-adhesive surface, hanging drop method, gyratory culture method, three-dimensional scaffold method, centrifugation method, a method using aggregation by an electric field or magnetic field and the like. For example, using a method using a container having a cell non-adhesive surface, the target cells are cultured in a culture container applied with a surface treatment to inhibit cell adhesion, whereby a sphere can be formed. When such cell non-adhesive culture container is used, the target cells are first collected, a cell suspension thereof is prepared and plated in the culture container to perform culture. When culture is continued for about 1 week, the cells spontaneously form a sphere. As a cell non-adhesive surface used here, a surface of a culture container generally used such as schale and the like, which is coated with a substance inhibiting cell adhesion and the like can be used. Examples of such substance include agarose, agar, copolymer of poly-HEMA(poly-(2-hydroxl-ethylmethacrylate)2-methacryloyloxyethylphosphoryl choline and other monomer (e.g., butylmethacrylate etc.), poly(2-methoxymethylacrylate), poly-N-isopropylacrylamide, mebiol gel (registered trade mark) and the like. When cytotoxicity is absent, the substance is not limited thereto.

As a method for forming a cell aggregate (sphere), the methods described in Nature Biotechnology, 2010, Vol.28, No.4, p.361-366, Nature Protocols, 2011, Vol.6, No.5, p.689-700, Nature Protocols, 2011, Vol.6, No.5, p.572-579, Stem Cell Research, 2011, Vol.7, p.97-111, Stem Cell Reviews and Reports, 2010, Vol.6, p.248-259 and the like can also be used.

In addition, a medium used for culture for forming a sphere can also contain a component that promotes formation of a sphere or promotes maintenance thereof. Examples of the component having such effect include dimethyl sulfoxide, superoxide dismutase, caeruloplasmin, catalase, peroxidase, L-ascorbic acid, L-ascorbic acid phosphate, tocopherol, flavonoid, uric acid, bilirubin, selenium-containing compound, transferrin, unsaturated fatty acid, albumin, theophylline, forskolin, glucagon, dibutyryl cAMP and the like. As the selenium-containing compound, sodium selenite, sodium selenate, dimethyl selenide, hydrogen selenide, selenomethionine, Semethylselenocysteine, selenocystathionine, selenocysteine, selenohomocysteine, adenosine-5'-phosphoselenoic acid, Seadenosylselenomethionine can be mentioned. In addition, examples of the component having the above-mentioned effect include Rho-associated protein kinase (ROCK) inhibitors such as Y-27632, Fasudil (HA1077), H-1152, Wf-536 and the like. To obtain the desired cell aggregate having a uniform size, plural concaves having the same diameter as the desired cell aggregate can also be introduced onto a cell non-adhesive culture container to be used. When these concaves are in contact with each other or within the range of the diameter of the desired cell aggregate, and cells are plated, the plated cells do not form a cell aggregate between concaves but certainly form a cell aggregate with a size corresponding to the volume thereof in the concave, thus affording a cell aggregate population having a uniform size. As the shape of the concave in this case is preferably a hemisphere or cone.

Alternatively, a sphere can also be formed based on a support showing cell adhesiveness. Examples of such support include collagen, polyrotaxane, polylactic acid (PLA), polylactic acid glycolic acid (PLGA) copolymer, hydrogel and the like.

In addition, a sphere can also be formed by co-cultivating with a feeder cell. As a feeder cell to promote sphere formation, any adhesive cell can be used. Preferably, a feeder cell for each kind of cell is desirable. Although not limited, for example, when a sphere of cells derived from the liver or cartilage is formed, examples of the feeder cells include COS-1 cells and vascular endothelial cells as preferable cell types.

Alternatively, a hanging drop method can also be selected as a method for forming a sphere. As the hanging drop method, for example, a method including spotting a droplet (about 10 - 50 μL in volume) of a cell suspension on the ceiling side such as a lid of a culture vessel, and culturing in an inverted state such that the placed droplet hangs can be mentioned. By culturing in this manner, the cells are minimally influenced by a contact with the flat surface and form a sphere at the bottom of the droplet. Such droplet can also be prepared using a special culture vessel such as GravityPLUS Plate (manufactured by PerkinElmer). Specifically, a sphere can be prepared using a droplet containing 100 - 100000 cells, preferably 200 - 10000 cells, more preferably 500 - 10000 cells. To form spheres, it is preferable to culture for 6 -48 hr.

The size of the sphere varies depending on the cell type and culture period and is not particularly limited. When it has a spherical shape or ellipse spherical shape, the diameter thereof is 20 μm to 1000 μm, preferably 40 μm to 500 μm, more preferably 50 μm to 300 μm, most preferably 80 μm to 200 μm.

Such sphere can maintain proliferative capacity for not less than 10 days, preferably not less than 13 days, more preferably not less than 30 days, by continuing the standing culture. By regularly further performing, during the standing culture, mechanical division, or a single cell -forming treatment and aggregation, the proliferative capacity can be maintained substantially infinitely.

The culture container to be used for culturing sphere is not particularly limited as long as it generally permits animal cell culture. For example, flasks, dishes, petri dishes, tissue culture dishes, multidishes, microplates, microwell plates, multiplates, multiwell plates, chamber slides, cell culture flasks, spinner flasks, schales, tubes, trays, culture bags, roller bottles, EZSPHERE (manufactured by AGC TECHNO GLASS CO., LTD.), Sumilon celltight plates (manufactured by SUMITOMO BAKELITE CO., LTD.) and the like can be mentioned.

Of these culture containers, microplates, microwell plates, multiplates and multiwell plates are preferably used when evaluation of many pharmaceutical product candidate compounds or pharmaceutical products is performed. While the well bottom shape of these plates is not particularly limited, flat bottom, U-shaped bottom and V-shaped bottom can be used, and U-shaped bottom is preferably used. While the materials of these culture tools are not particularly limited, for example, glass, plastics such as polyvinyl chloride, cellulosic polymers, polystyrene, polymethylmethacrylate, polycarbonate, polysulfone, polyurethane, polyester, polyamide, polystyrene, polypropylene and the like, and the like can be mentioned.

The medium used for embedding culture can contain a cell adhesion factor, and examples thereof include Matrigel (registered trade mark), Geltrex (registered trade mark), collagen, gelatin, poly-L-lysine, poly-D-lysine, laminin, iMatrix-511, iMatrix-411, iMatrix-221, fibronectin, vitronectin, tenascin, selectin, hyaluronic acid, fibrin and the like. Two or more kinds of these cell adhesion factors can also be added in combination. Furthermore, the medium to be used for embedding culture can be mixed with a thickener such as agar, guar gum, tamarind gum, propylene glycol alginate, locust bean gum, gum arabic, tara gum, tamarind gum, methylcellulose, carboxymethylcellulose, agarose, tamarind seed gum, pullulan and the like. Two or more kinds of these thickeners can also be added in combination.

A method for forming an organoid (mini-organ formed by culturing stem cells or progenitor cells in vitro in a three-dimensional environment) or Cyst (luminal structure formed by epithelial cells) is not particularly limited, and can be appropriately selected by those of ordinary skill in the art. As an example, a method using the above-mentioned embedding culture can be mentioned. Specifically, an organoid or cyst (Cyst) can be formed by culturing target cells or tissues in medium for embedding culture containing the above-mentioned cell adhesion factor. As used herein, a stem cell or progenitor cell collected from a tissue, a pluripotent stem cell, or the like is desirable as the cell from which the organoid is derived. For example, after target cell or tissue is collected, a suspension thereof is prepared, and the suspension is seeded in a medium for embedding culture and cultured. After culturing for 3 to 45 days, the cells spontaneously form an organoid or cyst (Cyst).

The medium used in the method of the present invention (particularly three-dimensional cell culture medium) may be the medium of the present invention.

The concentration of the compound of the present invention or the composition of the present invention, cell type, and the like in the method of the present invention are the same as those described in the above-mentioned "2. Composition for addition to a medium".

[Cell adhesion promoting method]

The present invention provides a method for promoting cell adhesion, including adding the composition of the present invention to a medium (hereinafter sometimes referred to as "the cell adhesion promoting method of the present invention"). The cell adhesion promoting method of the present invention can promote adhesion of a cell (particularly adherent cell) to a culture container.

A medium to be used in the cell adhesion promoting method of the present invention is not particularly limited as long as a desired effect is obtained. The cell culture conditions (e.g., temperature, carbon dioxide concentration, culture period etc.) in the cell adhesion promoting method of the present invention may be those known per se, or may be appropriately modified according to the purpose. For example, the temperature for culturing cells is generally 25°C - 39°C, preferably 33°C - 39°C (e.g., 37°C) in the case of animal cell. The carbon dioxide concentration is generally 4% by volume - 10% by volume, preferably 4% by volume - 6% by volume, in the atmosphere of culture. The culture period is generally 1 to 45 days, which can be appropriately set according to the purpose of the culture.

A medium to be used in the cell adhesion promoting method of the present invention may be the medium of the present invention.

The concentration of the compound to be the aforementioned active ingredient, preferable cell type and the like in the cell adhesion promoting method of the present invention are the same as those explained in the aforementioned [Composition for addition to a medium] and [Medium].

[Production method of cell for transplantation or organoid for transplantation]

The present invention also provides a production method of a cell for transplantation or organoid for transplantation, including adding a medium containing an effective amount of a compound represented by the aforementioned formula (I) or a salt thereof (hereinafter sometimes to be referred to as "the production method of the present invention"). Examples of the cell for transplantation include, but are not limited to, liver cell, skin cell, osteoblast, chondrocyte, mesenchymal stem cell, pancreatic B cell, nerve cell, retinal cell, corneal epithelial cell and corneal endothelium cell. For example, a corneal epithelial cell and a corneal endothelial cell obtained by the production method of the present invention can be preferably used as a cell for transplantation in treating corneal diseases. Examples of the organoid for

transplantation include organoids of cerebrum, cerebellum, thyroid gland, thymus, testis, liver, pancreas, small intestine, large intestine, colon, epithelial, lung, kidney and the like. For example, an organoid of large intestine or colon obtained by the production method of the present invention can be preferably used as an organoid for transplantation in treating bowel diseases.

The compound represented by the formula (I), an amount thereof to be added, medium, culture container, cell or organoid to be cultured, culture method of cell or organoid, and the like in the production method of the present invention are the same as those described in the aforementioned [Composition for addition to a medium], [Medium] and [Cell proliferation promoting method, sphere formation method, cyst formation promoting method and organoid formation promoting method].

The cell culture conditions (e.g., temperature, carbon dioxide concentration, culture period etc.) in the production method of the present invention may be those for a method known per se, or may be appropriately modified according to the purpose. For example, the temperature for culturing cells or organoid in the case of animal cells or organoids is generally 25°C - 39°C, preferably 33°C - 39°C (e.g., 37°C) . The carbon dioxide concentration is generally 4% by volume - 10% by volume, preferably 4% by volume - 6% by volume, in the atmosphere of culture. The culture period is generally 1 to 45 days, which can be appropriately set according to the purpose of the culture.

[Kinase inhibitor]

The present invention provides a kinase inhibitor containing the compound of the present invention (hereinafter sometimes referred to as "the kinase inhibitor of the present invention").

The amount of the compound of the present invention which is contained in the kinase inhibitor of the present invention is not particularly limited as long as the desired effect of the present invention is obtained. It may be generally 0.01 - 100 wt%, preferably 0.1 - 100 wt%, more preferably 1 - 100 wt%, further preferably 5 - 100 wt%, particularly preferably 10 - 100 wt%.

The kinase inhibitor of the present invention can have any shape during provision or preservation. The composition may be in the form of a formulated solid such as tablet, pill, capsule, granule, or a liquid such as a solution obtained by dissolving in an appropriate solvent using a solubilizer or a suspension, or may be bonded to a substrate or a carrier. Examples of the solvent used for formulating include aqueous solvents such as water, saline, dimethyl sulfoxide (DMSO), various alcohols (e.g., methanol, ethanol, butanol, propanol, glycerol, propylene glycol, butylene glycol and the like), and the like. Examples of the additive used formulating include preservatives such as p-oxybenzoic acid esters and the like; excipients such as lactose, glucose, sucrose, mannit and the like; lubricants such as magnesium stearate, talc and the like; binders such as poly(vinyl alcohol), hydroxypropylcellulose, gelatin and the like; surfactants such as fatty acid ester and the like; plasticizers such as glycerol and the like; and the like. These additives are not limited to those mentioned above, and can be selected freely as long as they are utilizable for those of ordinary skill in the art.

The kinase inhibitor of the present invention may be sterilized as necessary. The sterilization method is not particularly limited, and for example, radiation sterilization, ethylene oxide gas sterilization, autoclave sterilization, filter sterilization and the like can be mentioned. When filter sterilization (hereinafter sometimes to be referred to as filtration sterilization) is to be performed, the material of the filter part is not particularly limited and for example, glass fiber, nylon, polyethersulfone (hereinafter to be abbreviated as PES), hydrophilic polyvinylidene fluoride (hereinafter to be abbreviated as PVDF), cellulose mixed ester, cellulose acetate, polytetrafluoroethylene and the like can be mentioned. While the size of the pore in the filter is not particularly limited, it is preferably 0.1 μm to 10 μm, more preferably 0.1 μm to 1 μm, most preferably 0.1 μm to 0.5 μm. The sterilization treatment may be applied when the specific compound is in a solid state or a solution state.

In the kinase inhibitor of the present invention, "inhibiting kinase" means inhibiting the phosphorylation function of kinase to make its activity disappear or attenuate. Examples of the kind of kinase to be inhibited by the kinase inhibitor of the present invention include, but are not limited to, LATS1 and LATS2. In the present specification, "attenuating the activity of kinase" means, for example, attenuating the activity of kinase by at least 5% or more, at least 10% or more, at least 15% or more, at least 20% or more, at least 25% or more, at least 30% or more, at least 35% or more, at least 40% or more, at least 50% or more, at least 55% or more, at least 60% or more, at least 65% or more, at least 70% or more, at least 75% or more, at least 80% or more, at least 85% or more, at least 90% or more, at least 95% or more, or at least 99% or more, as compared with the phosphorylation activity level of a given kinase to be the control. For the measurement of the kinase activity, a method known per se such as Adenosine diphosphite (hereinafter to be abbreviated as ADP) quantification method, Mobility Shift Assay (hereinafter to be abbreviated as MSA) method and the like can be used as explained in the following Examples. Alternatively, when the kinase is LATS2 or the like located at the upstream of the Hippo signal transduction pathway, the degree of attenuation of the kinase activity can also be indirectly evaluated by confirming the intracellular localization of YAP and/or TAZ, which are/is downstream effector(s) of LATS2 or the like and the target of phosphorylation, by a molecular biological method known per se.

In one embodiment of the present invention, when the kinase is LATS2, "inhibiting LATS2" means inhibiting the function of LATS2 as kinase to make its activity disappear or attenuate. LATS2 is evolutionarily conserved in a wide range of species from yeast to human. Therefore, LATS2 whose activity is inhibited by the kinase inhibitor of the present invention can be LATS2 in all organisms, and may be LATS of mammals such as rat, mouse, rabbit, guinea pig, squirrel, hamster, vole, duckbill, dolphin, whale, dog, cat, goat, bovine, horse, sheep, swine, elephant, Common marmoset, squirrel monkey, rhesus monkey, chimpanzee, human and the like. LATS2 to be inhibited by the kinase inhibitor of the present invention may be preferably human LATS. When simply referred to as "LATS" in the present specification, it is a concept including both "LATS1" and "LATS2" and a complex of LATS1 and LATS2.

Examples of the embodiment of use of the kinase inhibitor of the present invention include, but are not limited to, use for molecular biological tests and research purposes, and the like. In one embodiment of the use for molecular biological tests and research purposes, the kinase inhibitor of the present invention is administered to a cell that expresses a specific kinase such as LATS2 or the like, whereby a cell showing suppressed activity of the specific kinase can be easily prepared. The thus-obtained cells may be useful for functional analysis of a specific kinase, screening of candidate substances capable of regulating the function of the specific kinase, and the like.

In one embodiment, a cell expressing a specific kinase may be a cell derived from a mammal. Examples thereof include, but are not limited to, somatic cells constituting the living body, normal cell line, cancer cell line, progenitor cells, stem cell, cells separated from the living body and applied with artificial genetic modification (e.g., gene transfer using virus and genetic modification by genome editing), cells separated from the living body wherein the nucleus is artificially exchanged and the like. The derivation of these cells is not particularly limited and the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like can be mentioned. The tissue or organ from which the cells are derived is not particularly limited. Examples of the tissue include tissues such as skin, kidney, spleen, adrenal gland, liver, lung, ovary, pancreas, uterus, stomach, colon, small intestine, large intestine, bladder, prostate, testis, thymus, muscle, connective tissue, bone, cartilages, vascular tissue, blood, heart, eye, brain, nerve tissue and the like. Examples of the organ include organs such as liver, lung, kidney, heart, pancreas, stomach, spleen, small intestine, large intestine, reproductive organ, eye and the like. In the present specification, the "stem cells" mean cells concurrently having the ability to replicate itself, and the ability to differentiate into other plural lineages. Examples thereof include, but are not limited to, embryonic stem cells (ES cells), embryonic tumor cells, embryonic germ stem cells, artificial pluripotent stem cells (iPS cells), neural stem cells, hematopoietic stem cells, mesenchymal stem cells, hepatic stem cells, pancreas stem cells, muscle stem cells, germ stem cells, intestinal stem cells, cancer stem cells, hair follicle stem cells and the like. Examples of the pluripotent stem cells include ES cells, embryonic germ stem cells and iPS cells, from among the aforementioned stem cells. Progenitor cells are cells on the way to differentiate from the aforementioned stem cells into particular somatic cells or reproductive cells.

The kinase inhibitor of the present invention is administered to a cell that expresses a specific kinase by adding the kinase inhibitor of the present invention to a medium in which the cell is cultured. The amount of the kinase inhibitor of the present invention to be added is not particularly limited as long as the desired effect of the present invention is achieved. For example, the concentration of the compound of the present invention in the medium is generally 0.001 - 100 $\mu$M, preferably 0.01 M - 50 $\mu$M, more preferably 0.1 - 30 $\mu$M, further preferably 1 - 20 $\mu$M, particularly preferably 5 - 10 $\mu$M.

When the kinase inhibitor of the present invention is used, the medium in which the cells are cultured is not particularly limited, and a commercially available medium can also be used. Preferable examples of the commercially available medium include, but are not limited to, the media described in the aforementioned [Medium].

In addition, it is possible to appropriately add, according to the purposes of test and research, further substances such as sodium, potassium, calcium, magnesium, phosphorus, chlorine, various amino acids, various vitamins, antibiotics, serum, fatty acids, sugars, cell growth factors, differentiation inducing factors, cell adhesion factors, antibodies, enzymes, cytokines, hormones, lectins, extracellular matrices, bioactive substances, and the like to the above-mentioned medium.

The cell culture conditions (e.g., temperature, carbon dioxide concentration, culture period etc.) may be those known per se, or may be appropriately modified according to the purpose. For example, the temperature for culturing cells is generally 25°C - 39°C, preferably 33°C - 39°C (e.g., 37°C). The carbon dioxide concentration is generally 4% by volume - 10% by volume, preferably 4% by volume - 6% by volume, in the atmosphere of culture. The culture period is generally 1 to 35 days, which can be appropriately set according to the purpose of the culture.

[Hippo signal transduction pathway inhibitor]

The present invention also provides a Hippo signal transduction pathway inhibitor containing the compound of the present invention (hereinafter sometimes referred to as "the Hippo signal transduction pathway inhibitor of the present invention").

Hippo signal transduction pathway is a signal transduction pathway involved in cell proliferation, cell death, organ size, self-renewal, differentiation of stem cell and progenitor cell, wound therapy, and tissue regeneration. This pathway is evolutionarily conserved in various kinds of organisms, particularly highly conserved in mammals. Key factors in the Hippo signal transduction pathway in mammals include LATS1 and LATS2. LATS is activated by association with the scaffold protein Mob1A/B. LATS is also activated by phosphorylation by STE20 family protein kinases Mst1 and Mst2. LATS phosphorylates downstream effectors YAP and TAZ which are transcription co-factors. Phosphorylation of YAP and TAZ by LATS is a very important event in the Hippo signal transduction pathway. Phosphorylated YAP and TAZ transfer from the cell nucleus to the cytoplasm and are degraded by the ubiquitin proteasome system. Therefore, when the Hippo signal transduction pathway is in an activated state, YAP and/or TAZ are/is phosphorylated by LATS, transferred into the cytoplasm and degraded. On the other hand when the Hippo signal transduction pathway is inactive, YAP and/or TAZ are/is not phosphorylated and are localized in the cell nucleus.

As described above, YAP and/or TAZ are/is transcription co-factor(s), and are known to induce the expression of various genes when localized in the cell nucleus. Many of the genes whose expression is induced by YAP and/or TAZ are known to mediate cell survival and proliferation. Examples of such gene include, but are not limited to, CCND1, CTGF, BIRC2, CYR61, AMOTL2, TGFB2, ANKRD1 and the like. The CCND1 gene (also referred to as CYCLIN D1, "PRAD1", etc.) is present on chromosome 11 in human. The protein encoded by the gene (295 amino acids, molecular weight: about 36 kDa) relates to promoting the cell cycle. The CTGF gene (connective tissue growth factor, also referred to as "CCN2", etc.) is present on chromosome 6 in human and encodes a polypeptide (349 amino acids, molecular weight: about 35 kDa) that induces promotion of cell growth and the like. The BIRC2 gene (baculoviral IAP repeat containing 2, also referred to as "API1", etc.) is present on chromosome 11 in human and encodes a protein that inhibits apoptosis by caspase-degrading activity (618 amino acids, molecular weight: about 70 kDa). The CYR61 gene (cysteine rich angiogenic inducer 61, also referred to as "CCN1", etc.) is present on chromosome 1 in human. The protein encoded by the gene (381 amino acids, molecular weight: about 39 kDa) plays an important role in angiogenesis, VEGF enhancement, and bone formation. The AMOTL2 gene (angiomotin like 2, also referred to as "LCCP") is present on chromosome 3 (3q22.2) in human. The protein encoded by the gene (837 amino acids, molecular weight: about 92 kDa) relates to angiomotin, which inhibits angiogenesis, and belongs to the motin protein family. The ANKRD1 gene (Ankyrin repeat domain-containing protein 1, Cardiac adriamycin-responsive protein, also referred to as "CARP") is present on chromosome 10 in human. The protein encoded by the gene (319 amino acids, molecular weight: about 36 kDa) is highly expressed in cardiac muscle and skeletal muscle and functions as a transcription factor.

The Hippo signal transduction pathway inhibitor of the present invention inhibits the kinase activity of LATS, and inhibits the phosphorylation of YAP and/or TAZ by LATS. Since YAP and/or TAZ are/is not phosphorylated, the nuclear translocation of YAP and/or TAZ is promoted. As a result, gene expression of genes whose expression is induced by YAP and/or TAZ is enhanced. Therefore, the Hippo signal transduction pathway inhibitor of the present invention can also be paraphrased as "nuclear translocation promoter of YAP and/or TAZ", or "promoter of gene expression induced by YAP and/or TAZ".

In the present specification, "promoting nuclear translocation of YAP and/or TAZ" means that the abundance of YAP and/or TAZ is increased in the nucleus rather than in the cytoplasm by inhibiting phosphorylation and proteolysis of YAP and/or TAZ. Increasing the abundance of YAP and/or TAZ means that, for example, the abundance of YAP and/or TAZ in the nucleus increases at least 1.3 times or more, at least 1.5 times or more, at least 2 times or more, at least 3 times or more, at least 4 times or more, at least 5 times or more, at least 6 times or more, at least 7 times or more, at least 8 times or more, at least 9 times or more, at least 10 times or more, as compared with the abundance of YAP and/or TAZ to be the control in the nucleus. The abundance of YAP and/or TAZ in the nucleus can be measured by a method known per se such as imaging analysis using an anti-YAP (or TAZ) antibody bound with a fluorescent label, as used in Examples described later, and the like. In addition, the abundance of YAP and/or TAZ in the nucleus can be indirectly measured by observing the phosphorylation state of YAP and/or TAZ by using a Western blotting method or the like.

In the present specification, "promotion of gene expression induced by YAP and/or TAZ" means that, as a result of promotion of transfer of YAP and/or TAZ into the cell nucleus, the level of gene expression induced by YAP and/or TAZ (e.g., at least one or more of CCND1, CTGF, BIRC2, CYR61, AMOTL2, TGFB2, ANKRD1) is promoted. That the gene expression is promoted means, for example, that the expression level of at least one or more genes of the above-mentioned genes increases at least 1.3 times or more, at least 1.5 times or more, at least 2 times or more, at least 3 times or more, at least 4 times or more, at least 5 times or more, at least 6 times or more, at least 7 times or more, at least 8 times or more, at least 9 times or more, at least 10 times or more, as compared with the expression level of a given control amount (e.g., cell population free of addition of the Hippo signal transduction pathway inhibitor of the present invention). The promotion of gene expression may be evaluated at the transcription level by using a microarray method, a real-time PCR method, or the like, or at the protein translation level by using a Western blotting method or the like.

The amount, dosage form, sterilization treatment and the like of the compound of the present invention in the

Hippo signal transduction pathway inhibitor of the present invention are similar to those explained in 1. Kinase inhibitor of the present invention.

Examples of the embodiment of use of the Hippo signal transduction pathway inhibitor of the present invention include, but are not limited to, use for molecular biological tests and research purposes, and the like. As a specific example of the use for molecular biological tests and research purposes, a cell with inhibited Hippo signal transduction pathway can be easily prepared by administering the Hippo signal transduction pathway inhibitor of the present invention to a cell having the Hippo signal transduction pathway. As described above, the Hippo signal transduction pathway inhibitor of the present invention promotes transfer of YAP and/or TAZ into the cell nucleus, so that the phenotype of the disease involving YAP and/or TAZ appears more clearly in the cell. Therefore, by using cells having such properties, it is possible to efficiently search for a substance that can regulate the functions of YAP and/or TAZ.

In one embodiment, a cell having Hippo signal transduction pathway may be a cell derived from a mammal. Examples of such cell include, but are not limited to, somatic cells constituting the living body, normal cell line, cancer cell line, progenitor cells, stem cell, cells separated from the living body and applied with artificial genetic modification, cells separated from the living body wherein the nucleus is artificially exchanged and the like. While the derivation of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, rhesus monkey, chimpanzee, human and the like can be mentioned. The tissue or organ from which the cells are derived is not particularly limited. Examples of the tissue include tissues such as skin, kidney, spleen, adrenal gland liver, lung, ovary, pancreas, uterus, stomach, colon, small intestine, large intestine, bladder, prostate, testis, thymus, muscle, connective tissue, bone, cartilages, vascular tissue, blood, heart, eye, brain, nerve tissue and the like. Examples of the organ include organs such as liver, lung, kidney, heart, pancreas, stomach, spleen, small intestine, large intestine, reproductive organ, eye and the like.

The Hippo signal transduction pathway inhibitor of the present invention is administered to a cell having Hippo signal transduction pathway by adding the Hippo signal transduction pathway inhibitor of the present invention to a medium in which the cell is cultured. The amount of the kinase inhibitor of the present invention to be added is not particularly limited as long as the desired effect of the present invention is achieved. For example, the concentration of the compound of the present invention in the medium is generally 0.001 - 100 $\mu$M, preferably 0.01 - 50 $\mu$M, more preferably 0.1 - 30 $\mu$M, further preferably 1 - 20 $\mu$M, particularly preferably 5 - 10 $\mu$M.

When the Hippo signal transduction pathway inhibitor of the present invention is used, the medium in which the cells are cultured is not particularly limited, and a commercially available medium can also be used. The commercially available medium that can be used when using the Hippo signal transduction pathway inhibitor of the present invention is the same as the commercially available medium that can be used when using the kinase inhibitor of the present invention. In addition, the cell culture conditions and the like are also the same as those described for the kinase inhibitor of the present invention.

[Pharmaceutical composition]

The present invention also provides a pharmaceutical composition containing the compound of the present invention (hereinafter sometimes referred to as "the pharmaceutical composition of the present invention").

Hippo signal transduction pathway is a signal transduction pathway involved in cell proliferation, cell death, organ size, self-renewal, differentiation of stem cell and progenitor cell, wound therapy, and tissue regeneration. Particularly, cell proliferation, wound therapy, and tissue regeneration were promoted by inhibiting the Hippo signal transduction pathway by inhibiting kinases such as LATS and the like. Therefore, the pharmaceutical composition of the present invention containing the compound of the present invention having a kinase inhibitory activity can also be used for diseases associated with failure of cell proliferation and regeneration of damaged tissues.

The amount, sterilization treatment and the like of the compound of the present invention in the pharmaceutical composition of the present invention are similar to those explained in the above-mentioned [Kinase inhibitor].

The pharmaceutical composition of the present invention may be used alone or may be used in combination with at least one kind of other therapeutic drug. In this case, the pharmaceutical composition of the present invention may be administered simultaneously with the therapeutic drug, or may be administered prior to or after the administration of the therapeutic drug. The pharmaceutical composition of the present invention may be administered by an administration route the same as or different from that of the therapeutic drug. Examples of the therapeutic drug include chemotherapy drugs, supporting therapeutic drugs, and a combination thereof.

In the present specification, "treating" means partially or substantially achieving one or more of partially or completely reducing the extent of a disease, improving or ameliorating clinical symptoms or indices related to a disease, slowing, suppressing, or preventing the progression of a disease, and partially or completely slowing, suppressing, or preventing the onset or progression of a disease.

The animals to which the pharmaceutical composition of the present invention is applied is generally mammal,

preferably human, or may be pets (e.g., dog, cat, mouse, rat, guinea pig, rabbit, squirrel etc.) or domestic animals (bovine, sheep, swine, horse etc.). Preferably, the target of application of the pharmaceutical composition of the present invention is a human.

In addition, the pharmaceutical composition of the present invention may take any shape during provision or preservation. The pharmaceutical composition of the present invention can be generally administered as oral administration agents such as tablet, capsule, powder, granule, pill, syrup and the like, eye drop, ophthalmic ointment, percutaneous absorber, ophthalmic injection, rectal administration agent, percutaneous absorber, muscle injection, intravenous injection or subcutaneous injection. The agent can be administered as a single therapeutic drug or as a mixture with other therapeutic drugs. They may be administered alone, or are generally administered in the form of pharmaceutical compositions. These preparations can be produced by a conventional method by adding pharmacologically and pharmaceutically acceptable additives. That is, additives such as general excipient, lubricant, binder, disintegrant, wetting agent, plasticizer, coating agent and the like can be used for the oral preparation. Oral solution may be in the form of aqueous or oily suspension, solution, emulsion, syrup, elixir, or the like, or may be provided as dry syrup to be prepared with water or other suitable solvent prior to use. The aforementioned liquids can contain general additives such as suspending agent, flavor, diluent and emulsifier. For intrarectal administration, they can be administered as suppository. Suppositories use suitable substances such as cocoa butter, lauric oil, macrogol, glycerol gelatin, Witepsol, sodium stearate or mixtures thereof as a base, and emulsifier, suspending agent, preservative and the like can be added as necessary. For intravenous or subcutaneous injections, preparation components such as dissolving agent or solubilizing agent (e.g., distilled water for injection, physiological saline, 5% glucose solution, propylene glycol, and the like), pH adjuster, isotonicity agent, stabilizer and the like are used to form an aqueous dosage form or a dosage form for dissolution at the time of use. For ophthalmic injections, preparation components such as dissolving agent or solubilizing agent (e.g., distilled water for injection, physiological saline, 5% glucose solution, propylene glycol, and the like), pH adjuster, isotonicity agent, stabilizer and the like are used to form an aqueous dosage form or a dosage form for dissolution at the time of use. The ophthalmic ointment can be prepared using a general-purpose base material such as white petrolatum, liquid paraffin and the like. The eye drop can be prepared using as necessary an isotonicity agent such as sodium chloride, potassium chloride, glycerol, propylene glycol and the like, a buffering agent such as sodium phosphate, sodium acetate, sodium borate, sodium carbonate and the like, a surfactant such as polyoxyethylene sorbitan fatty acid ester, polyoxyl stearate 40, polyoxyethylene polyoxypropylene glycol, polyoxyethylene castor oil, polyoxyethylene hydrogenated castor oil and the like, a stabilizer such as disodium edetate, sodium citrate and the like, a preservative such as benzalkonium chloride, sorbic acid, methyl p-hydroxybenzoate and the like, a viscosity agent such as methylcellulose, hydroxymethylcellulose, polyvinylpyrrolidone and the like, an antioxidant such as ascorbic acid, tocophenol and the like. The pH may be any as long as it is within the range acceptable for ophthalmic preparations, and is preferably within the range of 4 to 8. When adjusting the pH, hydrochloric acid, phosphoric acid, citric acid, sodium hydroxide, potassium hydroxide, sodium carbonate and the like can be used. In the following, examples of the pharmaceutical composition of the present invention and a preparation method thereof are illustrated; however, the present invention is not limited thereto.

[Preparation Example 1]

Granules containing the following components are produced.

| Components | |
|---|---|
| compound represented by the formula (I) | 10 mg |
| lactose | 700 mg |
| cornstarch | 274 mg |
| low-viscosity hydroxypropyl cellulose aqueous solution | 16 mg |
| total | 1000 mg |

A compound represented by the formula (I) and lactose are passed through a 60-mesh sieve. Cornstarch is passed through a 120 mesh sieve. These are mixed in a V-type mixer. 2%(w/v) Low-viscosity hydroxypropyl cellulose (hereinafter to be abbreviated as HPC-L) aqueous solution is added to the mixed powder, kneaded and granulated (extrusion-granulation pore size 0.5 - 1 mm), and then dried. The obtained dried granules are sieved with a vibrating sieve (12/60 mesh) to obtain granules.

[Preparation Example 2]

Powder to be filled in a capsule and containing the following components is produced.

| Components | |
|---|---|
| compound represented by the formula (I) | 10 mg |
| lactose | 79 mg |
| cornstarch | 10 mg |
| magnesium stearate | 1 mg |
| total | 100 mg |

A compound represented by the formula (I) and lactose are passed through a 60-mesh sieve. Cornstarch is passed through a 120 mesh sieve. These and magnesium stearate are mixed in a V-type mixer. The 10% powder (100 mg) is filled in a No. 5 hard gelatin capsule.

[Preparation Example 3]

Granules to be filled in a capsule and containing the following components are produced.

| Components | |
|---|---|
| compound represented by the formula (I) | 15 mg |
| lactose | 90 mg |
| cornstarch | 42 mg |
| HPC-L aqueous solution | 3 mg |
| total | 150 mg |

A compound represented by the formula (I) and lactose are passed through a 60-mesh sieve. Cornstarch is passed through a 120 mesh sieve. These are mixed in a V-type mixer. 2%(w/v) HPC-L aqueous solution is added to the mixed powder, kneaded and granulated, and then dried. The obtained dried granules are sieved and screened with a vibrating sieve (12/60 mesh), and 150 mg thereof is filled in a No. 4 hard gelatin capsule.

[Preparation Example 4]

Tablet containing the following components is produced.

| | Components |
|---|---|
| compound represented by the formula (I) | 10 mg |
| lactose | 90 mg |
| crystalline cellulose | 30 mg |
| magnesium stearate | 5 mg |
| carboxymethylcellulose sodium salt | 15 mg |
| total | 150 mg |

A compound represented by the formula (I), lactose, crystalline cellulose, and carboxymethylcellulose sodium salt (CMC-Na) are passed through a 60-mesh sieve and mixed. Magnesium stearate is added to the mixed powder to obtain a mixed powder for preparation. The mixed powder is directly compressed to obtain a tablet.

[Preparation Example 5]

| Components | |
|---|---|
| compound represented by the formula (I) | 100 mg |
| saturated fatty acid glycerides | 1000 mL |

An intravenous preparation is produced by adding the compound represented by the formula (I) to saturated fatty acid glyceride. The solution is generally administered intravenously to patients at a rate of 1 mL per min.

[Formulation Example 6]

An ophthalmic ointment (in 100 g) containing the following components is produced.

| Components | |
|---|---|
| compound represented by the formula (I) | 300 mg |
| liquid paraffin | 10 g |
| white petrolatum | q.s. |

[Formulation Example 7]

An eye drop (in 100 mL) containing the following components is produced.

| Components | |
|---|---|
| compound represented by the formula (I) | 500 mg |
| sodium hydroxide | 900 mg |
| sterile purified water | q.s. |

[Formulation Example 8]

An eye drop (in 100 mL) containing the following components is produced.

| Components | |
|---|---|
| compound represented by the formula (I) | 500 mg |
| polyoxyl 35 castor oil | 90 mg |
| hydrochloric acid | q.s. |
| sodium hydroxide | q.s. |
| sterile purified water | q.s. |

[Preparation Example 9]

A tablet containing the following components is produced.

| Components | |
|---|---|
| compound represented by the formula (I) | 10 mg |
| lactose | 90 mg |
| crystalline cellulose | 30 mg |
| magnesium stearate | 5 mg |
| CMC-Na | 15 mg |
| total | 150 mg |

When the pharmaceutical composition of the present invention is administered to a human, the dose thereof is appropriately determined according to the age and sex of the patient, disease to be the treatment target, and the condition and level of tissue damage. Generally, in the case of an adult, the dose of the compound of the present invention for oral preparation or intrarectal administration is about 0.1 - 1000 mg/human/day, and about 0.05 mg - 500 mg/human/day for injection. For topical ocular administration, the dose is 0.00001 - 100 mg, preferably 0.001 - 10 mg, more preferably 0.01 - 1 mg, most preferably 0.1 - 0.5 mg, per day. The administration may be a single administration or multiple administrations. For example, when the therapeutic agent of the present invention is an eye drop, a single dose of 1 to 5 drops, more preferably 1 to 2 drops, most preferably 1 drop, can be administered 1 to 3 times a day, more preferably 1 to 2 times a day, most preferably 1 time, per day. Here, one drop is generally 0.01 to 0.1 mL. These numerical values are mere examples, and the dose is determined according to the symptoms of the patients.

The compound of the present invention that can be used as a prodrug is a derivative of the present invention which has a group that can be chemically or metabolically decomposed and which produces the pharmacologically active compound of the present invention by solvolysis or in vivo under physiological conditions. The method for selecting and producing a suitable prodrug is described, for example, in Design of Prodrugs (Elsevier, Amsterdam 1985). In the present invention, when the compound has a hydroxyl group, an acyloxy derivative obtained by reacting the compound and a suitable acyl halide or suitable acid anhydride is exemplified as a prodrug. Examples of acyloxy particularly preferable as a prodrug include $-OCOC_2H_5$, -OCO(t-Bu), $-OCOC_{15}H_{31}$, -OCO(m-$CO_2$Na-Ph), $-OCOCH_2CH_2CO_2Na$, -$OCOCH(NH_2)CH_3$, $-OCOCH_2N(CH_3)_2$ and the like. When the compound of the present invention has an amino group, an amide derivative produced by reacting a compound having an amino group with a suitable acid halide or a suitable mixed acid anhydride is exemplified as a prodrug. Examples of amide particularly preferable as a prodrug include $-NHCO(CH_2)_{20}OCH_3$, - $NHCOCH(NH_2)CH_3$ and the like.

The diseases to which the pharmaceutical composition of the present invention can be applied not particularly limited as long as they are diseases or tissue damages that can be treated as a result of promotion of cell proliferation, wound therapy, and tissue regeneration by inhibiting the Hippo signal transduction pathway. In one embodiment, the disease associated with failure of cell proliferation includes a disease associated with abnormal activation of the Hippo signal transduction pathway. In another embodiment, the disease associated with failure of cell proliferation includes a disease associated with abnormal activation of YAP and/or TAZ. Examples of such disease or tissue damage associated with failure of cell proliferation include, but are not limited to, inflammatory disease (e.g., inflammation, dermatitis, atopic dermatitis, hepatitis, nephritis, glomerulonephritis, pancreatitis, psoriasis, gout, Addison's disease, inflammatory bowel disease, Crohn's disease, ulcerative colitis and the like), neurodegenerative disease (e.g., Alzheimer's disease, Parkinson's disease, amyotrophic lateral sclerosis (ALS), Creutzfeldt-Jakob disease, Huntington's disease, spinocerebella degeneration (SCD), multiple system atrophy (MSA), spinal muscular atrophy (SMA), spinal and bulbar muscular atrophy and the like), immune-nerve disease (e.g., multiple sclerosis, Guillain-Barre syndrome, myasthenia gravis, polymyositis and the like), muscular dystrophy, myopathy, trauma, burn, chemical burn, skin damage, skin ulcer, lower leg ulcer, pressure ulcer, diabetic skin ulcer, giant pigmented nevus, scar, disorder due to tattoo, vitiligo vulgaris, leukopathia, spinal cord damage, muscle damage, liver failure, drug-induced hepatopathy, alcohol-induced hepatopathy, ischemic hepatopathy, viral hepatopathy, autoimmune hepatopathy, acute hepatitis, chronic hepatitis, cirrhosis, myocardial infarction, brain edema, cerebral hemorrhage, cerebral infarction, endothelial corneal dystrophy, bullous keratopathy, eye disorder associated with cornea transplantation, contact lens eye disorder, aniridia, Stevens Johnson Syndrome, keratitis, corneal epithelium disorder, superficial punctate keratopathy, cornea erosion, corneal ulcer, dry eye, corneal epithelium detach, pterygium, sclerocornea, cornea dystrophy, keratitis, limbal stem cell deficiency, chemical injury, burn, ocular pemphigoid, diabetes keratopathy, corneal endotheliitis, Fuchs corneal endothelial dystrophy, guttate cornea, iris corneal endothelium syndrome, transplantation failure after cornea transplantation, and the like. In this embodiment, the pharmaceutical composition of the present invention can also be paraphrased as "therapeutic agent of the present invention for diseases or tissue damages associated with cell proliferation failure".

In one embodiment, the pharmaceutical composition of the present invention may be effective in promoting engraftment and recovery of cell, tissue, organ and the like transplanted to a mammal. Examples of the cell to be transplanted include, but are not limited to, stem cells (hematopoietic stem cells, mesenchymal stem cells, neural stem cells, etc.), β-cells and the like. Examples of the tissue to be transplanted include, but are not limited to, blood, erythrocytes, platelets, lymphocytes, bone marrow, cord blood, blood vessel, cornea, retina, eyeball, skin and the like. Examples of the organ to be transplanted include, but are not limited to, pancreas, lung, kidney, liver, heart, small intestine, eye and the like. These cells, tissues, and organs can be subjected to gene transfer using virus or genetic modification by genome editing before transplantation. In this embodiment, the pharmaceutical composition of the present invention can also be paraphrased as "an engraftment promoter of cell, tissue, and/or organ transplantation".

[Example]

The present invention is described in more detail in the following by specifically describing Synthetic Examples and Experimental Example of the hydrazide compound represented by the formula (I) used in the composition and the like of the present invention as examples.

[Synthetic Examples of compounds]

[Synthetic Example 1] Synthesis method of (E)-N'-(1-(2,4-dihydroxy-3-methylphenyl)propylidene)-2-(pyridin-2-ylamino)acetohydrazide (compound No. A-001)

2-(Pyridin-2-ylamino)acetohydrazide (120 mg) and 2',4'-dihydroxy-3'-methylpropiophenone (100 mg) were suspended in 1.1 mL of dimethyl sulfoxide, and the mixture was stirred at 100°C for 21 hr. After completion of the reaction,

the reaction solution was allowed to cool to room temperature, and directly purified by medium pressure silica gel column chromatography [silica gel (10 g), methanol/methylene chloride = 1/99 - 8/92 (volume ratio, hereinafter the same) gradient]. Water (5 mL) was added to the obtained purified product and the precipitated solid was collected by filtration. The solid was further suspension-washed with methylene chloride to give 47.9 mg of the desired product as a white solid.

[Synthetic Example 2] Synthesis method for synthesis of optically active compound of (E)-N'-(1-(2,4-dihydroxy-3-methylphenyl)propylidene)-2-(pyridin-3-ylamino)propanehydrazide (compound No. A-004) (compound Nos. A-004A and A-004B)

By a method according to Synthetic Example 1, 81.6 mg of A-004 was synthesized from 2-(pyridine-3-amino)propanehydrazide (135 mg) and 2',4'-dihydroxy-3'-methylpropiophenone (135 mg). Using supercritical fluid chromatography (SFC) manufactured by Waters, A-004 was optically resolved to give 6.6 mg of A-004A and 4.2 mg of A-004B.

[optical resolution conditions]

column; CHIRALPAK IA-3 (20×250 mm, 5 μm; manufactured by Daicel Corporation)
mobile phase; $CO_2$:MeOH=60:40 (volume ratio)
column temperature; 40°C
flow rate; 15 mL/min

Under the above-mentioned conditions, A-004 showed two peaks having a retention time of 8.14 min and a retention time of 13.40 min. After optical resolution, the compound with a retention time of 8.14 min was used as A-004A (optical purity 99.9%ee), and the compound with a retention time of 13.40 min was used as A-004B (optical purity 99.9%ee).

[Synthetic Example 3] Synthesis method of (E)-2-(1-(2,4-dihydroxy-3-methylphenyl)propylidene)-N-(pyridin-3-ylmethyl)hydrazine-1-carboxamide (compound No. B-001)

Step 1;

3-Aminomethylpyridine (1.0 g) was suspended in a mixed solvent of methylene chloride (11 mL) and water (11 mL). To the suspension was added sodium hydrogen carbonate (2.3 g) and the mixture was cooled to 0°C. To the solution was slowly added dropwise phenyl chloroformate (1.3 mL), and the mixture was gradually allowed to warm to room temperature. The mixture was stirred for 22 hr, water (10 mL) was added to discontinue the reaction, and the mixture was partitioned. The organic layer was washed with brine (10 mL), dried over anhydrous magnesium sulfate, and filtered. The filtrate was concentrated under reduced pressure and the obtained residue was purified by medium pressure silica gel column chromatography (silica gel 30 g, ethyl acetate/hexane=5/95 - 50/50 gradient) to give 0.54 g of phenyl(pyridin-3-ylmethyl)carbamate as a white solid.

Step 2;

The desired product (0.54 g) of Step 1 was suspended in ethanol (4.7 mL), and hydrazine monohydrate (0.57 mL) was added. The reaction mixture was stirred at 60°C for 17 hr. After completion of the reaction, the reaction mixture was concentrated under reduced pressure, and dehydrated by azeotropic distillation with toluene. The obtained residue was washed with toluene and filtered to give N-(pyridin-3-ylmethyl)hydrazine carboxamide (0.28 g) as a white solid.

Step 3;

By a method according to Synthetic Example 1, 95.0 mg of the desired product was obtained as a white solid from the desired product (120 mg) of Step 2 and 2',4'-dihydroxy-3'-methylpropiophenone (100 mg).

[Synthetic Example 4] Synthesis method of (E)-N'-(1-(2,4-dihydroxy-3-methylphenyl)propylidene)-2-(pyridin-3-yloxy)propanehydrazide (compound No. C-001)

Step 1;

3-Hydroxypyridine (1.26 g), methyl lactate (1.0 mL) and triphenyl phosphine (3.5 g) were added to toluene (30 mL), and an about 1.9 mol/L toluene solution of diisopropyl azodicarboxylate_(manufactured by Tokyo Chemical Industry Co., Ltd., hereinafter the same) (7.0 mL) was added under icecooling. The reaction mixture was allowed to warm to room temperature and stirred for 19 hr. To the reaction mixture was added an about 1.9 mol/L toluene solution (2.32 mL) of triphenyl phosphine (1.17 g) and diisopropyl azodicarboxylate, and the mixture was further stirred at room temperature for 28 hr. The reaction solution was concentrated under reduced pressure and the obtained residue was purified by medium pressure silica gel column chromatography (silica gel 100 g, ethyl acetate/hexane=10/90 - 40/60) to give 735 mg of methyl 2-(pyridin-3-yloxy)propionate as a yellow liquid.

Step 2;

The desired product (370 mg) of Step 1 was dissolved in acetonitrile (5.1 mL), hydrazine monohydrate (0.50 mL) was added, and the mixture was stirred at 60°C for 6 hr. The reaction solution was concentrated under reduced pressure, the obtained residue was washed with toluene and filtered to give 285 mg of 2-(pyridin-3-yloxy)propanehydrazide as a white solid.

Step 3;

By a method according to Synthetic Example 1, 93.6 mg of the desired product was obtained as a white solid from the desired product (130 mg) of Step 2 and 2',4'-dihydroxy-3'-methylpropiophenone (100 mg).

[Synthetic Example 5] Synthesis method of (R,E)-2-[(1H-indazol-6-yl)amino]-N'-[1-(2,4-dihydroxy-3-methylphenyl)propanehydrazide (compound No. A-016)

Step 1;

Under the nitrogen stream, to a 1,2-dichloroethane solution (10mL) of 1H-indazol-6-amine (500 mg) and 2,6-dimethylpyridine (553 mg) was added ethyl (S)-2-{[(trifluoromethyl)sulfonyl]oxy}propanoate (1.03 g) at 20°C, and the mixture was stirred at 70°C for 16 hr. To the reaction mixture was added water, and the mixture was extracted 3 times with ethyl acetate. The combined organic layer was dried over anhydrous sodium sulfate, filtered, and concentrated. The residue was purified by silica gel column chromatography (petroleum ether/ethyl acetate =4/1), and ethyl (1H-indazol-6-yl)-D-alaninate (247 mg) was obtained as a yellow oil.

Step 2;

By a method according to Synthetic Example 3, Steps 2 and 3 and using the desired product (247 mg) of Step 1, the desired product (17 mg) was obtained as a white solid.

[Synthetic Example 6] Synthesis method of (R,E)-N'-[1-(2,4-dihydroxy-3-methylphenyl)propylidene]-2-(pyrimidin-5-ylamino)propanehydrazide (compound No. A-017)

Step 1;

To a tetrahydrofuran solution (5 mL) of hexamethylphosphoric triamide (5.1 g) was added sodium hydride (338 mg) at 0 - 5°C. Successively, t-butylpyrimidin-5-ylcarbamate (550 mg) was added and the mixture was stirred at 20°C for 0.5 hr. A tetrahydrofuran solution (2 mL) of ethyl (S)-2{[(trifluoromethyl)sulfonyl]oxy}propanate (2.11 g) was added and the reaction mixture was stirred at 50°C for 16 hr. Water was added to the reaction mixture, and the mixture was extracted 5 times with ethyl acetate. The combined organic layer was dried over anhydrous sodium sulfate, filtered, and concentrated to dryness. The residue was purified by silica gel column chromatography (petroleum ether/ethyl acetate 0→10%) to give ethyl N-(t-butoxycarbonyl)-N-(pyrimidin-5-yl)-D-alaninate (325 mg) as a yellow oil.

Step 2;

To an ethyl acetate solution (3 mL) of N-(t-butoxycarbonyl)-N-(pyrimidin-5-yl)-D-alaninate (325 mg) was added 4M hydrogen chloride-ethyl acetate (5.5 mL) at 0 - 5°C, and the mixture was stirred at 20°C for 19 hr. To the reaction mixture was added saturated aqueous sodium hydrogen carbonate, and the mixture was extracted 3 times with ethyl acetate. The combined organic layer was dried over anhydrous sodium sulfate, filtered, and concentrated to dryness to give ethyl pyrimidin-5-yl-D-alaninate (160 mg) as a yellow oil.

Step 3;

By a method according to Synthetic Example 3, Step 2 and using the desired product of Step 2 and hydrazine monohydrate, (R)-2-(pyrimidin-5-ylamino)propanehydrazide (147 mg) was obtained as a yellow oil.

Step 4;

To the desired product (100 mg) of Step 3 and an ethanol solution of 2',4'-dihydroxy-3'-methylpropiophenone (101 mg) was added acetic acid (63 μL), and the mixture was stirred at 65°C for 5 days. The reaction mixture was concentrated and purified by silica gel column chromatography (dichloromethane/menol=10/1) and reversed-phase chromatography to give the desired product (11 mg) as a yellow solid.

[Synthetic Example 7] Synthesis method of (E)-2-[(1H-benzo[d]imidazol-6-yl)amino]-N'-[1-(2,4-dihydroxy-3-methylphenyl)propanehydrazide (compound No. A-018)

Step 1;

To a 1,2-dichloroethane solution (35 mL) of 1H-benzo[d]imidazole-6-amine (2 g) were added ethyl 2-oxopropanoate (2.09 g) and acetic acid (859 μL), and the mixture was stirred at 50°C for 30 min. Successively, to the reaction mixture was added sodium triacetoxyhydride (4.14 g) and the mixture was stirred at 50°C for 15 hr. To the reaction mixture was added saturated aqueous sodium hydrogen carbonate, and the mixture was extracted 4 times with dichloromethane. The combined organic layer was washed with brine, dried over anhydrous sodium sulfate, filtered, and concentrated to give ethyl (1H-benzo[d]imidazol-6-yl)alaninate (1.2 g) as a yellow solid.

Step 2;

By a method according to Synthetic Example 3, Step 2 and Synthetic Example 6, Step 4 and using the desired product (300 mg) of Step 1, the desired product (32 mg) was obtained as a yellow solid.

[Synthetic Example 8] Synthesis method of (R,E)-N'-[1-(2,4-dihydroxy-3-methylphenyl)propylidene]-2-(quinolin-7-ylamino)propanehydrazide (compound No. A-019)

Step 1;

A 1,4-dioxane solution (10 mL) of ethyl D-alaninate (0.5 g), 7-bromoquinoline (554 mg), tris(dibenzylideneacetone)dipalladium (0) (220 mg), xantphos (139 mg) and cesium carbonate (2.35 g) was deaerated 3 times with nitrogen under reduced pressure, and stirred at 90°C for 16 hr. To the reaction mixture was added ethyl acetate and the mixture was filtered through Celite. The filtrate was washed 3 times with water, dried over anhydrous sodium sulfate, filtered, and concentrated. The residue was purified by silica gel column chromatography (petroleum ether/ethyl acetate:0→50%) to give ethylquinolin-7-yl-D-alaninate (235 mg) as a yellow oil.

Step 2;

By a method according to Synthetic Example 3, Step 2 and Synthetic Example 6, Step 4 and using the desired product (100 mg) of Step 1, the desired product (9.1 mg) was obtained as a yellow solid.

The compound represented by the formula (I) can be synthesized by a method according to the aforementioned Synthetic Example 1 to Synthetic Example 8. Examples of the compound represented by the formula (I) and synthesized in the aforementioned Synthetic Examples are shown in the First Table to the Sixth Table. However, the compound of the present invention is not limited thereto.

In the Tables, Me shows methyl, Et shows ethyl, (R) in $R^2$ means R configuration, and (rac) means racemate.

In $R^a$, "-" shows unsubstituted.

In the Tables, moreover, D-1, D-2, D-3, D-4, D-5, D-6, D-7, D-8, D-9, D-10, D-11 and D-12 show the following structures, and the number indicated in the structural formulas show the substitutable positions of $R^a$. In the formulas, "m" is 0, 1, 2, 3 or 4, and "p" is 0, 1, 2 or 3.

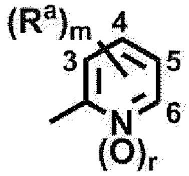
D-1

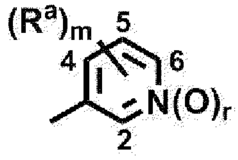
D-2

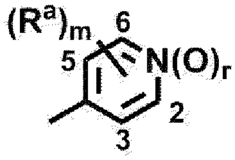
D-3

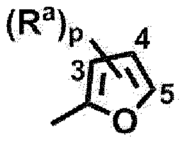
D-4

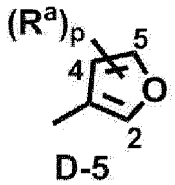
D-5

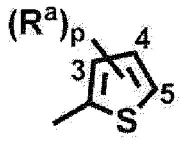
D-6

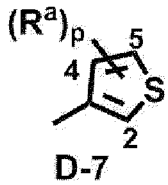
D-7

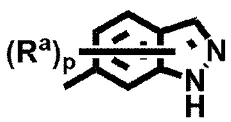
D-8

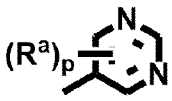
D-9

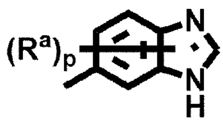
D-10

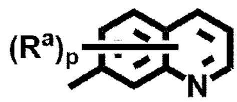
D-11

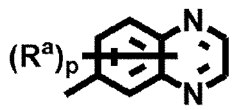
D-12

[First Table]

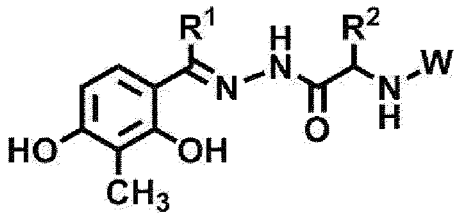

[Table 1]

| compound No. | $R^1$ | $R^2$ | W | r | $R^a$ | m |
|---|---|---|---|---|---|---|
| A-001 | Et | H | D-1 | 0 | - | |
| A-002 | Et | H | D-2 | 0 | - | |
| A-003 | Et | H | D-3 | 0 | - | |
| A-004 | Et | Me | D-2 | 0 | - | |
| A-004A | Et | Me | D-2 | 0 | - | |
| A-004B | Et | Me | D-2 | 0 | - | |
| A-005 | Et | Et | D-2 | 0 | - | |
| A-006 | Et | Me | D-2 | 0 | 6-Me | 1 |
| A-007 | Et | Me | D-2 | 0 | 5-F | 1 |

[Second Table]

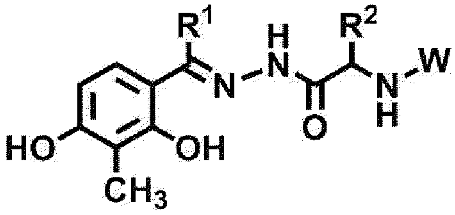

[Table 2]

| compound No. | $R^1$ | $R^2$ | W | r | $R^a$ | m |
|---|---|---|---|---|---|---|
| A-007R | Et | Me (R) | D-2 | 0 | 5-F | 1 |
| A-008 | Et | Me (R) | D-2 | 0 | 5-Me | 1 |
| A-009 | Et | Me (R) | D-2 | 0 | 5-OH | 1 |
| A-010 | Et | Me (R) | D-2 | 0 | 6-F | 1 |
| A-011 | Et | Me (R) | D-2 | 0 | 5-Cl | 1 |
| A-012 | Et | Me (R) | D-2 | 0 | 6-Cl | 1 |
| A-013 | Et | Me (R) | D-2 | 0 | 5-$CF_3$ | 1 |
| A-014 | Et | Me (R) | D-2 | 0 | 6-$CF_3$ | 1 |

[Third Table]

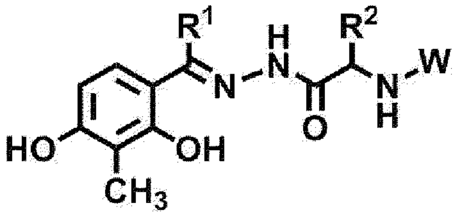

[Table 3]

| compound No. | $R^1$ | $R^2$ | W | $R^a$ | p |
|---|---|---|---|---|---|
| A-015 | Et | Me(R) | D-9 | 6-$CF_3$ | 1 |
| A-016 | Et | Me(R) | D-8 | - | 0 |
| A-017 | Et | Me(R) | D-9 | - | 0 |
| A-018 | Et | Me(rac) | D-10 | - | 0 |
| A-019 | Et | Me(R) | D-11 | - | 0 |
| A-020 | Et | Me(R) | D-12 | - | 0 |

[Fourth Table]

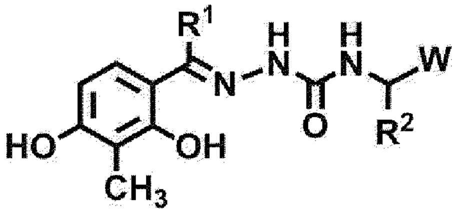

[Table 4]

| compound No. | $R^1$ | $R^2$ | W | r | $R^a$ |
|---|---|---|---|---|---|
| B-001 | Et | H | D-2 | 0 | - |
| B-002 | Et | H | D-4 | - | - |
| B-003 | Et | H | D-5 | - | - |
| B-004 | Et | H | D-6 | - | - |
| B-005 | Et | H | D-7 | - | - |

[Fifth Table]

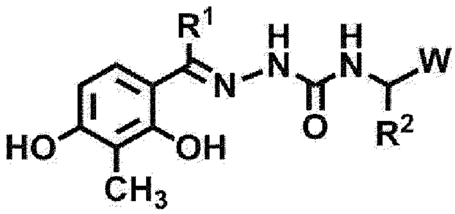

[Table 5]

| compound No. | $R^1$ | $R^2$ | W | r | $R^a$ | m |
|---|---|---|---|---|---|---|
| B-006 | Et | H | D-2 | 0 | 5-F | 1 |
| B-007 | Et | Me (rac) | D-2 | 0 | 5-F | 1 |

[Sixth Table]

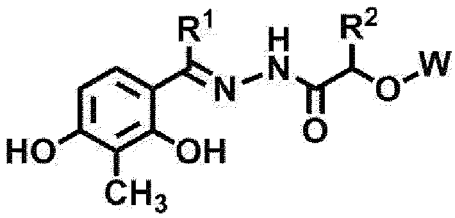

[Table 6]

| compound No. | $R^1$ | $R^2$ | W | r | $R^a$ |
|---|---|---|---|---|---|
| C-001 | Et | Me | D-2 | 0 | - |

The $^1$H-NMR data of the compounds described in the First Table to Sixth Table, among the compounds shown

by the formula (I), are shown below.

The chemical shift value of proton nuclear magnetic resonance was measured at 270 MHz or 400 MHz in dimethyl sulfoxide-d6, with the value of dimethyl sulfoxide-d6 being 2.49 ppm. The symbols in the $^1$H-NMR data have the following meanings. s: singlet, brs: broad singlet, d: doublet, dd: double doublet, t: triplet, q: quartet, m: multiplet.

A-001 (270MHz);

δ13.68(s, 1H), 10.95(s, 1H), 9.68(s, 1H), 7.98(d, J=2.7Hz, 1H), 7.41(t, J=8.1Hz, 1H), 7.25(d, J=8.1Hz, 1H), 6.93(t, J=8.1Hz, 1H), 6.63(d, J=8.1Hz, 1H), 6.53(t,J=8.1Hz, 1H), 6.39(d, J=8.1Hz, 1H), 4.11(d, J=8.1Hz, 2H), 2.78(q, J=8.1Hz, 2H), 1.96(s, 3H), 1.05(t, J=8.1Hz, 3H).

A-002 (270MHz);

δ13.66(s, 1H), 10.92(brs, 1H), 9.70(brs, 1H), 8.02(brs, 1H), 7.80(brs, 1H), 7.26(d, J=8.1Hz, 1H), 7.09(t, J=8.1Hz, 1H), 6.95(d, J=8.1Hz, 1H), 6.40(d, J=8.1Hz, 1H), 6.26(t, J=8.1Hz, 1H), 4.00(d, J=5.4Hz, 2H), 2.81(q, J=8.1Hz, 2H), 1.96(s, 3H), 1.08(t, J=8.1Hz, 3H).

A-003 (270MHz);

δ13.63(s, 1H), 11.03(brs, 1H), 9.72(brs, 1H), 8.08(d, J=5.4Hz, 2H), 7.36(t, J=8.1Hz, 1H), 7.27(d, J=8.1Hz, 1H), 6.64(d, J=5.4Hz, 2H), 6.41(d, J=8.1Hz, 1H), 4.10(d, J=5.4Hz, 2H), 2.83(q, J=8.1Hz, 2H), 1.96(s, 3H), 1.10(t, J=8.1Hz, 3H).

A-004 (270MHz);

δ13.63(s, 1H), 10.91(s, 1H), 9.71(s, 1H), 8.02(d, J=2.7Hz, 1H), 7.79(d, J=5.4Hz, 1H), 7.26(d, J=8.1Hz, 1H), 7.08(t, J=8.1Hz, 1H), 6.94(d, J=8.1Hz, 1H), 6.40(d, J=8.1Hz, 1H), 6.23(d, J=8.1Hz, 1H), 4.33(m, J=8.1Hz, 1H), 2.82(q, J=8.1Hz, 2H), 1.95(s, 3H), 1.42(d, J=8.1Hz, 3H), 1.05(t, J=8.1Hz, 3H).

A-005 (270MHz);

δ13.67(s, 1H), 10.95(s, 1H), 9.75(brs, 1H), 8.05(brs, 1H), 7.77(brs, 1H), 7.27(d, J=8.1Hz, 1H), 7.08(d, J=8.1Hz, 1H), 7.00(d, J=8.1Hz, 1H), 6.40(d, J=8.1Hz, 1H), 6.21(d, J=8.1Hz, 1H), 5.77(s, 1H), 4.21(m, 1H), 2.82(q, J=8.1Hz, 2H), 1.95(s,3H), 1.78(m, 2H), 1.06(t, J=8.1Hz, 3H), 0.99(t, J=8.1Hz, 3H).

A-006 (270MHz);

δ13.66(s, 1H), 10.92(brs, 1H), 9.74(brs, 1H), 7.90(brs, 1H), 7.27(d, J=8.1Hz, 1H), 6.96(d, J=8.1Hz, 1H), 6.93(d, J=8.1Hz, 1H), 6.40(d, J=8.1Hz, 1H), 6.01(d, J=8.1Hz, 1H), 4.30(m, 1H), 2.82(q, J=8.1Hz, 2H), 2.27(s, 3H), 1.95(s, 3H), 1.40(d,J=8.1Hz, 3H), 1.05(t, J=8.1Hz, 3H).

A-007 (270MHz);

δ13.61(s, 1H), 10.97(s, 1H), 9.73(s, 1H), 7.89(brs, 1H), 7.71(brs, 1H), 7.26(d,J=8.1Hz, 1H), 6.79(d, J=13.5Hz, 1H), 6.69(d, J=10.8Hz, 1H), 6.38(d, J=8.1Hz, 1H), 4.34(m, 1H), 2.82(q, J=8.1Hz, 2H), 1.93(s, 3H), 1.40(d, J=8.1Hz, 3H), 1.05(t, J=8.1Hz, 3H).

A-007R (270MHz);

δ13.63(s, 1H), 10.97(s, 1H), 9.73(s, 1H), 7.91(brs, 1H), 7.73(brs, 1H), 7.28(d,J=8.1Hz, 1H), 6.81(d, J=13.5Hz, 1H), 6.69(d, J=8.1Hz, 1H), 6.40(d, J=8.1Hz, 1H), 4.35(m, 1H), 2.84(q, J=8.1Hz, 2H), 1.95(s, 3H), 1.42(d, J=5.4Hz, 3H), 1.07(t, J=8.1Hz, 3H).

A-008 (270MHz);

δ13.64(s, 1H), 10.91(s, 1H), 9.72(s, 1H), 7.84(brs, 1H), 7.64(brs, 1H), 7.27(d,J=8.1Hz, 1H), 6.79(brs, 1H), 6.40(d, J=8.1Hz, 1H), 6.15(d, J=8.1Hz, 1H), 4.33(m, 1H), 2.82(q, J=Hz, 2H), 2.16(s, 3H),1.95(s, 3H), 1.41(d, J=8.1Hz, 3H), 1.06(t, J=8.1Hz, 3H).

A-009 (270MHz);

δ13.65(s, 1H), 10.89(s, 1H), 9.72(s, 1H), 9.45(s, 1H),7.52(brs, 1H), 7.39(brs, 1H), 7.27(d,J=8.1Hz, 1H), 6.40(d, J=8.1Hz, 1H), 6.36(d, J=2.7Hz, 1H), 6.14(d, J=8.1Hz, 1H), 4.25(m, 1H), 2.82(q, J=8.1Hz, 2H), 1.95(s, 3H), 1.39(d, J=5.4Hz, 3H), 1.06(t, J=8.1Hz, 3H).

A-010 (270MHz);

δ13.64(s, 1H), 10.93(s, 1H), 9.72(s, 1H), 7.53(brs, 1H), 7.27(d, J=8.1Hz, 1H), 7.19(m, 1H), 6.92(m, 1H), 6.40(d, J=8.1Hz, 1H), 6.23(d, J=8.1Hz, 1H), 4.31(m, 1H), 2.81(q, J=8.1Hz, 2H), 1.95(s, 3H), 1.41(d, J=8.1Hz, 3H), 1.06(m, 3H).

A-011 (270MHz);

δ13.62(s, 1H), 10.98(s, 1H), 9.73(s, 1H), 7.99(d, J=2.7Hz, 1H), 7.78(d, J=2.7Hz, 1H), 7.28(d,J=8.1Hz, 1H), 7.03(s, 1H),6.67(d, J=8.1Hz, 1H), 6.40(d, J=10.8Hz, 1H), 4.37(m, 1H), 2.84(q, J=8.1Hz, 2H), 1.96(s, 3H), 1.42(d, J=8.1Hz, 3H), 1.08(t, J=8.1Hz, 3H).

A-012 (270MHz);

δ13.63(s, 1H), 10.95(s, 1H), 9.72(s, 1H), 7.79(d, J=2.7Hz, 1H), 7.27(d, J=8.1Hz, 1H), 7.20(d, J=8.1Hz, 1H), 7.06(m, 1H), 6.48(d, J=8.1Hz, 1H), 6.40(d, J=8.1Hz, 1H), 4.33(m, 1H), 2.82(q, J=5.4Hz, 2H), 1.95(s, 3H), 1.41(d, J=5.4Hz, 3H), 1.07(t, J=8.1Hz, 3H).

A-013 (270MHz);

δ13.60(s, 1H), 11.02(s, 1H), 9.72(s, 1H), 8.29(brs, 1H), 8.10(brs, 1H), 7.25(m, 2H), 6.84(d, J=8.1Hz, 1H), 6.39(d, J=8.1Hz, 1H), 4.44(m, 1H), 3.16(d, J=5.4Hz, 1H), 2.82(q, J=8.1Hz, 2H), 1.94(s, 3H), 1.43(d, J=8.1Hz, 3H), 1.06(t, J=8.1Hz, 3H).

A-014 (270MHz);

δ13.62(s, 1H), 11.02(s, 1H), 9.72(s, 1H), 8.13(d, J=2.7Hz, 1H), 7.58(d, J=8.1Hz, 1H), 7.28(d, J=8.1Hz, 1H), 7.05(s, 1H), 7.01(s, 1H), 6.40(d, J=10.8Hz, 1H), 4.43(m, 1H), 2.84(q, J=8.1Hz, 2H), 1.96(s, 3H), 1.45(d, J=5.4Hz, 3H), 1.08(t, J=8.1Hz, 3H).

A-015 (270MHz);

δ13.61(s, 1H), 11.07(s, 1H), 9.73(s, 1H), 8.29(s, 1H), 7.30(t, J=8.1Hz, 2H), 6.41(d, J=8.1Hz, 1H), 4.50(m, 1H), 2.84(m, 2H), 1.97(s, 3H), 1.47(d, J=8.1Hz, 3H), 1.10(t, J=8.1Hz, 3H).

A-016 (400MHz);

δ13.6(s, 1H), 12.4(s, 1H), 10.9(s, 1H), 9.71(s, 1H), 7.74(s, 1H), 7.42(d, J=8.8Hz, 1H), 7.25(d, J=8.8Hz, 1H), 6.64(d, J=10Hz, 1H), 6.40-6.30(m, 2H), 6.20(d, J=8.0Hz, 1H), 4.33-4.30(m, 1H), 2.83-2.78(m, 2H), 1.94(s, 3H), 1.44(d, J=6.8Hz, 3H), 1.02(t, J=7.6Hz, 3H).

A-017 (400MHz);

δ13.6(s, 1H), 11.1-10.9(m, 1H), 9.75-9.65(m, 1H), 8.42(s, 1H), 8.17(s, 2H), 8.10-8.00(m, 1H), 7,27(d, J=8.8Hz, 1H), 6.53(d, J=8.8Hz, 1H), 6.40(d, J=8.8Hz, 1H), 4.40-4.30(m, 1H), 2.75-2.65(m, 1H), 2.00-1.90(m, 3H), 1.43(d, J=6.8Hz, 3H), 1.07(t, J=7.4Hz, 3H).

A-018 (400MHz);

δ13.6(s, 1H), 12.1-11.8(m 1H), 10.8(s, 1H), 9.70(s, 1H), 8.00-7.80(m,1H), 7.40-7.20(m, 2H), 6.80-6.60(m, 2H), 6.38(d, J=8.8Hz, 1H), 5.90-5.60(m, 1H), 4.35-4.25(m, 1H), 2.78(d, J=7.2Hz, 2H), 1.96(s, 3H), 1.43(d, J=6.4Hz, 3H), 1.05-0.96(m, 3H).

A-019 (400MHz);

δ13.6(s, 1H), 11.1(s, 1H), 9.70(s, 1H), 8.60(d, J=3.2Hz, 1H), 8.03(d, J=7.6Hz, 1H), 7.65(d, J=8.8Hz, 1H), 7.26(d, J=8.8Hz, 1H), 7.16-7.09(m, 2H), 6.86(s, 1H), 6.66(d, J=8.0Hz, 1H), 6.39(d, J=8.8Hz, 1H), 4.48(t, J=7.0Hz, 1H), 2.85(q, J=7.3Hz, 1H), 1.94(s, 3H), 1.47(d, J=7.0Hz, 3H), 1.08(t, J=7.3Hz, 3H).

A-020 (400MHz);

δ13.6(s, 1H), 11.1(s, 1H), 9.71(s, 1H), 8.62(d, J=1.6Hz, 1H), 8.48(d,J=1.6Hz,1H), 7.77(d, J=9.2Hz, 1H), 7.40(dd, J=9.2 and 2.4Hz, 1H), 7.27(d, J=8.8Hz, 1H), 7.01(d, J=8.0Hz, 1H), 6.83(d, J=2.0Hz, 1H), 6.39(d, J=8.0Hz, 1H), 4.55-4.45(m, 1H), 2.86(q, J=7.4Hz, 1H), 1.94(s, 3H), 1.49(d, J=6.8Hz, 3H), 1.08(t, J=7.4Hz, 3H).

B-001 (270MHz);

δ13.37(s, 1H), 9.62(s, 1H), 9.54(s, 1H), 8.54(s, 1H), 8.47(d, J=2.7Hz, 1H), 7.73(d, J=8.1Hz, 1H), 7.37(d, J=8.1Hz, 1H), 7.17(d, J=8.1Hz, 1H), 6.94(t, J=8.1Hz, 1H), 6.37(d, J=8.1Hz, 1H), 4.37(d, J=8.1Hz, 2H), 2.66(q, J=8.1Hz, 2H), 1.97(s, 3H), 1.07(t, J=8.1Hz, 3H).

B-002 (270MHz);

δ13.41(s, 1H), 9.55(s, 1H), 9.53(s, 1H), 7.61(s, 1H), 7.17(d, J=8.1Hz,1H), 6.76(t, J=8.1Hz, 1H), 6.41(t, J=8.1Hz, 1H), 6.37(d, J=8.1Hz, 1H), 6.29(d, J=2.7Hz, 1H), 4.33(d, J=5.4Hz, 2H), 2.64(q, J=8.1Hz, 2H), 1.97(s, 3H), 1.07(t, J=8.1Hz, 3H).

B-003 (270MHz);

δ13.44(s, 1H), 9.53(s, 1H), 9.52(s, 1H), 7.62(s, 1H), 7.60(d, J=8.1Hz, 1H), 7.16(d, J=8.1Hz, 1H), 6.63(t, J=8.1Hz, 1H), 6.48(s, 1H), 6.37(d, J=8.1Hz, 1H), 4.17(d, J=2.7Hz, 2H), 2.64(q, J=8.1Hz, 2H), 1.97(s, 3H), 1.07(t, J=8.1Hz, 3H).

B-004 (270MHz);

δ13.39(s, 1H), 9.58(s, 1H), 9.54(s, 1H), 7.41(d, J=2.7Hz, 1H), 7.17(d, J=8.1Hz,1H), 7.00(d, J=5.4Hz, 1H), 6.97(t, J=5.4Hz, 1H), 6.88(t, J=8.1Hz, 1H), 6.37(d, J=8.1Hz, 1H), 4.51(d, J=8.1Hz, 2H), 2.65(q, J=8.1Hz, 2H), 1.97(s, 3H), 1.07(t, J=8.1Hz, 3H).

B-005 (270MHz);

δ13.44(s, 1H), 9.54(s, 1H), 9.53(s, 1H), 7.51(d, J=5.4Hz, 1H), 7.34(s, 1H), 7.16(d, J=8.1Hz, 1H), 7.09(d, J=5.4Hz, 1H), 6.76(t, J=8.1Hz, 1H), 6.37(d, J=8.1Hz, 1H), 4.32(d, J=5.4Hz, 2H), 2.64(q, J=8.1Hz, 2H), 1.97(s, 3H), 1.07(t, J=8.1Hz, 3H).

B-006 (270MHz);

δ13.33(s, 1H), 9.67(s, 1H), 9.56(s, 1H), 8.45(s, 1H), 8.42(s, 1H), 7.63(d, J=8.1Hz, 1H), 7.16(d, J=8.1Hz, 1H), 7.00(m, 1H), 6.36(d, J=8.1Hz, 1H), 4.40(d, J=5.4Hz, 2H), 2.66(q, J=8.1Hz, 2H), 1.96(s, 3H), 1.06(t, J=8.1Hz, 3H).

B-007 (270MHz);

δ13.40(s, 1H), 9.56(s, 1H), 9.54(s, 1H), 8.48(s, 1H), 8.46(s, 1H), 7.71(m, 1H), 7.17(d, J=8.1Hz, 1H), 7.00(d, J=8.1Hz, 1H), 6.36(d, J=10.8Hz, 1H), 4.93(m, 1H), 2.63(q, J=8.1Hz, 2H), 1.95(s, 3H), 1.46(d, J=8.1Hz, 3H), 1.09(t, J=8.1Hz, 3H).

C-001 (270MHz);

δ13.58(s, 1H), 11.11(s, 1H), 9.77(s, 1H), 8.30(s, 1H), 8.19(t, J=2.7Hz, 1H), 7.35(d, J=2.7Hz, 1H), 7.33(d, J=2.7Hz, 1H), 7.29(d, J=8.1Hz, 1H), 6.40(d, J=8.1Hz,1H), 5.20(q, J=8.1Hz, 1H), 2.82(q, J=8.1Hz, 2H), 1.95(s, 3H), 1.58(d, J=8.1Hz, 3H), 1.06(t, J=8.1Hz, 3H).

[Experimental Example 1] Evaluation of action of the compound of the present invention on SKOV3 cell proliferation - 1

Human ovarian cancer cell line SKOV3 (manufactured by DS Pharma Biomedical Co., Ltd) was precultured (single layer culture) in a 15% fetal bovine serum (hereinafter to be abbreviated as FBS, manufactured by Corning)-containing McCoy's 5a medium (manufactured by Sigma-Aldrich). The above-mentioned cells in the logarithmic growth phase were washed with PBS, a 0.25%(w/v) trypsin-1 mmol/L ethylenediaminetetraacetic acid (EDTA) solution (manufactured by FUJIFILM Wako Pure Chemical Corporation) was added, and adherent cells were detached by incubating at 37°C for 3 min. The above-mentioned medium was added and the mixture was centrifuged and resuspended in the same medium.

According to the method of patent document 1 (WO 2014/017513), a composition of McCoy's 5a medium (manufactured by Sigma-Aldrich) containing 0.015%(w/v) deacylated gellan gum (KELCOGEL CG-LA, manufactured by Sansho Co., Ltd.) and 15%(v/v) FBS, 30 ng/mL human EGF (manufactured by PEPROTECH) was prepared using FCeM-series Preparation Kit (manufactured by FUJIFILM Wako Pure Chemical Corporation). Then, the SKOV3 cells prepared above were suspended in the above-mentioned medium composition added with deacylated gellan gum, and dispensed into the wells of a 96 well flat bottom Ultra-Low Attachment surface microplate (manufactured by Corning Incorporated, #3474) at 2000 cells/90 μL/well. The compound of the present invention dissolved in dimethyl sulfoxide was diluted with the above-mentioned medium, and the diluted solution was added by 10 μL such that the final concentration of the compound of the present invention was 5 μM, 20 μM, 40 μM. Dimethyl sulfoxide (DMSO) alone was added to some wells as a control. Each plate was cultured in a standing state in a $CO_2$ incubator (37°C, 5% $CO_2$) for 4 days. An ATP reagent (100 μL) [CellTiter-Glo (registered trade mark) Luminescent Cell Viability Assay, manufactured by Promega) was added to the culture medium on day 4, and the mixture was stirred by a plateshaker (manufactured by AS ONE Corporation, Micro plate mixer NS-P) at room temperature for 2 min and then stood for 10 min. 100uL was transferred to a 96 well flat bottom white plate (manufactured by Corning, #3912), the luminescence intensity (RLU value) was measured by EnSpire (manufactured by Perkin Elmer), and the luminescence value of the medium alone was subtracted to measure the number of viable cells. The numbers of the compounds of the present invention that showed a relative value of not less than 125% at each final concentration when the RLU value (ATP measurement, luminescence intensity) of the control was 100% are shown below.

Number of the compound of the present invention (5 μM); A-002, A-004, A-004A, A-005, A-007, A-007R, A-010, A-011, A-012.

Number of the compound of the present invention (20 μM); A-001, A-002, A-004, A-004A, A-005, A-007, A-007R, A-010, A-016, B-003, B-004, B-005 and B-007.

Number of the compound of the present invention (40 μM); A-001, A-002, A-004, A-004A, A-005, A-016, A-017, B-001, B-003, B-004, B-005, B-006 and B-007.

[Experimental Example 2] Evaluation of action of the compound of the present invention on SKOV3 cell proliferation - 2

SKOV3 cells precultured similarly to Experimental Example 1 were suspended in 15% FBS/McCoy's 5a medium (manufactured by Sigma-Aldrich), and dispensed into the wells of a 96 well U-bottom microplate (manufactured by Corning Incorporated, #4515) at 700 cells/90 μL/well. Successively, the compound of the present invention dissolved in dimethyl sulfoxide (DMSO) was diluted with the above-mentioned medium. The diluted solution was added by 10 μL to the above-mentioned cell suspension (90 μL) such that the final concentration of the compound was 10 μM, 20 μM or 40 μM. DMSO alone was added to some wells as a control. Each plate was cultured in a standing state in a $CO_2$ incubator (37°C, 5% $CO_2$) for 4 days. To the culture medium on day 4 was added ATP reagent (100 μL) (CellTiter-Glo (registered trade mark) Luminescent Cell Viability Assay, manufactured by Promega), and the mixture was stirred for 2 min by a plate shaker (manufactured by AS ONE Corporation, Micro plate mixer NS-P) at room temperature, and stood for 10 min. 100 μL was transferred to a 96 well flat bottom white plate (manufactured by Corning, #3912), the luminescence intensity (RLU value) was measured by Enspire (manufactured by Perkin Elmer), and the luminescence value of the medium alone was subtracted to measure the number of viable cells. The numbers of the compounds of the present invention that showed a relative value of not less than 125% at each final concentration when the RLU value (ATP measurement, luminescence intensity) of the control was 100% are shown below.

Number of the compound of the present invention (10 μM); A-004A, A-007, A-007R, A-010, A-011 and A-012.

Number of the compound of the present invention (20 μM); A-001, A-002, A-004, A-005, A-007, A-007R, A-010, A-011, A-012, A-013 and B-005.

Number of the compound of the present invention (40 μM); A-007, A-007R, A-010, A-011, A-012, A-013, A-016, B-004 and B-007.

[Experimental Example 3] Evaluation of action of the compound of the present invention on NIH3T3 cell proliferation - 1

Mouse fetal fibroblast NIH3T3 (manufactured by ATCC) was precultured (single layer culture) in a 10% fetal calf serum (hereinafter to be abbreviated as FCS, manufactured by ATCC)-containing DMEM medium (manufactured by ATCC)). The above-mentioned cells in the logarithmic growth phase were washed with PBS. A 0.25%(w/v) trypsin-1 mmol/L ethylenediaminetetraacetic acid (EDTA) solution (manufactured by Fujifilm Wako Pure Chemical Corporation) was added, and adherent cells were detached by incubating at 37°C for 3 min. The above-mentioned medium was added and the mixture was centrifuged and resuspended in the same medium.

According to the method of patent document 1 (WO 2014/017513), a composition of DMEM medium (manufactured by ATCC) containing 0.015%(w/v) deacylated gellan gum (KELCOGEL CG-LA, manufactured by Sansho Co., Ltd.) and 10%(v/v) FCS was prepared by FCeM-series Preparation Kit (manufactured by FUJIFILM Wako Pure Chemical Corporation). Then, the NIH3T3 cells prepared above were suspended in the above-mentioned medium composition added with deacylated gellan gum, and dispensed into a 96 well flat bottom Ultra-Low Attachment surface microplate (manufactured by Corning Incorporated, #3474) at 2000 cells/90 μL/well. The compound of the present invention dissolved in dimethyl sulfoxide was diluted with the above-mentioned medium, and the diluted solution was added by 10 μL such that the final concentration of the compound of the present invention was 10 μM. Dimethyl sulfoxide (DMSO) alone was added to some wells as a control. Each plate was cultured in a standing state in a $CO_2$ incubator (37°C, 5% $CO_2$) for 4 days. An ATP reagent (100 μL) [CellTiter-Glo (registered trade mark) Luminescent Cell Viability Assay, manufactured by Promega] was added to the culture medium on day 4, and the mixture was stirred by a plateshaker (manufactured by AS ONE Corporation, Micro plate mixer NS-P) at room temperature for 2 min, and left standing for 10 min. 100 μL was transferred to a 96 well flat bottom white plate (manufactured by Corning, #3912), the luminescence intensity (RLU value) was measured by EnSpire (manufactured by Perkin Elmer) and the luminescence value of the medium alone was subtracted to measure the number of viable cells. The numbers of the compounds of the present invention that showed a relative value of not less than 125% at each final concentration when the RLU value (ATP measurement, luminescence intensity) of the control was 100% are shown below.

Number of the compound of the present invention (10 μM); A-004.

[Experimental Example 4] Study of action of the compound of the present invention on MDCK cell

Canine kidney renal tubule epithelial cells (MDCK cells, manufactured by DS PHARMA BIO MEDICAL) were precultured in EMEM medium containing 10% FBS and 1% NEAA. Cold Matrigel (registered trade mark) matrix growth factor reduced (hereinafter sometimes to be denoted as Matrix GFR, manufactured by Corning) was spread on a 24-well plate by 50 μL and fixed by incubating at 37°C for 15 min. The aforementioned MDCK cells were suspended in a medium at a concentration of 11000 cells/mL, cold Matrigel (registered trade mark) Matrix GFR was added at 20 μL/mL and seeded at 0.9 mL/well. The compound of the present invention dissolved in a medium at a final concentration of 10 μM, 20 μM or 40 μM was added by 0.1 mL, and the cells were cultured for 6 days under the conditions of 37°C, 5% $CO_2$ in an incubator. A test plot added with DMSO alone at a final concentration of 0.1% without adding the compound of the present invention was used as a control. Six days later, the size and number of Cysts formed was measured by Cell3iMager (manufactured by SCREEN Holdings Co., Ltd.). The proportion (%) of Cyst with a diameter of not less than 80 μm in Cysts with a diameter of not less than 30 μm was calculated by the following calculation formula.

Proportion (%) of Cyst with diameter of not less than 80 μm
= number of Cyst with diameter of not less than 80 μm/number of Cyst with diameter of not less than 30 μm ×100

In DMSO as a control, the proportion of Cyst with a diameter of not less than 80 μm was 12%. The numbers of the compounds of the present invention that showed a proportion of Cyst with a diameter of not less than 80 μm of not less than 20% at each final concentration are shown below.

Number of the compound of the present invention (10 μM); A-002, A-004, A-004A and B-005.

Number of the compound of the present invention (20 μM); A-002, A-004 and B-001.

Number of the compound of the present invention (40 μM); B-004.

[Experimental Example 5] Observation of Cyst form with confocal fluorescence microscope

As for the respective Matrix GFRs containing Cyst cultured in a medium added with DMSO or A-004A (10 μM) and obtained in Experimental Example 3, the cells were washed with PBS (1 mL/well), 4% paraformaldehyde/PBS (manufactured by FUJIFILM Wako Pure Chemical Corporation) was added (1 mL/well) and fixed at room temperature for 20 min. Thereafter, the supernatant was removed, staining buffer [0.2% Triton X-100 (manufactured by Sigma-Aldrich), 0.05% Tween 20 (manufactured by Sigma-Aldrich)-containing PBS] were added at 1 mL/well, stood for 30 min and removed. Penetration buffer (0.5% Triton X-100 (manufactured by Sigma-Aldrich)/PBS) was added at 1 mL/well and incubated at room temperature for 30 min. The supernatant was removed, washed 3 times every 5 min with staining buffer, blocking buffer (1% bovine serum albumin (BSA manufactured by Sigma-Aldrich)/staining buffer) was added at 0.5 mL/well and incubated for 30 min. The supernatant was removed, an anti-β catenin antibody (manufactured by BD Bioscience) diluted 100-fold with blocking buffer was added at 250 μL/well, and the cells were incubated at room temperature for 60 min. The cells were washed 3 times every 5 min with staining buffer, the secondary antibody (Alexa Fluor 555, manufactured by Thermo Fisher Scientific) and Phalloidin (Alexa Fluor 488, manufactured by Thermo Fisher Scientific), each diluted 250-fold with blocking buffer, were added at 250 μL/well, and the cells were incubated at room temperature in shading for 60 min. After washing 3 times every 5 min with staining buffer, VECTASHIELD Mounting Medium with DAPI (manufactured by Vector Laboratories) was added dropwise, and the cells were observed with a confocal fluorescence microscope (FV1200 IX83, manufactured by Olympus Corporation). As a result of the observation, it was confirmed that Cyst with a normal form was formed during culture in the presence of compound A-004A (Fig. 1).

[Experimental Example 6] Evaluation of kinase inhibitory activity of the compound of the present invention against LATS1 and LATS2

Evaluation of the inhibitory activity (calculation of kinase inhibitory rate) of the compound of the present invention against LATS1 and LATS2 (manufactured by Carna Biosciences) was performed. The activity was measured using the Kinase Assay method described below. The test concentration of the compound of the present invention was set such that the final concentration was 100 nM or 1000 nM.

As the analysis method of the data, the mean signal of the control well containing reaction components other than the compound of the present invention was set to 0% inhibition, the mean signal without enzyme addition was set to 100% inhibition, and the inhibition rate was calculated from the mean signal of 2 wells of each compound.

Kinase Assay method:

2 μL of a compound of the present invention solution at 2.5-fold concentration, 1 μL of substrate at 5-fold concentration (SGKtide: $NH_2$-CKKRNRRLSVA-COOH, manufactured by SignalChem), ATP (manufactured by Sigma-Aldrich), $MgCl_2$ (manufactured by FUJIFILM Wako Pure Chemical Corporation) solution (final concentration 10 μM, 400 μM and 5 mM, respectively), and 2 μL of kinase solution at 2.5-fold concentration (final concentration of LATS1 or LATS2 5 μg/mL), each prepared with assay buffer (20 mM HEPES, 0.01% Triton X-100, 2 mM DTT, 50 μg/mL BSA, pH 7.5), were mixed in the wells of a polypropylene 384-well plate (manufactured by Greiner Bio-One, #784900) and reacted at room temperature for 5 hr. To the solution was added 5 μL detection solution [Fluorospark (registered trade mark) Kinase/ADP Multi-Assay Kit; manufactured by FUJIFILM Wako Pure Chemical Corporation], and the mixture was reacted at room temperature for 30 min. Successively, 5 μL of a reaction quenching liquid [Fluorospark (registered trade mark) Kinase/ADP Multi-Assay Kit; manufactured by FUJIFILM Wako Pure Chemical Corporation] was added to discontinue the reaction. Thereafter, the ADP concentration of the reaction solution was quantified by EnSpire (manufactured by PerkinElmer) to detect the kinase reaction. The kinase inhibitory rate by the compound of the present invention was calculated, and the numbers of the compounds of the present invention that showed an inhibitory rate of not less than 40% are shown below.

inhibitory effect on LATS1:

Number of the compound of the present invention (100 nM); A-001, A-002, A-004, A-004A, A-005, A-006, A-007, A-007R, A-009, A-010, A-011, A-012, A-013, A-014, A-015, A-016, A-017, A-019 and B-005.

Number of the compound of the present invention (1000 nM); A-001, A-002, A-004, A-004A, A-004B, A-005, A-006, A-007, A-007R, A-008, A-009, A-010, A-011, A-012, A-013, A-014, A-015, A-016, A-017, A-018, A-019, A-020, B-001, B-002, B-003, B-004, B-005, B-006, B-007 and C-001.

inhibitory effect on LATS2:

Number of the compound of the present invention (100 nM); A-001, A-002, A-004, A-004A, A-005, A-006, A-007, A-007R, A-009, A-010, A-011, A-012, A-013, A-014, A-016, A-017 and B-005.
Number of the compound of the present invention (1000 nM); A-001, A-002, A-004, A-004A, A-004B, A-005, A-006, A-007, A-007R, A-008, A-009, A-010, A-011, A-012, A-013, A-014, A-015, A-016, A-017, A-018, A-019, A-020, B-001, B-002, B-003, B-004, B-005, B-006, B-007 and C-001.

[Experimental Example 7] Action of the compound of the present invention on human mesenchymal stem cell

Bone marrow-derived human mesenchymal stem cells (BM-hMSC, manufactured by PromoCell GmbH.) were precultured by a single layer culture using a mesenchymal stem cell proliferation medium (manufactured by PromoCell GmbH.). The above-mentioned cells were washed with HEPES-Buffered Saline Solution (manufactured by PromoCell GmbH.), detached by adding 0.04% trypsin/0.03% EDTA solution (manufactured by PromoCell GmbH.) and left standing at room temperature for 3 min. An equal amount of Trypsin Neutralizing Solution (manufactured by PromoCell GmbH.) was added and the cells were recovered. The supernatant was removed by centrifugation, and the cells were resuspended in the same medium.
According to the method of patent document 1 (WO 2014/017513), a composition of a mesenchymal stem cell proliferation medium (manufactured by PromoCell GmbH.) containing 0.015%(w/v) deacylated gellan gum (KELCOGEL CG-LA, manufactured by Sansho Co., Ltd.) was prepared by FCeM-series Preparation Kit (manufactured by FUJIFILM Wako Pure Chemical Corporation). Then, the BM-hMSC cells prepared above were suspended in the above-mentioned medium composition added with deacylated gellan gum, and seeded on a 96 well flat bottom Ultra-Low Attachment surface microplate (manufactured by Corning Incorporated, #3474) at 6000 cells/90 μL/well (3D). The cells were also similarly suspended in a deacylated gellan gum-free medium, and seeded on a 96 well flat bottom adhesion surface microplate (manufactured by Corning Incorporated, #3585) and an EZSPHERE (registered trade mark)-SP 96 well microplate (manufactured by AGC TECHNO GLASS CO., LTD.) each at 2000 cells/90 μL/well (each 2D, EZSPHERE). Successively, the compound of the present invention dissolved in a medium at a final concentration of 10 or 12.5 μM was added at 10 μL/well, and the mixture was cultured for 4 days in an incubator under the conditions of 37°C, 5% $CO_2$. A group added with DMSO alone at a final concentration of 0.1% without adding the compound of the present invention was used as a control. To the culture medium on day 4 was added ATP reagent (100 μL) [CellTiter-Glo (registered trade mark) Luminescent Cell Viability Assay, manufactured by Promega], and the mixture was stirred by a plate shaker (Micro plate mixer NS-P, manufactured by AS ONE Corporation) at room temperature for 2 min, and then stood for 10 min. 100 μL was transferred to a 96 well flat bottom white plate (manufactured by Corning, #3912), the luminescence intensity (RLU value) was measured by Enspire (manufactured by Perkin Elmer), and the luminescence value of the medium alone was subtracted to measure the number of viable cells. The numbers of the compounds of the present invention that showed a relative value of not less than 125% at each final concentration when the RLU value (ATP measurement, luminescence intensity) of the control was 100% are shown below.

2D culture conditions

Number of the compound of the present invention (10 μM); A-005 and A-007.
Number of the compound of the present invention (12.5 μM); A-002, A-001 and A-004.

3D culture conditions

Number of the compound of the present invention (10 μM); A-005 and A-007.
Number of the compound of the present invention (12.5 μM); A-002, B-005, A-001 and A-004.

EZSPHERE culture conditions

Number of the compound of the present invention (10 μM); A-005 and A-007.
Number of the compound of the present invention (12.5 μM); A-002, B-005, A-001 and A-004.

[Experimental Example 8] Action of the compound of the present invention on various cells

Various cells were precultured in each medium shown below. Human liver cancer-derived cell line HuH-7 (manufactured by JCRB cell bank, 10%FBS-containing D-MEM), canine kidney renal tubule epithelial cell-derived cell line MDCK (manufactured by DS Pharma Biomedical Co., Ltd., 1% NEAA- and 10% FBS-containing E-MEM), mouse

fetal fibroblast-derived cell line C3H 10T1/2 (manufactured by Riken BioResource Research Center, 10% FBS-containing BME (manufactured by ThermoFisher)), Chinese hamster ovary-derived cell line CHO-K1 (manufactured by DS Pharma Biomedical Co., Ltd., 10% FBS-containing Ham's F12 (manufactured by FUJIFILM Wako Pure Chemical Corporation)), African green monkey kidney epithelial cell-derived cell line Vero (manufactured by JCRB cell bank, 5%FBS-containing Medium 199 (manufactured by Sigma-Aldrich)), human umbilical vein endothelial cell line HUVEC (manufactured by PromoCell, Endothelial Cell Growth medium (manufactured by PromoCell)). The above-mentioned cells in the logarithmic growth phase were washed with PBS, 0.25%(w/v) trypsin - 1 mmol/L ethylenediaminetetraacetic acid (EDTA) solution (manufactured by FUJIFILM Wako Pure Chemical Corporation) was added and the cells were detached by incubating at 37°C for 2 - 8 min. HUVEC cells were incubated using DetachKit (manufactured by PromoCell) at room temperature for 3 min and similarly detached. After addition of each medium, the cells were centrifuged, resuspended in the same medium, and recovered each as single cells.

According to the method of patent document 1 (WO 2014/017513), a composition of each medium containing 0.015%(w/v) deacylated gellan gum (KELCOGEL CG-LA, manufactured by SANSHO Co., Ltd.) was prepared using FCeM-series Preparation Kit (manufactured by FUJIFILM Wako Pure Chemical Corporation). Then, various cells prepared above were suspended in the above-mentioned medium composition added with deacylated gellan gum or a medium without the addition, and dispensed into the wells of a 96 well flat bottom Ultra-Low Attachment surface microplate (manufactured by Corning, deacylated gellan gum-containing medium) or a 96 well U-bottom Ultra-Low Attachment surface microplate (manufactured by Corning, deacylated gellan gum-free medium) at 700 - 2000 cells/90 μL/well. The compound of the present invention dissolved in dimethyl sulfoxide was diluted with the above-mentioned medium, and the diluted solution was added by 10 μL such that the final concentration of the compound of the present invention was 10 μM. Dimethyl sulfoxide (DMSO) alone was added to some wells as a control. Each plate was cultured in a standing state in a $CO_2$ incubator (37°C, 5% $CO_2$) for 4 days. ATP reagent (100 μL) [CellTiter-Glo (registered trade mark) Luminescent Cell Viability Assay, manufactured by Promega] was added to the culture medium on day 4, and the mixture was stirred by a plate shaker (Micro plate mixer NS-P, manufactured by AS ONE Corporation) at room temperature for 2 min and stood for 10 min. 100 μL was transferred to a 96 well flat bottom white plate (manufactured by Corning, #3912), the luminescence intensity (RLU value) was measured by Enspire (manufactured by Perkin Elmer), and the luminescence value of the medium alone was subtracted to measure the number of viable cells. The numbers of the compounds of the present invention that showed a relative value of not less than 125% at each final concentration when the RLU value (ATP measurement, luminescence intensity) of the control was 100% are shown below.

U-bottom Ultra-Low Attachment surface microplate:

Number of the compound of the present invention (HuH-7 cells); A-001, A-002, A-004, A-005 and A-007.
Number of the compound of the present invention (MDCK cells); A-001, A-002 and A-004.
Number of the compound of the present invention (10T1/2 cells); A-001, A-002, A-004 and B-005.
Number of the compound of the present invention (Vero cells); A-001, A-004 and A-007.
Number of the compound of the present invention (HUVEC cells); A-001, A-002, A-004, A-005, A-007 and B-005.

flat bottom Ultra-Low Attachment surface microplate:

Number of the compound of the present invention (HuH-7 cells); A-001, A-002, A-004, A-005 and A-007.
Number of the compound of the present invention (MDCK cells); A-001, A-002, A-004 and B-005.
Number of the compound of the present invention (10T1/2 cells); A-004.
Number of the compound of the present invention (CHO-K1 cells); A-005 and A-007.
Number of the compound of the present invention (Vero cells); A-001, A-004 and A-007.
Number of the compound of the present invention (HUVEC cells);
A-001, A-002, A-004, A-005, A-007 and B-005.

[Experimental Example 9] Action of the compound of the present invention on A431 cell hanging drop culture method

Human epithelial-like cell cancer-derived cell line (A431, manufactured by ATCC) was precultured by single layer culture using 10% FBS- and 1% NEAA-containing EMEM medium. The above-mentioned cells in the logarithmic growth phase were washed with PBS, a 0.25%(w/v) trypsin - 1 mmol/L ethylenediaminetetraacetic acid (EDTA) solution (manufactured by FUJIFILM Wako Pure Chemical Corporation) was added and the cells were incubated at 37°C for 3 min and detached. The medium was added, the mixture was centrifuged and the supernatant was removed. Successively, the cells were resuspended in the above-mentioned medium at 50000 cells/mL, and a compound to be used in the present invention dissolved in DMSO was further added at a final concentration of 10 μM to the medium. The cell suspension was seeded by 10 μL on the backside of the lid of a 35 mm dish (manufactured by Falcon) (15 drops in total) to form a droplet. In this case, a cell suspension added with DMSO (DMSO concentration 0.05%) was seeded by

10 μL (15 drops in total). The lid was put back to the 35 mm dish added with 2 mL of PBS, and the cells were cultured in an incubator at 37°C, 5% $CO_2$ for 2 days. The cultured droplet was recovered in a 1.5 mL tube, and the medium was added to a final amount of 150 μL. ATP reagent (150 μL) [CellTiter-Glo (registered trade mark) Luminescent Cell Viability Assay, manufactured by Promega] was added, and the mixture was stirred by a plate shaker (Micro plate mixer NS-P, manufactured by AS ONE Corporation) at room temperature for 2 min and stood for 10 min. 100 μL was transferred to a 96 well flat bottom white plate (manufactured by Corning, #3912), the luminescence intensity (RLU value) was measured by Enspire (manufactured by Perkin Elmer), and the luminescence value of the medium alone was subtracted to measure the number of viable cells. The numbers of the compounds of the present invention that showed a relative value of not less than 125% at each final concentration when the RLU value (ATP measurement, luminescence intensity) of the control was 100% are shown below.

Number of the compound of the present invention (10 μM); A-001, A-002, A-004, A-005, A-007R and A-010.

[Experimental Example 10] Action of the compound of the present invention on human pluripotent stem cell (hiPS cell)

hiPS cell 253G1 (distributed by RIKEN) was cultured on a dish coated with iMatrix-511 (manufactured by Nippi, Incorporated) and using StemFit (registered trade mark) AK02N (manufactured by Ajinomoto Co., Inc.) medium. The above-mentioned cells in the logarithmic growth phase were washed with PBS, 0.5 mmol/L-EDTA/PBS solution (Nacalai Tesque) was added, and the mixture was incubated at 37°C for 8-10 min and the cells were detached. The above-mentioned medium was added, and the mixture was centrifuged to remove the supernatant, and the cells were resuspended in the same medium. The above-mentioned cells were suspended in a medium containing 10 μM of Y-27632 (manufactured by FUJIFILM Wako Pure Chemical Corporation), and seeded in a 96 well EZSPHERE-SP plate (manufactured by AGC TECHNO GLASS CO., LTD.) at 16000 cells/180 μL/well. After seeding, the compound of the present invention dissolved in dimethyl sulfoxide was diluted with the above-mentioned medium, and the diluted solution was added by 20 μL such that the final concentration of the compound of the present invention was 12.5 μM. Dimethyl sulfoxide (DMSO) alone was added to some wells as a control. Each plate was cultured in a $CO_2$ incubator (37°C, 5% $CO_2$), and cultured for 4 days while exchanging half the amount (100 μL) of the medium supernatant with a new medium added with the compound at the above-mentioned concentration every day. The supernatant (100 μL) was gently removed by suction with a pipette man from the culture medium on day 4 (200 μL), ATP reagent (100 μL) [CellTiter-Glo (registered trade mark) Luminescent Cell Viability Assay, manufactured by Promega] was added, and the mixture was stirred by a plate shaker (Micro plate mixer NS-P, manufactured by AS ONE Corporation) at room temperature for 2 min, and stood for 10 min. 100 μL was transferred to a 96 well flat bottom white plate (manufactured by Corning, #3912), the luminescence intensity (RLU value) was measured by Enspire (manufactured by Perkin Elmer), and the luminescence value of the medium alone was subtracted to measure the number of viable cells. The numbers of the compounds of the present invention that showed a relative value of not less than 125% at each final concentration when the RLU value (ATP measurement, luminescence intensity) of the control was 100% are shown below.

Number of the compound of the present invention (12.5 μM); A-001, A-002, A-004 and A-005.

[Experimental Example 11] YAP phosphorylation inhibitory action of compound of the present invention

Human ovarian cancer cell line SKOV3 (manufactured by DS Pharma Biomedical Co., Ltd.) was precultured using a 15% FBS-containing McCoy's 5a medium (manufactured by Sigma-Aldrich) (single layer culture). The above-mentioned cells in the logarithmic growth phase were washed with PBS. A 0.25%(w/v) trypsin - 1 mmol/L ethylenediaminetetraacetic acid (EDTA) solution (manufactured by FUJIFILM Wako Pure Chemical Corporation) was added and the adherent cells were incubated at 37°C for 3 min and detached. The above-mentioned medium was added, and the cells were centrifuged and resuspended in the same medium.

According to the method of patent document 1 (WO 2014/017513), a composition of 10% FBS-containing DMEM medium (Phenol-red free, manufactured by FUJIFILM Wako Pure Chemical Corporation) containing 0.015%(w/v) deacylated gellan gum (KELCOGEL CG-LA, manufactured by SANSHO Co., Ltd.) was prepared using FCeM-series Preparation Kit (manufactured by FUJIFILM Wako Pure Chemical Corporation). Then, various cells prepared above were suspended in the above-mentioned medium composition (3D culture conditions) added with deacylated gellan gum or a medium without the addition (2D culture conditions), and seeded on a 50 μL/well (manufactured by Corning Incorporated, a 96 well flat bottom plate, #3585, 2D culture conditions) or 25 μL/well (manufactured by Corning Incorporated, 96 well low adhesion flat bottom plate, #3474) at 100000 cells/well. The plate was allowed to stand at 37°C in a 5% $CO_2$ incubator, and the compound of the present invention diluted with the medium at 2 or 6 times the final concentration, DMSO, or the medium alone was added to the plate 3 hr after the seeding at 50 μL/well (2D culture conditions) or 5 μL/well (3D culture conditions) to make the final concentration of each compound 10 μM. After culture at 37°C in a 5% $CO_2$ incubator for 1 hr, the supernatant was aspirated, supplemented Lysis buffer (1-fold concentration, manufactured by Cisbio, YAP-total kit or YAP phospho-S27 kit) (50 μL) was added under 2D culture conditions, and

supplemented Lysys buffer (4-fold concentration) (10 μL) was added to the culture supernatant under 3D conditions, and the mixture was stirred by a plate shaker for 30 min. Thereafter, the plate was centrifuged, 16 μL of the supernatant was transferred to a 384-well white plate (Corning, #4512), and each antibody solution (pYAP d2 antibody or Total-YAP d2 antibody, and pYAP Cryptate antibody or Total-YAP Cryptate antibody) included in the kit was added at 4 μL/well in total, and the mixture was allowed to stand at room temperature overnight. As a blank control, pYAP Cryptate antibody or Total-YAP Cryptate antibody alone was added to cells free of stimulation with compound and the mixture was treated in the same manner. The next day, the wavelengths at 665 nm and 615 nm were measured in the HTRF mode of EnVision (manufactured by PerkinElmer).

The obtained signal was calculated as follows. Ratio=signal(665 nm)/signal(615 nm)×10000, ΔR=Signal(Ratio)-Background fluorescence, and the relative phosphorylated YAP amount or total YAP amount for each compound when ΔR of the compound non-addition group (non-treatment) is 100% is shown in Tables 7 and 8 (2D culture), and Tables 9 and 10 (3D culture). From the results, it was shown that the compound of the present invention has a YAP phosphorylation inhibitory action under both 2D and 3D culture conditions.

[Table 7]

| 2D culture conditions | | |
|---|---|---|
| compound | phosphorylated YAP amount (%) | total YAP amount (%) |
| no treatment | 100 | 100 |
| DMSO | 99 | 100 |
| A-002 | 50 | 85 |
| A-004 | 19 | 87 |
| A-004A | 14 | 97 |
| A-005 | 27 | 94 |
| A-007 | 16 | 91 |

[Table 8]

| compound | phosphorylated YAP amount (%) | total YAP amount (%) |
|---|---|---|
| A-007R | 8 | 98 |
| A-010 | 11 | 94 |
| A-011 | 46 | 91 |
| A-012 | 19 | 87 |
| A-013 | 42 | 91 |

[Table 9]

| 3D culture conditions | | |
|---|---|---|
| compound | phosphorylated YAP amount (%) | total YAP amount (%) |
| no treatment | 100 | 100 |
| DMSO | 106 | 97 |
| A-002 | 78 | 100 |
| A-004 | 33 | 105 |
| A-004A | 11 | 112 |
| A-005 | 30 | 102 |
| A-007 | 21 | 96 |

[Table 10]

| compound | phosphorylated YAP amount (%) | total YAP amount (%) |
|---|---|---|
| A-007R | 15 | 108 |

(continued)

| compound | phosphorylated YAP amount (%) | total YAP amount (%) |
|---|---|---|
| A-010 | 20 | 114 |
| A-011 | 55 | 98 |
| A-012 | 23 | 103 |
| A-013 | 49 | 96 |

[Experimental Example 12] Adhesion promoting action on HeLa, human primary epidermal keratinocyte and HUVEC cell

Various human-derived cells were cultured using each medium shown below. Human cervical cancer-derived cell line HeLa (manufactured by ATCC, 10% FBS (manufactured by Corning)-containing DMEM (manufactured by Wako Pure Chemical Industries, Ltd.)), human umbilical vein endothelial cell line HUVEC (manufactured by PromoCell, Endothelial Cell Growth medium (manufactured by PromoCell)), human primary epidermal keratinocyte (American Type Culture Collection (ATCC), #PCS-200-010, epidermal cell basal medium (ATCC, #PCS-200-030) added with keratinocyte additive kit (ATCC, #PCS-200-040). The above-mentioned cells in the logarithmic growth phase were washed with PBS, 0.25 w/v% trypsin - 1 mmol/L EDTA (ethylenediaminetetraacetic acid) solution (manufactured by Wako Pure Chemical Industries, Ltd.) was added to HeLa and the mixture was incubated at 37°C for 3 min, HUVEC and human primary epidermal keratinocyte were incubated using DetachKit (manufactured by PromoCell) at room temperature for 3 min. The cells were detached, respective media were added, the mixtures were centrifuged and the supernatant was removed. Thereafter, the cells were resuspended in the same medium, and a portion thereof was suspended in Trypan Blue (manufactured by Wako Pure Chemical Industries, Ltd.) and the number of viable cells was counted using TC-20 (manufactured by BIO-RAD).

A solution of the compound of the present invention dissolved in DMSO was added to each medium in which HeLa or HUVEC was suspended at a final concentration of 10 μM, and seeded on a 96 well adhesion plate (manufactured by Corning Incorporated, #3585) at 8000 cells/100 μL/well. In addition, a solution of the compound of the present invention dissolved in DMSO was added to each medium in which human primary epidermal keratinocyte was suspended at a final concentration of 10 μM, and seeded on a 48 well adhesion plate (manufactured by Corning Incorporated, #3548) at 19000 cells/190 μL/well. As a control, a cell suspension added with DMSO was seeded (DMSO final concentration 0.1%). These plate were subjected to standing culture in an incubator at 37°C, 5% $CO_2$ for 30 min (HeLa and HUVEC) or 60 min (human primary epidermal keratinocyte), the culture medium was aspirated, washed twice with PBS to remove cells not adhered to the culture plate, and each medium was added at 100 or 200 μL/well. Successively, an equal amount of ATP reagent (CellTiter-Glo (registered trade mark) Luminescent Cell Viability Assay, manufactured by Promega) was added and suspended in the culture medium, and the mixture was stood for 10 min at room temperature. The luminescence intensity (RLU value) was measured by EnSpire (manufactured by PerkinElmer) and the luminescence value of the medium alone was subtracted to measure the number of cells adhered to the plate. Those showing a value of not less than 120% when the RLU value (ATP measurement, luminescence intensity) of the group added with DMSO alone was 100% are shown below. The results show that the compound of the present invention has a cell adhesion promoting effect.

cell adhesion promoting effect on HeLa (120% or more):
Number of the compound of the present invention; A-001, A-002, A-004, A-005, A-007 and B-005.

cell adhesion promoting effect on HeLa (150% or more):
Number of the compound of the present invention; A-001, A-002, A-007 and B-005.

cell adhesion promoting effect on HUVEC (120% or more):
Number of the compound of the present invention; A-001, A-002, A-004, A-007 and B-005.

cell adhesion promoting effect on human primary epidermal keratinocyte (200% or more):
Number of the compound of the present invention; A-004, A-004A and A-007.

[Experimental Example 13] Cell proliferation test using human corneal epithelial cell

Human primary corneal epithelial cell (ATCC, #PCS-700-010) was precultured using a corneal epithelial cell basal medium (ATCC, #PCS-700-030) added with corneal epithelial cell additive kit (ATCC, #PCS-700-040) and by a monolayer culture method. After suspending the corneal epithelial cell in the same medium, the compound of the present invention dissolved in DMSO was added to a final concentration of 10 μM (final concentration 5 μM for A-011 and A-012), and the mixture was seeded on a 96 well flat bottom plate (Corning Incorporated, #3585) at 700 cells/64 μL/well in the single layer culture (2D culture conditions). In three-dimensional culture method (3D culture conditions), the cells were seeded in a 96 well U-bottom cell low-adhesion plate (Corning Incorporated, #4520) at 700 cells/64 μL/well. These

plates were cultured at 37°C in a 5% $CO_2$ incubator for 4 days. As a control, DMSO was added to a final concentration of 0.1%. After culturing, the number of viable cells was measured using an ATP reagent (Promega KK, CellTiter-Glo (registered trade mark) Luminescent Cell Viability Assay). ATP reagent was added to the culture medium in the flat bottom plate at 64 μL/well and suspended therein, and the suspension (100 μL/well) was transferred to an assay white plate (Corning Incorporated, #3912). After allowing to stand at room temperature for 10 min, the amount of luminescence was measured using a plate reader (PerkinElmer Inc., EnSpire).

The relative value of the measured luminescence amount with respect to the control group (DMSO) was calculated, and it was found that the luminescence amount increased 1.2 times or more under 2D culture conditions and 4 times or more under 3D culture conditions by adding the compound with the following number. That is, the number of cells increased 2 times or more by the addition of the compound with the following number. From this, it was shown that the compound of the present invention has a promoting effect on the proliferation of human corneal epithelial cells.

Number of compound of the present invention with 1.2 times or more increase in cell count (2D culture conditions): A-004, A-004A, A-005, A-007, A-007R, A-010, A-011, A-012 and A-016.

Number of compound of the present invention with 2 times or more increase in cell count (2D culture conditions): A-004, A-004A and A-007.

Number of compound of the present invention with 4 times or more increase in cell count (3D culture conditions): A-004, A-004A, A-005, A-007, A-007R, A-010, A-011 and A-012.

[Experimental Example 14] Cell proliferation test using bovine corneal endothelial cell

Bovine corneal endothelial cell line BCE C/D-1b (ATCC, #CRL-2048) was precultured using DMEM (ATCC, #30-2002) added with 10% bovine serum (ATCC, #30-2030). Corneal endothelial cells were suspended in the same medium, and seeded on a 96 well flat bottom plate (Corning Incorporated, #3585) at 700 cells/90 μL/well by a single layer culture method (2D culture conditions). In the three-dimensional culture method (3D culture conditions), the cells were seeded in a 96 well U-bottom cell low-adhesion plate (Corning Incorporated, #4520) at 700 cells/90 μL/well. Successively, the compound of the present invention dissolved in DMSO at a final concentration of 10 μM was added at 10 μL/well, and the cells were cultured for 4 days in an incubator at 37°C, 5% $CO_2$. As a control, DMSO was added at a final concentration of 0.1%. After culturing, the number of viable cells was measured using an ATP reagent (Promega KK, CellTiter-Glo (registered trade mark) Luminescent Cell Viability Assay). ATP reagent was added to the culture medium at 100 μL/well and suspended therein, and the suspension (100 μL/well) was transferred to an assay white plate (Corning Incorporated, #3912). After allowing to stand at room temperature for 10 min, the amount of luminescence was measured using a plate reader (PerkinElmer Inc., EnSpire).

The relative value of the measured luminescence amount with respect to the control group (DMSO) under respective 2D and 3D culture conditions was calculated, and it was found that the luminescence amount increased 1.2 times or more under 2D and 2 times or more under 3D by adding the following compounds. That is, the number of cells increased 2 times or more by the addition of the compound with the following number. From this, it was shown that the compound of the present invention has a promoting effect on the proliferation of corneal epithelial cells.

Number of compound of the present invention with 1.2 times or more increase in cell count (2D culture conditions): A-004, A-004A, A-005, A-007, A-007R, A-010, A-011 and A-012.

Number of compound of the present invention with 4 times or more increase in cell count (3D culture conditions): A-007, A-007R and A-012.

Number of compound of the present invention with 3 times or more increase in cell count (3D culture conditions): A-004A, A-007, A-007R, A-010 and A-012.

Number of compound of the present invention with 2 times or more increase in cell count (3D culture conditions): A-004, A-004A, A-007, A-007R, A-010, A-011 and A-012.

[Experimental Example 15] Cell proliferation test using human primary epidermal keratinocyte

Human primary epidermal keratinocyte (ATCC, #PCS-200-010) was precultured using an epidermal cell basal medium (ATCC, #PCS-200-030) supplemented with a keratinocyte additive kit (ATCC, #PCS-200-040) and by a monolayer culture method. After suspending the epidermal keratinocytes in the same medium, the compound of the present invention dissolved in DMSO was added to a final concentration of 10 μM and the mixture was seeded in a 96 well U-bottom cell low-adhesion plate (Corning Incorporated, #4520) at 700 cells/64 μL/well. The cells were cultured at 37°C in a 5% $CO_2$ incubator for 4 days. As a control, DMSO was added to a final concentration of 0.1%. After culturing, the

number of viable cells was measured using an ATP reagent (Promega KK, CellTiter-Glo (registered trade mark) Luminescent Cell Viability Assay). ATP reagent was added to the culture medium in the flat bottom plate at 64 μL/well and suspended therein, and the suspension (100 μL/well) was transferred to an assay white plate (Corning Incorporated, #3912). After allowing to stand at room temperature for 10 min, the amount of luminescence was measured using a plate reader (PerkinElmer Inc., EnSpire).

The relative value of the measured luminescence amount with respect to the control group (DMSO) was calculated, and it was found that the luminescence amount increased 4 times or more by adding the compound of the following number. That is, the number of cells increased 4 times or more by the addition of the compound of the following number. From this, it was shown that the compound of the present invention has an effect of promoting the proliferation of corneal endothelial cells.

Number of compound of the present invention with 4 times or more increase in cell count: A-004, A-004A and A-007.
Number of compound of the present invention with 6 times or more increase in cell count: A-004A and A-007.

[Experimental Example 16] Action of the compound of the present invention on gene expression of SKOV3 cell, human primary corneal epithelial cell and human primary epidermal keratinocyte

Similar to Experimental Example 13 (proliferation test using human primary corneal epithelial cell), human primary corneal epithelial cell (ATCC, #PCS-700-010) was precultured using a corneal epithelial cell basal medium (ATCC, #PCS-700-030) added with corneal epithelial cell additive kit (ATCC, #PCS-700-040) and by a single layer culture method. As regards human primary epidermal keratinocyte, similar to Experimental Example 15 (proliferation test using human primary epidermal keratinocyte), human primary epidermal keratinocyte (ATCC, #PCS-200-010) was precultured using a epidermal cell basal medium (ATCC, #PCS-200-030) supplemented with a keratinocyte additive kit (ATCC, #PCS-200-040) and by a single layer culture method. The corneal epithelial cell and epidermal keratinocyte were seeded on a 24 well flat bottom plate (Corning Incorporated, #3526) at 50,000 cells/380 μL/well and cultured at 37°C in a 5% $CO_2$ incubator for 24 hr. Then, the medium was exchanged with a medium containing, at a final concentration of 10 μM (final concentration 5 μM for A-011 and A-012), the compound of the present invention dissolved in DMSO, and the cells were further cultured for 24 hr. After culturing, the cell culture medium was removed, D-PBS (FUJIFILM Wako Pure Chemical Corporation, #049-29793) was added at 500 μL/well and removed. Successively, total RNA (ribonucleic acid) was isolated from the cell using RNeasy Mini Kit (manufactured by Qiagen). Gene expression analysis of the RNA was performed using TaqmanAssay (AppliedBioSystems), and changes in the human gene expression at the downstream of YAP and TAZ proteins due to the addition of the compound of the present invention were analyzed. The analyzed gene name and the AssayID of the probe used are shown below.

gene name AssayID
GAPDH Hs02758991_g1
CYR61 Hs00155479_m1
ANKRD1 Hs00173317_m1
CCND1 Hs00765553_m1

Human ovarian cancer cell line SKOV3 (manufactured by DS Pharma Biomedical Co., Ltd.) was precultured (single layer culture) in a 15% FBS-containing McCoy's 5a medium (manufactured by Sigma-Aldrich). The above-mentioned cells in the logarithmic growth phase were washed with PBS. To the adherent cell was added 0.25%(w/v) trypsin - 1 mmol/L ethylenediaminetetraacetic acid (EDTA) solution (manufactured by FUJIFILM Wako Pure Chemical Corporation) and the cells were incubated at 37°C for 3 min and detached. The above-mentioned medium was added, and the cells were centrifuged and resuspended in the same medium.

According to the method of patent document 1 (WO 2014/017513), a composition of 15% FBS-containing McCoy's 5a medium containing 0.015%(w/v) deacylated gellan gum (KELCOGEL CG-LA, manufactured by SANSHO Co., Ltd.) was prepared using FCeM-series Preparation Kit (manufactured by FUJIFILM Wako Pure Chemical Corporation), and EGF (manufactured by PeproTech Inc.) was added at a final concentration of 30 ng/mL. Then, the cells prepared above were suspended in the above-mentioned medium composition added with deacylated gellan gum, and seeded in an Ultra-Low Attachment surface 24 well plate (manufactured by Corning, #3473) at 300000 cells/1 mL/well. After allowing to stand in an incubator at 37°C, 5% $CO_2$ overnight, the compound of the present invention, DMSO, or a medium alone was added to the plate to make the final concentration of each compound 10 μM. 24 hr after addition of the compound, the cells and the culture medium were recovered into tubes from the plate. Cold D-PBS (FUJIFILM Wako Pure Chemical Corporation, #049-29793) was added, and the mixture was centrifuged at 400 g for 3 min and the

supernatant was removed. Successively, total RNA (ribonucleic acid) was isolated from the cell using RNeasy Mini Kit (manufactured by Qiagen). Gene expression analysis of the RNA was performed using TaqmanAssay (AppliedBioSystems), and changes in the human gene expression at the downstream of YAP and TAZ proteins due to the addition of the specific compound were analyzed. The analyzed gene name and the AssayID of the probe used are shown below.

gene name AssayID
GAPDH Hs02758991_g1
CYR61 Hs00155479_m1
ANKRD1 Hs00173317_m1
CCND1 Hs00765553_m1
CTGF Hs00170014_m1

The relative value of the obtained expression level with respect to GAPDH was calculated. Furthermore, the relative expression level with the addition of the compound of the present invention was calculated where the relative value (expression level) with the addition of DMSO was 1. As a result, the relative expression level increased as compared to the control group (DMSO) by the addition of the compound of the present invention. That is, it was clarified that the compound of the present invention has an effect of increasing the expression level of a gene whose expression is regulated by YAP protein.

Number of compound of the present invention with 2 times or more increase in CYR61 relative expression level (human primary corneal epithelial cell): A-004, A-004A, A-005, A-007, A-007R, A-010, A-011, A-012 and A-016.
Number of compound of the present invention with 2 times or more increase in CYR61 relative expression level (human primary epidermal keratinocyte): A-004, A-004A and A-007.
Number of compound of the present invention with 30 times or more increase in CYR61 relative expression level (SKOV3 cell): A-004, A-004A, A-007, A-007R and A-010.
Number of compound of the present invention with 2 times or more increase in ANKRD relative expression level (human primary corneal epithelial cell): A-004, A-004A, A-007, A-007R and A-010.
Number of compound of the present invention with 5 times or more increase in ANKRD relative expression level (human primary corneal epithelial cell): A-004, A-004A, A-007 and A-007R. Number of compound of the present invention with 5 times or more increase in ANKRD relative expression level (human primary epidermal keratinocyte): A-004, A-004A and A-007.
Number of compound of the present invention with 100 times or more increase in ANKRD relative expression level (SKOV3 cell): A-004, A-004A, A-007, A-007R and A-010.
Number of compound of the present invention with 1.5 times or more increase in CCND1 relative expression level (human primary corneal epithelial cell): A-004, A-007, A-007R, A-010, A-011, A-012 and A-016.
Number of compound of the present invention with 1.5 times or more increase in CCND1 relative expression level (human primary epidermal keratinocyte): A-004, A-004A and A-007.
Number of compound of the present invention with 4 times or more increase in CCND1 relative expression level (SKOV3 cell): A-004, A-004A, A-007, A-007R and A-010.
Number of compound of the present invention with 20 times or more increase in CTGF relative expression level (SKOV3 cell): A-004, A-004A, A-007, A-007R and A-010.

[Experimental Example 17] Action of compound of the present invention on nuclear translocation of YAP protein and TAZ protein

The compound of the present invention was evaluated for the nuclear translocation of YAP protein and TAZ protein. For the nuclear translocation, imaging analysis was performed using the human ovarian cancer cell line SKOV3 (DS Pharma Biomedical Co., Ltd) and OperettaCLS (PerkinElmer) described below. The specific compound was added such that the final concentration of the test concentration of the compound of the present invention was 10 $\mu$M.

Imaging analysis:

Human ovarian cancer cell line SKOV3 cultured according to the protocol recommended by DS Pharma Biomedical Co., Ltd. was seeded on a 96-well plate (PerkinElmer, CellCarrier-Ultra) at 15,000 cells/90 $\mu$L/well on the cycloolefin bottom surface, and cultured in a $CO_2$ incubator (37°C, 5% $CO_2$) for 1 day. After culturing, a solution of the compound to be used in the present invention dissolved in DMSO was added. The cells were further cultured in a $CO_2$

incubator at 37°C for 4 hr, the medium was exchanged with 4% para-formaldehyde, and the cells were fixed by allowing to stand at room temperature for 10 min. The fixed cells were subjected to immunostaining with AlexaFluor(R) 647-labeled YAP antibody (Cell Signaling Technology), anti-mouse TAZ (D-8) antibody (SantaCruz), AlexaFluor (R) 555-labeled anti-mouse IgG antibody (Thermo Fisher Scientific), according to the recommended protocol produced by Cell Signaling Technology, and the cell nucleus was further stained with 10 μg/mL Hoechst33342 solution.

Nine fields of view were imaged per well using a 20x objective lens of Operatta CLS. The cell nucleus region and the cytoplasm region were identified from the obtained fluorescence images by using the analysis software Harmony (PerkinElmer), and YAP protein-AlexaFluor(R)647 fluorescence intensity and TAZ protein-AlexaFluor(R)555 fluorescence intensity in each region were extracted. Furthermore, the ratio of the fluorescence intensity of the cell nucleus region to the fluorescence intensity of the cytoplasmic region was calculated from the extracted values. This time, the case where the fluorescence intensity ratio was 1.4 or more was defined as the cell population in which the YAP protein and the TAZ protein were transferred into the nucleus, and the ratio thereof was calculated.

The relative value of the calculated ratio of the cell population with respect to the control group (DMSO) was calculated, and it was found that the cell population increased 5 times or more for YAP protein and 1.5 times or more for TAZ protein by the addition of the compound of the following number. Therefrom it was clarified that the compound of the present invention has an action of promoting the nuclear translocation of the YAP protein and TAZ protein.

Number of compound of the present invention with 5 times or more increase in cell population in which YAP protein has transferred into nucleus: A-004, A-004A and A-007.

Number of compound of the present invention with 1.5 times or more increase in cell population in which TAZ protein has transferred into nucleus: A-004, A-004A and A-007.

[Experimental Example 18] Action on liver weight increase in mouse partial liver resection model

A partial liver resection model in which the mouse liver was partially resected was prepared as described below, and the liver weight increasing effect of the compound of the present invention was evaluated by comparing the liver weight immediately after the resection with the liver weight 3 days after the resection.

Evaluation sample:

A 0.5w/v% methylcellulose aqueous solution (0.5% MC, manufactured by FUJIFILM Wako Pure Chemical Corporation) and A-007 were evaluated. A-007 was suspended in 0.5% MC, and prepared at a final concentration of 1 mg/mL.

Procedure:

Mice, BALB/cAnNCrlCrlj, 5-week-old, male, were purchased from CHARLES RIVER LABORATORIES JAPAN. In terms of the 0.5% MC administration-control group, 0.5% MC 10 mL/kg was administered intraperitoneally once, and 24 hr later, the entire liver was isolated and the liver weight was measured (n=3). As used herein, the control group means a group without removal of the liver. In terms of the 0.5% MC administration-liver resection group, 0.5% MC 10 mL/kg was intraperitoneally administered 4 times in total every 24 hr.

Immediately after the second administration, the abdomen was opened under isoflurane (manufactured by Intervet Co., Ltd.) anesthesia, and the root of liver lobe was ligated with a suture thread, and about 30% of the liver was finally removed by excising the tip of the ligated liver lobe. After that, the abdomen was closed, and 24 hr after the final administration, the entire liver was removed and the liver weight was measured (n=3). In terms of the A-007 administration-control group, 10 mg/kg A-007 was intraperitoneally administered once, and 24 hr later, the entire liver was isolated and the liver weight was measured (n=3). In terms of the A-007-liver resection group, 10 mL/kg A-007 was intraperitoneally administered continuously 4 times in total every 24 hr. Immediately after the second administration, the abdomen was opened under isoflurane anesthesia, the root of liver lobe was ligated with a suture thread, and about 30% of the liver was finally removed by excising the tip of the ligated lobe. After that, the abdomen was closed, and 24 hr after the final administration, the entire liver was removed and the liver weight was measured (n=3).

As the result of this analysis, Fig. 2 shows changes in the liver weight ratio of the partial liver resection mouse administered with 0.5% MC, A-007. As shown in Fig. 2, it was clarified that A-007 has an action of promoting a liver weight increase.

[Example 19] Inflammation suppressive effect on colitis model mouse

Colitis model administered with dextran sulfate sodium (DSS) (manufactured by FUJIFILM Wako Pure Chemical Corporation) was produced as described below. The length of the large intestine is used as a morphological parameter in the evaluation of colitis, and the large intestine shortens according to the degree of inflammation. Therefore, the antiinflammatory effect of the compound was evaluated by comparing the length of the large intestine.

evaluation sample:

As the Buffer, a 0.5 w/v% methylcellulose aqueous solution (manufactured by FUJIFILM Wako Pure Chemical Corporation) added with Dimethyl sulfoxide (manufactured by FUJIFILM Wako Pure Chemical Corporation) at 1 v/v% was used. It was prepared by suspending compound A-004A in the above-mentioned Buffer at 5 mg/mL.

Procedure:

As the mouse, male C57BL/6NCrl, 6-week-old, was purchased from CHARLES RIVER LABORATORIES JAPAN, INC. As drinking water, distilled water or a 2.5 w/v% DSS aqueous solution was freely given. The DSS drinking start date was set as day 1, and the water was given until day 8. Compound A-004A was intraperitoneally administered at 50 mg/kg every 24 hr from day 1. As a negative control, Buffer alone was administered similarly. On day 8, the mouse was dissected, the large intestine was isolated and the length of the large intestine was measured. Conducted in 3-4 cases in each group.

As the result of this test, Fig. 3 shows the length of the large intestine of the mouse administered with Buffer and compound A-004A. As shown in Fig. 3, the large intestine of the DSS drinking water - Buffer administration group was shorter than that of the distilled water drinking group. The shortening of the large intestine was suppressed more in the DSS drinking water - compound A-004A administration group. From the above, it was clarified that compound A-004A has an effect of suppressing the onset of DSS-derived colitis.

[Example 20] Action on mouse intestine organoid culture

7-Week-old male C57BL/6N mice were purchased from CHARLES RIVER LABORATORIES JAPAN, INC. After the acclimation period, the small intestine and colon were isolated under anesthesia. Crypts were collected from the excised small intestine and colon according to the protocol of IntestiCult (trade mark) Organoid Growth Medium. To be specific, the inside of the intestine was washed with 1 mL of cold PBS, then incised vertically, and washed 3 times with 1 mL of cold PBS. The intestine was transferred to a 10 cm petri dish filled with cold PBS, washed thoroughly again, and transferred to a 50 mL tube containing 15 mL of cold PBS while cutting into 2 mm pieces. The pieces were stirred 3 times with a 10 mL pipette and allowed to stand for 30 sec (after sedimentation of the pieces), then the supernatant was gently removed. This was repeated 15 times or more. After removal of the supernatant, the pieces were incubated with 25 mL of Gentle Cell Dissociation Reagent (STEMCELL Technologies) for 15 min (small intestine) or 20 min (colon) with stirring at room temperature at 20 rpm. After standing and sedimentation of the pieces, the supernatant was removed, and the mixture was stirred with 10 mL of 0.1% BSA/cold PBS three times. After sedimentation of the pieces, the supernatant was taken and collected in a tube through a 70 $\mu$m cell strainer. This was repeated three more times to produce Flactions 1 - 4. Centrifugation was performed at 4°C, 290 g$\times$5 min, the supernatant was removed, the mixture was suspended again in 10 mL of 0.1% BSA/cold PBS, and transferred to a 15 mL tube. After centrifugation at 4°C, 200 g$\times$3 min, the supernatant was removed to produce pellets. To the aforementioned pellets was added 10 mL of cold DMEM/F-12 medium for resuspending, and 1 mL thereof was transferred to a 6-well plate and observed under a microscope to determine Flactions rich in crypts. The number of crypts contained in 10 $\mu$L of the suspension was counted under a microscope to determine the concentration, and the mixture was dispensed into a 15 mL tube at 800 crypts/tube, and centrifuged at 4°C, 200 g$\times$5 min. The supernatant was removed, and 200 $\mu$L of IntestiCult (trade mark) Organoid Medium was added to each tube and the mixture was stirred. Furthermore, 200 $\mu$L of Matrigel GFR (Corning) was added and mixed, and then dispensed by 50 $\mu$L into a 24 well plate preheated to 37°C and warmed in an incubator at 37°C for 10 min to allow solidification into a dome shape. 750 $\mu$L of IntestiCult (trade mark) Organoid Medium was added to each well, and A-007 dissolved in DMSO was added at a final concentration of 10 $\mu$M (0.025% or less in terms of DMSO). DMSO alone was added to some wells as a control. Successively, the cells were subjected to standing culture in an incubator at 37°C, 5% $CO_2$, and the medium was changed every 2-3 days. Organoids were image-analyzed over time by Cell3iMager duos (SCREEN Holdings Co., Ltd.). For image analysis, the number of crypts having a diameter of 30-200 $\mu$m was counted as the number of seeded crypts on the first day of culture, and then those grown to 70 $\mu$m or more over time were counted as organoids, and the organoid formation rate from the crypts was calculated. At that time, the average diameter of the organoids measured at the same time was calculated. The organoid formation rate (%) and

average diameter (μm) are shown in the following Table.

[Table 11]

| sample | compound | formation rate (%) | average diameter (μm) |
|---|---|---|---|
| small intestine organoid day 9 of culture | DMSO | 39 | 202 |
| | A-007 | 70 | 257 |
| colon organoid day 21 of culture | DMSO | 14 | 207 |
| | A-007 | 49 | 251 |

In addition, the culture supernatants of the small intestine organoid on day 9 of culture and the colon organoid on day 21 of culture were removed, and total RNA (ribonucleic acid) was isolated from the cells by using RNeasy Mini Kit (manufactured by Qiagen). For this RNA, cDNA was synthesized using PrimeScript RT Master Mix manufactured by Takara Bio Inc., gene expression analysis was performed using TaqmanAssay (AppliedBioSystems), and the gene expression levels of small intestine, colon or intestinal tract specific gene in organoids added with DMSO or A-007 were analyzed. Furthermore, the expression level was compared with that of the internal standard glyceraldehyde-3-phosphate dehydrogenase gene (Gapdh). The genes and samples confirmed to have a relative expression level of 0.5 or more when the expression level of Gapdh was 1 are shown below.

small intestine organoid
small intestine specific gene Lyz1; DMSO, A-007.
colon organoid
colon specific gene Muc2; DMSO, A-007.
small intestine organoid and colon organoid
intestine specific gene Vil1; DMSO, A-007.

From the above results, A-007 drastically improved the formation rate of mouse small intestine and colon organoids, and also increased the average diameter. In addition, the formed small intestine and colon organoids were each confirmed to express a site-specific marker gene. From the above, it was clarified that A-007 has an effect of promoting the formation of intestine organoids.

[Industrial Applicability]

According to the present invention, promotion of cell proliferation can be achieved. The cells and the like prepared according to the present invention are highly useful, for example, in the field of drug discovery. In addition, according to the present invention, diseases or tissue damages associated with cell proliferation failure can be treated.

This application is based on a patent application No. 2019-014899 filed in Japan (filing date: January 30, 2019) and a patent application No. 2019-103322 filed in Japan (filing date: May 31, 2019), the contents of which are incorporated in full herein.

## Claims

1. A hydrazide compound represented by the formula (I);

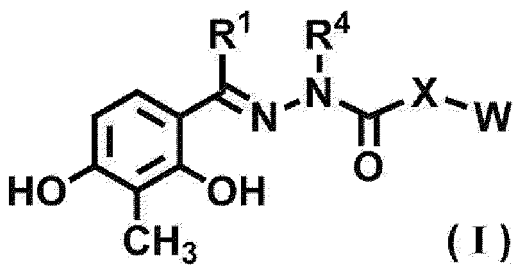

(I)

wherein X is -N($R^5$)C($R^2$)($R^3$)-, -C($R^2$)($R^3$)N($R^5$)- or -C($R^2$)($R^3$)O-,
W is a ring represented by any of the following D-1, D-2, D-3, D-4, D-5, D-6, D-7, D-8, D-9, D-10, D-11 and D-12,

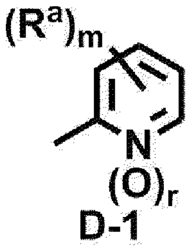
D-1

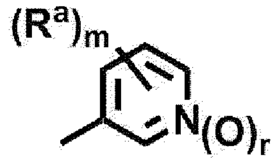
D-2

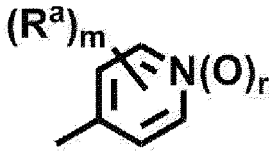
D-3

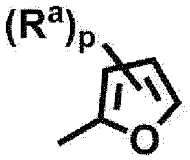
D-4

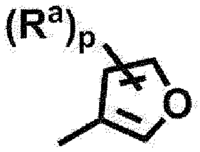
D-5

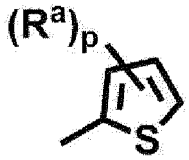
D-6

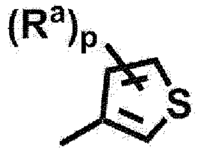
D-7

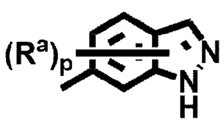
D-8

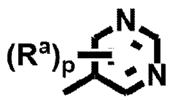
D-9

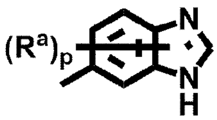
D-10

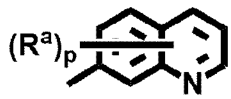
D-11

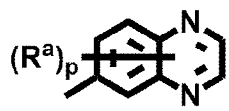
D-12

$R^1$ is $C_1$ - $C_6$ alkyl or halo ($C_1$ - $C_6$)alkyl,
$R^2$ and $R^3$ are each independently a hydrogen atom or $C_1$ - $C_6$ alkyl,
$R^4$ and $R^5$ are each independently a hydrogen atom or $C_1$ - $C_6$ alkyl,
$R^a$ is hydroxy, nitro, a halogen atom, $C_1$ - $C_6$ alkyl, halo ($C_1$ - $C_6$)alkyl, $C_2$ - $C_6$ alkenyl, $C_2$ - $C_6$ alkynyl, $C_1$ - $C_6$ alkoxy, halo ($C_1$ - $C_6$)alkoxy, $C_1$ - $C_6$ alkylthio, halo ($C_1$ - $C_6$)alkylthio, $C_1$ - $C_6$ alkylsulfinyl or $C_1$ - $C_6$ alkylsulfonyl,
m is 0, 1, 2, 3 or 4,
p is 0, 1, 2 or 3, and
r is 0 or 1, or a salt thereof.

**2.** The hydrazide compound according to claim 1, wherein

X is -C($R^2$)($R^3$)N($R^5$)-,
W is a ring represented by any of D-1, D-2 and D-3,
$R^4$ and $R^5$ are each a hydrogen atom,
$R^a$ is $C_1$ - $C_6$ alkyl or a halogen atom,
m is 0 or 1, and
r is 0, or a salt thereof.

**3.** The hydrazide compound according to claim 1, wherein

X is -N($R^5$)C($R^2$)($R^3$)-,
W is a ring represented by any of D-2, D-4, D-5, D-6 and D-7,
$R^2$, $R^3$, $R^4$ and $R^5$ are each a hydrogen atom, and
p, m and r are each 0, or a salt thereof.

**4.** The hydrazide compound according to claim 1, wherein

X is -C($R^2$)($R^3$)O-,
W is a ring represented by D-2, and
m and r are each 0, or a salt thereof.

5. The hydrazide compound according to claim 1, wherein

X is -C($R^2$) ($R^3$)N($R^5$)-,
W is a ring represented by any of D-8, D-9, D-10, D-11 and D-12,
$R^4$ and $R^5$ are each a hydrogen atom, and
p is 0, or a salt thereof.

6. A composition for addition to a medium, comprising the hydrazide compound according to any one of claims 1 to 5 or a salt thereof.

7. The composition according to claim 6, wherein the composition is for promoting cell proliferation.

8. The composition according to claim 7, wherein the cell is selected from the group consisting of a normal cell line, a cancer cell line and a stem cell.

9. The composition according to claim 6, wherein the composition is used for promoting sphere formation, cyst formation or organoid formation.

10. The composition according to claim 6, wherein the composition is used for producing a cell for transplantation or an organoid for transplantation.

11. The composition according to claim 6, wherein the composition is for promoting cell adhesion.

12. A cell culture medium comprising the hydrazide compound according to any one of claims 1 to 5 or a salt thereof.

13. A method for promoting cell proliferation, comprising adding the hydrazide compound according to any one of claims 1 to 5 or a salt thereof to a cell culture medium.

14. The method according to claim 13, wherein the cell is selected from the group consisting of a normal cell line, a cancer cell line and a stem cell.

15. A method for promoting cell adhesion, comprising adding the hydrazide compound according to any one of claims 1 to 5 or a salt thereof to a cell culture medium.

16. A kinase inhibitor, comprising the hydrazide compound according to any one of claims 1 to 5 or a salt thereof.

17. The kinase inhibitor according to claim 16, wherein the kinase is LATS.

18. A Hippo signal transduction pathway inhibitor, comprising the hydrazide compound according to any one of claims 1 to 5 or a salt thereof.

19. A pharmaceutical composition comprising the hydrazide compound according to any one of claims 1 to 5 or a salt thereof.

20. The pharmaceutical composition according to claim 19, wherein the composition is used for treating a disease in eye, liver, skin or intestine.

Fig. 1

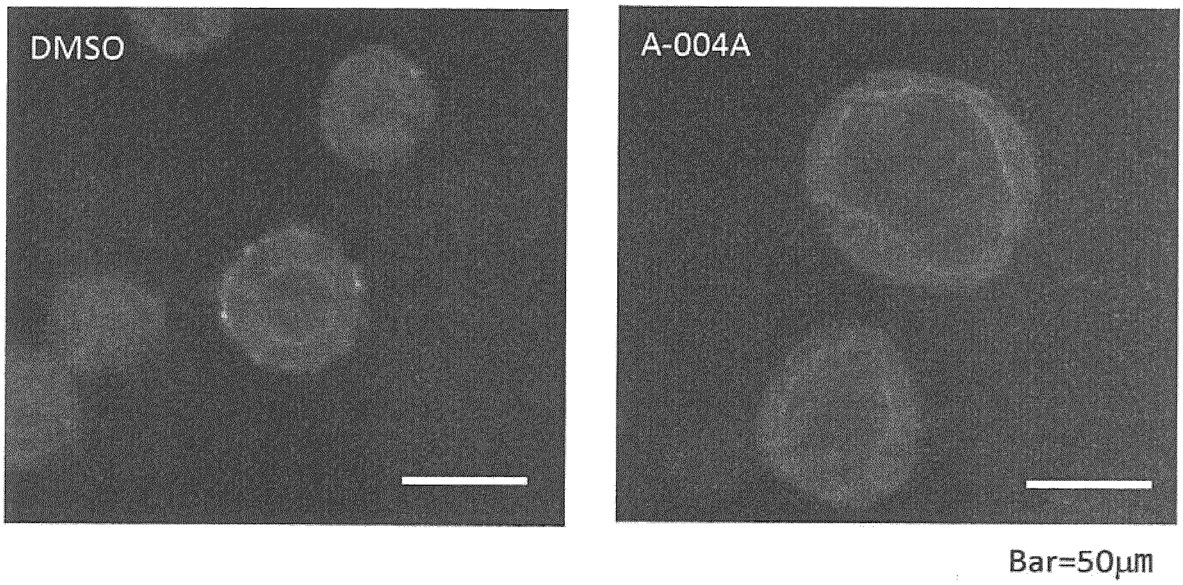
DMSO
A-004A
Bar=50μm

Fig. 2

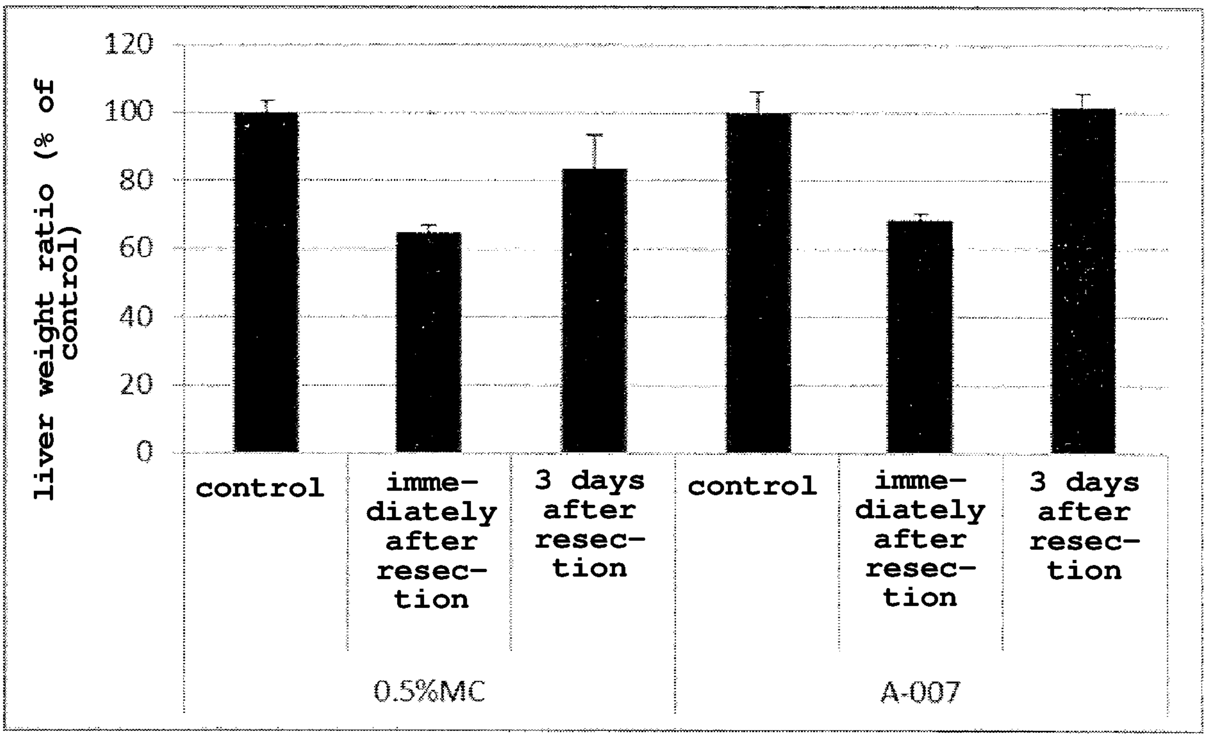
liver weight ratio (% of control)
120
100
80
60
40
20
0
control
imme-diately after resec-tion
3 days after resec-tion
control
imme-diately after resec-tion
3 days after resec-tion
0.5%MC
A-007

Fig. 3

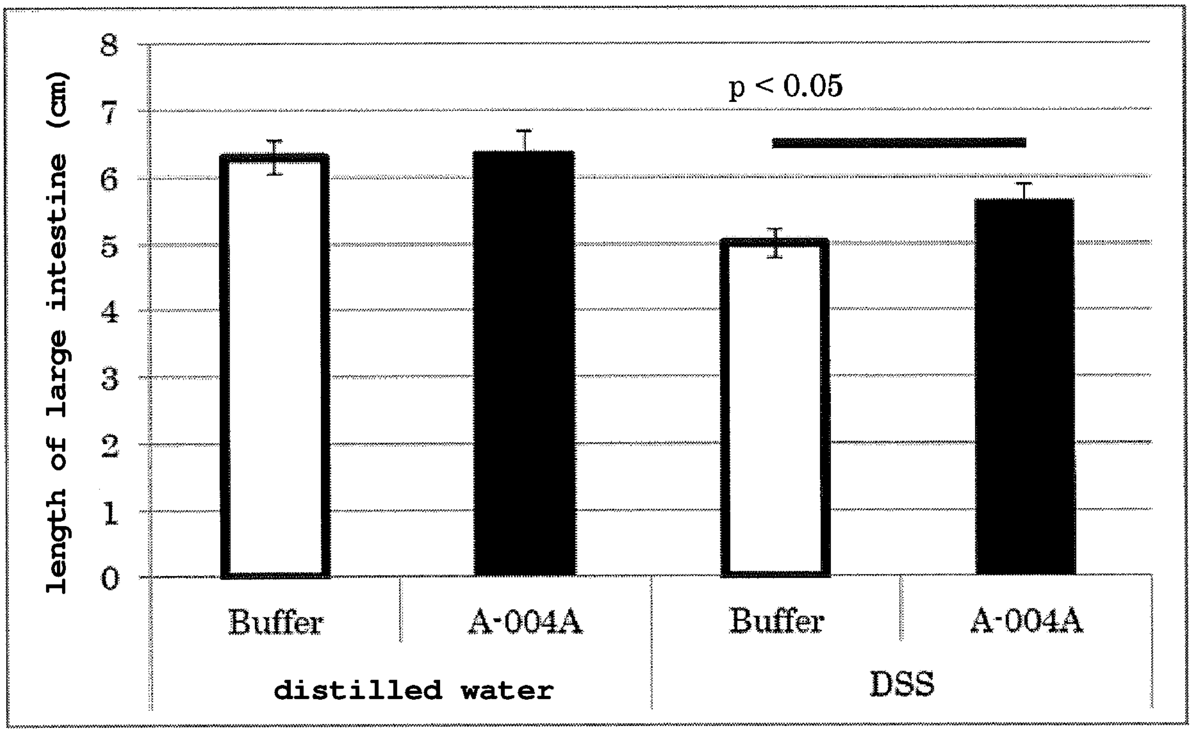
length of large intestine (cm)
8
7
6
5
4
3
2
1
0
p < 0.05
Buffer
A-004A
Buffer
A-004A
distilled water
DSS

| INTERNATIONAL SEARCH REPORT | International application No. PCT/JP2020/003311 |
|---|---|

A. CLASSIFICATION OF SUBJECT MATTER

A61P 1/04(2006.01)i; A61P 1/16(2006.01)i; A61P 9/10(2006.01)i; A61P 21/02(2006.01)i; A61P 21/04(2006.01)i; A61P 25/00(2006.01)i; A61P 25/28(2006.01)i; A61P 27/02(2006.01)i; A61P 29/00(2006.01)i; A61P 43/00(2006.01)i; C07D 235/04(2006.01)i; C07D 333/20(2006.01)i; A61P 17/00(2006.01)i; A61P 17/02(2006.01)i; C07D 213/40(2006.01)i; C07D 213/65(2006.01)i; C07D 213/74(2006.01)i; C12N 5/00(2006.01)i; C12N 5/07(2010.01)i; C07D 239/28(2006.01)i; C07D 307/52(2006.01)i; C12N 9/99(2006.01)i; A61K 31/341(2006.01)i; A61K 31/381(2006.01)i; A61K 31/44(2006.01)i; A61K 31/4402(2006.01)i; A61K 31/4406(2006.01)i; A61K 31/4409(2006.01)i

FI: C07D213/40 CSP; C12N9/99; C07D213/74; A61K31/4402; A61K31/4406; A61K31/4409; C07D213/65; C07D307/52; A61K31/341; C07D333/20; A61K31/381; A61P43/00 111; A61P29/00; A61P25/28; A61P25/00; A61P21/02; A61P21/04; A61P17/02; A61P1/16; A61P1/04; A61P9/10; A61P27/02; C12N5/00; A61P17/00; A61K31/44; C12N5/07; C07D235/04; C07D239/28

According to International Patent Classification (IPC) or to both national classification and IPC

B. FIELDS SEARCHED

Minimum documentation searched (classification system followed by classification symbols)

C07D213/40; A61K31/341; A61K31/381; A61K31/44; A61K31/4402; A61K31/4406; A61K31/4409; A61P1/04; A61P1/16; A61P9/10; A61P17/00; A61P17/02; A61P21/02; A61P21/04; A61P25/00; A61P25/28; A61P27/02; A61P29/00; A61P43/00; C07D213/65; C07D213/74; C07D235/04; C07D239/28; C07D307/52; C07D333/20; C12N5/00; C12N5/07; C12N9/99

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2009/154201 A1 (KYOTO UNIVERSITY) 23.12.2009 (2009-12-23) claims | 1-20 |

☒ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 24 March 2020 (24.03.2020) | 07 April 2020 (07.04.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/003311

C (Continuation). DOCUMENTS CONSIDERED TO BE RELEVANT

| Category* | Citation of document, with indication, where appropriate, of the relevant passages | Relevant to claim No. |
|---|---|---|
| A | JP 2018-530594 A (INVENTIVA) 18.10.2018 (2018-10-18) claims | 1-20 |
| A | US 5990141 A (SUGEN INC.) 23.11.1999 (1999-11-23) fig. 1E | 1-20 |
| P, A | WO 2019/022222 A1 (NISSAN CHEMICAL CORPORATION) 31.01.2019 (2019-01-31) claims | 1-20 |
| P, A | WO 2019/117262 A1 (NISSAN CHEMICAL CORPORATION) 20.06.2019 (2019-06-20) claims | 1-20 |
| P, A | WO 2019/235569 A1 (NISSAN CHEMICAL CORPORATION) 12.12.2019 (2019-12-12) claims | 1-20 |

Form PCT/ISA/210 (continuation of second sheet) (January 2015)

**INTERNATIONAL SEARCH REPORT**
Information on patent family members

International application No.
PCT/JP2020/003311

| Patent Documents referred in the Report | Publication Date | Patent Family | Publication Date |
|---|---|---|---|
| WO 2009/154201 A1 | 23 Dec. 2009 | US 2011/0117651 A1 claims<br>EP 2308960 A1<br>CN 102066554 A<br>KR 10-2011-0025976 A | |
| JP 2018-530594 A | 18 Oct. 2018 | US 2018/0297964 A1 claims<br>WO 2017/064277 A1<br>EP 3156404 A1<br>CA 3001397 A1<br>KR 10-2018-0063312 A<br>CN 108368104 A | |
| US 5990141 A | 23 Nov. 1999 | US 5700823 A fig. 1E<br>US 5610173 A<br>US 5700822 A<br>US 5783592 A<br>US 5932602 A<br>US 5958959 A<br>US 6335356 B1<br>WO 1995/019169 A2<br>WO 1996/033745 A1<br>EP 1000617 A2<br>EP 997153 A1<br>CN 1182371 A | |
| WO 2019/022222 A1 | 31 Jan. 2019 | TW 201909890 A claims | |
| WO 2019/117262 A1 | 20 Jun. 2019 | (Family: none) | |
| WO 2019/235569 A1 | 12 Dec. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11504000 W
- WO 2008155920 A
- US 5994503 A
- WO 2018198077 A
- WO 2014017513 A
- WO 2016136986 A
- JP 2019014899 A
- JP 2019103322 A


### Non-patent literature cited in the description


- **SUSAN BRESLIN.** *Drug Discovery Today,* 2013, vol. 18 (5), 240-249
- **ROBERT ROSKOSKI JR.** *Pharmacological Research,* 2016, vol. 100, 1-23
- **JUSTICE RW et al.** *Genes & Development,* 1995, vol. 9 (5), 534-546
- **HERGOVICH A.** *Cell & Bioscience,* 2013, vol. 3 (1), 32
- **PEFANI DE et al.** *The FEBS Journal,* 2016, vol. 283 (8), 1392-1403
- **MENG Z et al.** *Genes & Development,* 2016, vol. 30 (1), 1-17
- **ZHAO B et al.** *Current Opinion in Cell Biology,* 2008, vol. 20 (6), 638-646
- **IMAJO M et al.** *The EMBO Journal,* 2012, vol. 31, 1109-1122
- **HONG W et al.** *Seminars in Cell & Developmental Biology,* 2012, vol. 23 (7), 785-793
- **GUJRAL TS et al.** *Proceedings of the National Academy of Sciences of the United States of America,* 2017, vol. 114 (18), E3729-E3738
- **LEE DH et al.** *Nature Communication,* 2016, vol. 7, 11961
- **MOROISHI T et al.** *Cell,* 2016, vol. 167, 1525-1539
- **AYLON Y et al.** *Cell Death & Differentiation,* 2014, vol. 21 (4), 624-633
- **JOHNSON R et al.** *Nature Reviews Drug Discovery,* 2014, vol. 13 (1), 63-79
- **LEACH JP et al.** *Nature,* 2017, vol. 550 (7675), 260-264
- **FAN F et al.** *Science Translational Medicine,* 2016, vol. 8 (352), 352ra108
- **YANG Z et al.** *Molecular and Cellular Biology,* 2014, vol. 34 (9), 1607-1621
- *Synthesis,* 2002, vol. 13, 1857
- *Journal of Radioanalytical and Nuclear Chemistry,* 2013, vol. 298 (1), 9
- *Nature Biotechnology,* 2010, vol. 28 (4), 361-366
- *Nature Protocols,* 2011, vol. 6 (5), 689-700
- *Nature Protocols,* 2011, vol. 6 (5), 572-579
- *Stem Cell Research,* 2011, vol. 7, 97-111
- *Stem Cell Reviews and Reports,* 2010, vol. 6, 248-259
- Design of Prodrugs. Elsevier, 1985